# EUROPEAN PATENT SPECIFICATION

(11) **EP 1 589 104 B1**
(45) Date of publication and mention of the grant of the patent: **18.06.2014**
(21) Application number: 04703806.2
(22) Date of filing: 21.01.2004
(51) Int. Cl.: C12N 15/09, C12Q 1/68

(54) **METHOD AND DEVICE FOR THE DETECTION OF MUTATIONS IN ISOLATED GENE SEQUENCES OF THE LOW-DENSITY LIPOPROTEIN RECEPTOR (LDL-R) WHICH IS ASSOCIATED WITH FAMILIAL HYPERCHOLESTEROLEMIA**
VERFAHREN UND VORRICHTUNG ZUM NACHWEIS VON MUTATIONEN IN ISOLIERTEN GENSEQUENZEN DES MIT FAMILIÄRER HYPERCHOLESTEROLÄMIE-ASSOZIIERTEN LDL-R (LOW-DENSITY LIPOPROTEIN RECEPTOR)
PROCEDE ET DISPOSITIF PERMETTANT DE DETECTER DES MUTATIONS DANS DES SEQUENCES GENETIQUES ISOLEES DU RECEPTEUR DE LIPOPROTEINES DE BASSE DENSITE (LDL-R) ASSOCIE A L'HYPERCHOLESTEROLEMIE FAMILIALE

(30) Priority: 28.01.2003 ES 200300206; 17.11.2003 ES 200302671
(43) Date of publication of application: 26.10.2005
(73) Proprietor: Progenika Biopharma, S.A., 48160 Derio (Bizkaia) (ES)
(72) Inventor: MATA LOPEZ, Pedro, 08025 Barcelona (ES); ALONSO KARLEZI, Rodrigo, Alberto, 08025 Barcelona (ES); MOZAS ALONSO, Pilar, 08025 Barcelona (ES); REYES LEAL, Gilberto, 08025 Barcelona (ES); POCOVI MIERAS, Miguel, 08025 Barcelona (ES); CASTILLO FERNANDEZ, Sergio, 08025 Barcelona (ES); TEJEDOR HERNANDEZ, Diego, 08025 Barcelona (ES); MARTINEZ MARTINEZ, Antonio, 08025 Barcelona (ES); MALLEN PEREZ, Miguel, 08025 Barcelona (ES)
(74) Representative: Durán-Corretjer, S.L.P.
(86) International application number: PCT/ES2004/070001
(87) International publication number: WO 2004/067740

(56) References cited:
- EP-A1- 1 304 374
- WO-A1-02/06467
- US-A- 4 966 837
- FOUCHIER S. ET AL: 'The molecular basis of familial hypercholesterolemia in The Netherlands.' HUMAN GENETICS vol. 109, no. 6, December 2001, pages 602 - 615, XP002980736
- PISCIOTTA L. ET AL: 'A "de novo" mutation of the LDL-receptor gene as the cause of familial hypercholesterolemia.' BIOCHIMICA ET BIOPHYSICA ACTA vol. 1587, no. 1, 21 May 2002, AMSTERDAM, NL, pages 7 - 11, XP004354397
- LIND S. ET AL: 'Genetic characterization of Swedish patients with familial hyperchloresterolemia: a heterogeneous pattern of mutations in the LDL receptor gene.' ATHEROSCLEROSIS vol. 163, no. 2, August 2002, pages 399 - 407, XP002980737
- VARRET M. ET AL: 'Results of the molecular analysis of the 220 point mutations in the human LDL receptor gene database.' ATHEROSCLEROSIS vol. 134, no. 1-2, October 1997, page 74, XP002980738
- GARCIA-GARCIA A B ET AL.: "Molecular genetics of Familial Hypercholesterolemia in Spain: ten novel LDLR mutations and population analysis", HUMAN MUTATION, vol. 454, 2001, pages 1-8,
- THIART R ET AL: "Mutation analysis in a small cohort of New Zealand patients originating from the United Kingdom demonstrates genetic heterogeneity in familial hypercholesterolemia", MOLECULAR AND CELLULAR PROBES, vol. 14, 2000, pages 299-304,
- EBHARDT M ET AL: "Mutation analysis in 46 German families with familial hypercholesterolemia: identification of 8 new mutations", HUMAN MUTATION, vol. 226, 1999, page 1-6,

### SEQUENCE LISTING

<110> EFARMES, S.A.
<120> Method and device for detecting low density lipoprotein receptor gene mutations
   associated with familial hypercholesterolemia
<130> PCT-154
<160> 259
<150> ES200300206
   <151> 28.01.03
<150> ES200302671
   <151> 17.11.03
<210> SEQ ID NO.: 1
   <211> 60.000
   <212> polynucleotide
   <213> human
<220>
   <221> gene
   <223> rLDL
<400>

aaaagatggt gtatccattc aatggaacat tatttggcct ttaaaaggaa ggaaattctc 60
actgagcata gtggtttatg cctgtaatcc cagcactttg ggaggctgag gcagggggga 120
gggggcggtt cacctgaggt caggagttca agaccagcct ggccaacatg gtgaaatccc 180
gtctctacta aaaatacaaa aaaattagcc gagtgtggtg gcacacacct gtaagccagg 240
ctacacggga gactgaggca ggagaatcgc tggaacccgg.gaggcagagg ctgcagagag 300
ccgagattgc gtcactgcac tccagcctgg gtgacagagc gagactcttg tcttaaaaaa 360
aaaaagaagg aaggaaggaa ggaaggaagg aagttctgac acaggctcca acacagatgt 420
tatgctcagt gaaataagcc agacatgaaa ggacaaatac tgcctgatct cattcataag 480
aggtccctag aattgtagaa tggtgtgtgc cacgggctgg gagggggtgt ggccagagtt 540
tcagtttggg aagttgagaa tgttctggag atggatggcg gtagtggtgg ttgcacaact 600
gtgtgaatgc gcttaatgcc tctgaattgt gcagttacaa gtggttcgga tgggccgggc 660
gcggtggctc atgcctgtaa tcccagcact ttgggaggcc gaggcaggtg gatcatgaga 720
tcaggagatc gagaccatcc tggctaacac ggtgaaaccc catctctact aaaaaataca 780
aaaaattagc caggcatggt ggtgggcacc tgtagtccca gctacttggg aggcggaggc 840
aggagaatgg cgtgaacacg ggaggcagaa cttgcagtga gccgagatca cgccactgca 900
ctccagcctg ggcgacagag tgagactccg tctaaaaaaa aaaaagtggt taagatgggc 960

ctccagcctg ggcgacagag tgagactccg tctaaaaaaa aaaaagtggt taagatgggc 960
cgggcatggg ggatcacgct tgcaatccca acactttggg aggctgaggt gggtgattac 1020
gaggtcagga gttcgagacc agcctgacca ccatggtgaa accccgtctc tactaaaagt 1080
acaaaattag ccgggtgtcg tggcacacgt ctgtaatccc agctactggg gaggctgagt 1140
tgggaggatc acctgagccc agggaggtcc aggctgcagc aagccatgat tgcaccactg 1200
cactccagcc tgggtgagag agtgagaccc tgtctccaaa caaacacaca tgaaaaacag 1260
attttttttg ccaggtgcag tggctcacac ctgtaatccc agcactttgg gaggccaagg 1320
cgggtggatc acgaggtcag gtgactgaga gcatcctggc taacacggtg aaaccctggc 1380
tctactaaaa atacaaaaat ttagccgagc atggtggtgg gcacctgtag tcccagctac 1440
tcgggaggct gaggcaggag aatggcatga acctgggagg cggagcttgc agtgagctga 1500
gatcacgcca ctgcactcta gcctggggga cacagcaaaa ctgtctcaaa aaaaaaaaaa 1560
aaggtttttt taatttaaaa aggaaagaaa aggagagtgc tcgtgtggca ggcacctagc 1620
cctgtccagc gcaccctgag acagggatga tgtctcctcc ttgacctaag accacaagtt 1680
ctaaccaatt caaccgagga cagagcccca attccaggca gggcaatggg gtcgccttgt 1740
gaactaagat gcagatggag aagagcagac acagacacag gtcttggggc ccctgcaggg 1800
gtttctcact ggcttttccc ccctggattc ctatgggttc tggggaacag agttaggtcg 1860
gctggcaaga cagatgcatg aggctgtggc gcccttgaca ttgagccgga gggccagagt 1920
tcgtcattgc tgacgcagag aagctgggag ccaaggttag ccagatggtt tggaggagtt 1980
ttaaacaatc ttttcttttc tttctctttc catctgtctg tccttctttc ctcccttcct 2040
gccccctttc ttttctcctt tctttccttc ctctctcctt cctccctttt tttctttttt 2100
tttggttttc tttttgtatt agtattatta ttttttagac agggtcttgc tctgttgccc 2160
aggctggagg gcagtggcac gatcacagct cagtacaccc tcaaccttct gggttcaagc 2220
aatcctcctg ccttggcctc ccaggtagct gggactacag gcgtgtgcca ccacacctgg 2280
ttaatttttt ttttttttga gacggagtct tgctctgtca cccaggctgc agtgcagtgg 2340
cgtgatctcg gctcactgca acctccacct cccgggttca agcgatcctc ctgcctcagc 2400
ctcccgagta gctgggatta cacgcgcccg ccaccaagcc cggctaattt ttttattttt 2460
agtagagaca gagtttcacc acgttggcca ggctcgtctc aaactcctga cttagtgatc 2520
tacccacctt ggcctctcaa agtgctggga ttagaggcgt gagccaccat gcgcagccaa 2580
tttttgtatt tttagtagag atggggtttc accatgttgg tcagtctggt ctcgaactcc 2640
tgacctcaag tgatccacct gcctcagcct cccaaagtgc tggaattaca ggcatgagcc 2700
accgcgccca gccctcttaa ccatttttaa gtgcacagtt cagcagcatt aagcacattc 2760
acattgttgt gcaaccatca gcccccgtcc atctccagct ttctcttttt ttttgtttgt 2820
tttgagacag ggtcttactc tctcgcccag tatagagtgc agtggtgcgg tcttggctcg 2880
ctgcaacctc tgccttccag gttcaagcta ttctcctgcc tcagtctccc cagtagctgg 2940
gattacagac acacatcacc acgccctgct aattattttg catttttagt agagatggtg 3000
tttcaccata ttggccaggc tgatcttgaa ctcctggcct caagtggtct gctccaaact 3060
gctgagatta cagccgtgag ccactgctcc cagccatctg cacctttctc atcttcccaa 3120
atgtaactat gtccccgtga aacactcact ccccattcca cctccccagc ccctggcacc 3180
ccccatttta ttctggtgct aggggaattt caaaccaggc aagtctcaac acatgctcga 3240
gtgtaagaac cagcccacag cctcgttccc taatcacggt caaaccagaa ttctactcca 3300
ggttctactc tgtgaatctg ctttctgtga atctgttact ctggggaccg cctataagtt 3360
gaatcctaca gtgtctccac ttcagtgact ggcttatttc acttttctcc tctttattta 3420

tgagacaaaa tttcgctctt gttgctcagg ctggaatgca atggcgtgat ctcggctaat 3480
ttttttgtat ttttagtaga ggcggggttt caccatgttg gccaggctgg tctcgaactc 3540
ctgacctcag acgatccact ttggccttcc aaagtgctgg gattacaggc gcggcccacc 3600
tttctcctct taatcacaca ggtaatccat acatacgaca ttcttttttt tttttgacac 3660
ggagtcttac tctgtcacct aggctggagt gcagtggcgc aatcttggct cactgcaacc 3720
tctgcctccc aggatcaagc aattctcctg cctcagcctc ctgagtagct gggattacag 3780
gtaaccatca ccacacctgg ctaaattttg tatttttagt agagacgggg tttcaccacg 3840
ttggccacgc tggtattgaa ctcctggctt caagtgatct tcctgtctcg gtctcccgaa 3900
gtgctgggat tacaggaatg agccactgtg cccggccaat acgacatctg tgcaatgaag 3960
tgcaacatat aagacaccct tcccccaccc actgccccca ccaccgcccc cacgccccca 4020
cccccatctc cagatcagaa cctggggctg tgcaatttta aacgttgtag ccacttgcta 4080
cttgggtagt tgaagttcag tctcagccag gttggagtcc tggactctgg cccctctttt 4140
atttttattt tttatttttt tttgagacag agtctcgctc tgtcgcccag actggagcgc 4200
agtggtgcga tctcggctca ctgcaagctc tgcctcctga gttcacgcca ttcccccgcc 4260
tcagcctccc gagcagctgg gactacaggc gcccgccacc acacccggct aatttcttgt 4320
attttttagt agagatgggg tttcaccctg ttagccagga tggtctagat ttcctgacct 4380
tatgatccgc ctgcctcggg cctcccaaag tgctgggatg acaggagtga gccaccgcgc 4440
ccggcctctt tttttttttt tagacagtct ctgtcaccca ggctagagtg cgatggtgcg 4500
atctcggctc actgcaacct ccaccttccg ggttcaagcg attctcctgc ctcagcctcc 4560
tgagtatctg ggattacagg tgcctgtgac cacgcccggc tgatttttgt atttttagta 4620
gagacggggt ttcaccacat tggtcaggct agcctcaaac tcctgacccc gtgatccttc 4680
cgcctcagcc tcccaaagtg ctgggattac aggactctgg cccatcttgg ctgctgccaa 4740
tgtccttcct tctatcttgg tttttccaca gttacgcaca tgccagataa cggcgagtct 4800
gttccccagc aactgcaacg gatctgccca ccactgggaa atggaagacc ttgcagccca 4860
ggtctttgta gaccaagatt agattgtggt caacaaacac ctgaccttgg cctttggaac 4920
catcagccat gtcagctaaa ataaaagcag aatctggctg ggcgcagtgg ctcacgcctg 4980
taatcccagc actttggggg gctgaggtgg gcagaccacc tgaggtccgg cgttctagac 5040
cagcctgacc aatatgatga aaccccgtct ctactaaaca tacaaaaatt agctgggcat 5100
ggtggcgggc acctgtaatc ccagctactc gggaggctga ggaaggagaa ttgcttgaac 5160
cctggaggca gaggttgcag tgagccgaga ttgcgccact gcactccaac ctggactgca 5220
gaacaagact ctgtcccaaa agcagataaa taaaaataaa taaaaataaa aatatggccg 5280
ggcatggtgg ctcacacctg taatcccaac actgggaaga tgaggcgggc agatcacgag 5340
gtcagggatt cgagaccagc ctggccaaca tggtgaaacc ccgtctctac taaaaataca 5400
aaaattagcc gggcatgatg ctgcatgcct gtaatcccag ctactctgga ggctgaggca 5460
ggagaatcgc ttcatcccgg gaggtggagc ttgcagtgag ctgagatcgc gccactgcac 5520
tctagcctgg gcaaaagagt gagactccat cgcaagaaaa aaaaaaaaaa aagctgcaag 5580
ctctgtctcc cgggttcaag tgattctcct gcctcagcct tccaagtagc taggattata 5640
cgcgcccgcc accatgcctg gctaattttt gtatttttag tagagatgcg gtttcaccat 5700
gttggccagg ctggtctcaa actcctgacc tcacgtgatc cacctgcctc ggcctcccag 5760
agtgctggga ttacaggtgt gaacccctgc gcctggccaa gaaaagttgc ttgaatgaag 5820
agtaaataga agacccagaa agaaatgatt cgtccgagga aggtcacaga agcaacgtaa 5880
tcaagatgga aatctgactc ttcctaattt tggccagact tcccatccct ccaaagcttt 5940

ccagactctt ccagatcatt ctagatattt ccagaaatca ttcgtgaaat ctaactagga 6000
gtagtctgta aacaatgtgt ttcacacaga tacaattcat aaacgatgag aagacaagga 6060
cacttcatga atgaaatttt tacggccggg tatgttggct cacgcctata atcccaggac 6120
tttggaagac ccaggcagga ggattgcttg agtccaggag ttcaagacca gtctgggcca 6180
catagtgaga ccctgtcgct acaaaaaatt taaaaattag gtagatatgg tggtgtatgc 6240
ctctagtttt agcttttttg gaggctgaag caggaggatc tcttgagccc aggaggttga 6300
gctgcaatga gctacgattg aactactaca ctccagtctg ggtgacagag aaagaggctg 6360
cctcaaaaaa ataaaaataa aaaaataagg ccggacgcgg tggctcacgc ctgtaatccc 6420
agcactttgg gaggctgggg tgggcagacc acgaggtcag gagatcgagg ccatcctggc 6480
caacatgatg aaaccctgtc tctactgaaa acacaaaaat tagctgggcg tggtggcgta 6540
tacctgtaat cccagctact cgggaggctg aggcaggaga atcacttgaa ccagggagtc 6600
agaggttgca gcgagaggag attgtgccac tgcattccag cctggcaaca gagcaagact 6660
ccgtctcaaa aaagaaacaa caacagcaac aacaacaaaa aaaacataaa aaagttcggg 6720
cacggtggct cacacctgta atcccagcac tttgggaggc caaggtgggt agatctcttg 6780
aggtcaggag ttcaagacca gcctggccaa caaacatggt gaaaccccgt ctctactaaa 6840
aatacaaaaa gtagccgggt gtagtcccag ctactcggaa ggctgaggca ggagaatcgc 6900
ttcaacctgg gagatggaag ttgcagtgaa ctgagattgc gccactgggt gacagagtaa 6960
gactcttgtc tcaaaaaaaa aaaaagaaag aaagtttaat ttaatgattc aaataatgac 7020
ctgctcgaga gataaatata aagtctaacg taagaggtgt atactttttc ctctgtcctg 7080
ctgtcctcgc cccacctcac cccaagtccc aacctgattg atcagtctcc tttccctctg 7140
gtagccccac tcccatgacc gaaccgagaa gtcatgcacc cgcataagaa ctctaatttt 7200
ttttttcaaa gtcttctcac tgccccaaaa atagtttctt tcattcccag gggatgtgaa 7260
agtgtctctc ccaattttat ttcaacctcc cagcgttcca cacatatgcc ttgcctcagc 7320
cagctttcac tgatctgcca tttccacctc ggcgctgctc ctacctgcgg aaatcctgtc 7380
catccatagt ctgatttctg ttgttccaga acattctttt ttttttcccc tggaacattc 7440
tttaagatac ctcaataaat gaaaccagag ggtatagagc agtatgaatg ggtactacaa 7500
tgtacagggg gaaatggagg ggaatatgat atactctcct ccttgtatat gcttagaatg 7560
ttctagaagg atatgcttaa aaggttagca gtcctggcca ggcgtggtgg ctcacgcctg 7620
taatctcagc actttgggat gccaacgcgg acggatcaca aggtcaggag ttctagatca 7680
gcctgaccaa tatagtgaaa cctcatcttt actaaaaata caaaaattag ccgggtacgg 7740
tggcatgtgc ctgtagtccc agctactttg gaacctgagg caggagaatc gcttgaactc 7800
gggaggcaga ggttgcagtg agccgagact gtgccattgc actgcagcct gggtgacaga 7860
acaggactcc gtctcaaaaa aaaacaaaaa aggtcagcag tcttaattgt cagagggcag 7920
gggacctgca tgggatggag gtttttccat gtgtccacct tttgagccct tttgcttttt 7980
ttttttaaat ctttttattg tagcaaaata gatataaaat ttaccctttt tttttttgag 8040
acagggtctc actctgttgc ccaggttgga gtgcagtggc atgatcttgg ctcactgcag 8100
cctctgcctc ctgggttcaa gcgattttcc tgcctcagcc tcccgagtag ctgggattac 8160
aggtgcttgc caccataccc ggctaatttt gtatttttag tagagacggg gttacgccaa 8820
gttggccaag ctggtcgcaa actcctgacc tcaagtgatc cgcccccctc ggcctcccaa 8280
agtgctggga ttacaggcag gagccaccac gctcagccct aaaatttacc atattaacca 8340
ttttcaagtt cagaggcatt aaagtatact cacattgttg ttcaactgtc accactactc 8400
acctgcagaa gtttttcatc ttgcaaagtg aaaaccccat acccaatttc ccgttcttcc 8460

tctcagcccc tggtaatcac tattctactt tttgtctact ttttgtatga atttgcctat 8520
tctaggacct aatagaagtg gagtcaaacc tgtttgtcct tttgtggctg gcttatttca 8580
cccggcctta tatcctcaag gtttatccat gttggaggat gcctgaattt ccttgttttt 8640
aaggctaaat tttattctat tatattaata tgtcatattt tgtttatcct gatggacact 8700
tgggttgatt ccacctttgg ccattttgaa gaagcttcta tgtacatggt atacacatat 8760
atctttgggt ctctgctttc aatgcttttg gggatatttc agatgtggaa tttctggatt 8820
ataaggcaat tttttttttt gagacagact ctcgctcttg tcgcccaggc tagaatgtgg 8880
tggtgtgatc tatttttttt tttttttgga gatggagtct cgctctgtcg cccaggctgg 8940
agtgcagtgt cacgatctca gctcactgca agctccgcct cccaggttcg tgccattctt 9000
atgcctcagc ctcccaagta gctgggacca cagccgccca ccacctcacc cggctaattt 9060
ttgtattttt agtagagaca gggtttcact atgttggcca ggatggtctc gatctcctga 9120
cctcgtgatc cgcctgcctc ggcctcccaa agtgctggga ttacaggcgt gagccactgc 9180
acccggctgg tgtgatcttg gctcgctgca acctctgcct cccaggttca agcgattctt 9240
gtgcctcagc ctctccgcag ctgggactac aggtgtgcgc cactgtgccc agctactttt 9300
taaaaatata tgtgtattta ttatactttt aagttctggg atacatgtac agaacgtgca 9360
ggtttgttac ataggtatac atgtgccatg gtggtttgct gcacccatca accggtcatc 9420
tacattaggt atttctccta atgctatccc ttccctagcc ctccactctc ccggtttttt 9480
gttttgtttt gttttgttgt tttgttttta gtagagacag ggtctcacca tgttgcccag 9540
gctagtcttg aactcctgac ctcaagtgat ccgcccacct cagcctccca aagtgctggg 9600
attacaggtg tgacccacta cactcggcct tattttcact tatttatgca attttcacta 9660
ttgctatatt ctaggaggca ctgtggaatt gcactgtgga attttagtat tgctgtattt 9720
cagcaagcca tgaggtctgt cagcacacgg ctttgggcat tttgtgaaga taactgatgc 9780
cagctgagcc aaggcaggtt cctgattcca cccactggca ggcaccgagg tctctgctgt 9840
tactgatggt ttctctgtgg attgatgggc ttaaggccag accacagctg caatggctca 9900
cctctgccaa aggccaggct cgttggggca gagacctatt ccggactgag cctcctggtg 9960
aattagagag gtagaaaatg ggaggacggg ggcaggtggg ctattacagc gaggaaaatg 10020
cccaccctga gttgtattag ataactttgg gagttcagga actttccaat aaagtgggtt 10080
ccacagcagg attacttact gactccctaa tagaaagaag gcaggcacag gccgggcgtg 10140
ttggctcatg tctgtaatcc cagcacgttg ggaggctgag gcgggtggat cacaaggtca 10200
ggagatccag accatcctgg ctaacaaagt gaaaccccgt ctctactaaa aatacaaaaa 10260
attaggctgg gcgtggtggc tcgtgcctgt aatcccagca ctttgggagg ctgaggcggg 10320
cggatcacga ggtcaggaga tcgagaccgt cctggctaac acggtaaaac cccatctcta 10380
ctaaacatac aaaaaaaaat tagccaggtg tggtggcggg cgcctgtagt cccagctact 10440
caggaggctg aggcaggaga gtggtgtgaa ctcgggaggc gcagcttgca gtgagccgag 10500
actgcgccac tgcactccag cctgggcaac agacagagac tccgtctcaa aaaaaaaaaa 10560
aaaaaataca aaaaattagc caggcgtggt ggcacgtgca cgtgactgta gtcccagcta 10620
cttgggaggc tgaggcagga gaattgtttg aacccgggag acggaggttg cagtgagccg 10680
agatcgcgcc actgcactcc agcctgggtg acagagctag actccgtcaa aaaacaaaaa 10740
acaaaaaaca aaaaaacaaa aaaaaaaaaa cagcaggaac tggcaggtct tccctgaaga 10800
gataaaaaaa aaaaaatgca gttgcaacac aaaagcagcc acagagaaaa gcaaacccat 10860
atatggtatt tattatgcac cgagtgtggc tctaatcact tttttttttt taattgagag 10920
acagcctggc tctgttgatt gggctggagt gcagtggcgc gaccgtagct cattgcagcc 10980

tcaacctcct tggctcaagc aatcctccta cctcagcctc ctgagtagct gggaccacag 11040
gtgtgagcca ccacgcctgg ctaattgttt tttttttttt tgtagagaca gggtctcact 11100
atgtggccca ggctggtttc caactcctgg gctcaagtga tcctcccacc tctgcctccc 11160
aaagtgctgg ggattacagg catgagccac ctcgcctggc ctctagtcgc tttatatatt 11220
ttaacttaat ccttacaaga gccctgtgag ctagttacag gagcacaaat ggaaaccaag 11280
aaacagaaaa atttatcagc atgactcagt cctcagagcc atgtatggcc gtgtccgtgc 11340
atggcaggca ggtcaggggc ctggggaacg ctgttctgga aaccttggcc aggccttggc 11400
acccgaggaa tgtgcttttc agagtttttg tggctctttt ccagacctgc cctgacctct 11460
agctctggga actatgtaag ccaagtgcct tccgggaagg gagtccctct cctggtaact 11520
ctttctgggt aaccagatgt ggactcatga cacacactga gcctacgtct tataattttt 11580
tgtttttgtt tttgagacag tttcggtctt cttgcccagg ctggagtgca atggtgcgat 11640
ctcggctcac tgcaacctct gcctcccagg ttcaagcgat tctcctgcct cagcctccct 11700
agtagctgga attgcaggca tgcgccacca cgcctggcta attttttgta tttttttttt 11760
tttagtagaa acggggtttc accttgttag ccaggctggt caccaactcc tgacctcagg 11820
tgatccgccc acctctgcct cccaaagtgc tgggattaca ggtgtgagac agctgtgagc 11880
caccacgccc ggcgcatttt tttttctttt tttttcagag ggagtgtccc tctgtcaccc 11940
aggctgaagt gtagtggcgt gatctcggcc cactgtaacc tctatctccc aggttcaagt 12000
gattctcctg actcagcctc ccaagtagct gggactacag gcgcctgcta ccatgcctgg 12060
ctaatttttg tagttttagt agaaaccggg ttttgccatg ttggccaggc tggtctcaaa 12120
ctcttgactt caggtgatcc acctgccttg gccttctgaa gtgctgggat tatagggcat 12180
gagccactgt gactggccat cttaaatttt tttttttttt tttttttttt ttgagacagg 12240
gtttcactct gtcgcccagg ctggagtgca aaggcgcgat cttggttcac tgcaagctcc 12300
gcctcctggg ttcatgccat tctcctgcct ctgcctcatg agtaactgag actacaggcg 12360
cccaccacca cgcccggcta atttttttgt atttttttag tagagatggg gtttcacctt 12420
gttagccagg atggtctcga tctcctgacc tcgtgatcca cccgtctcgg cctcccaaaa 12480
tgctggcatt acaggcgtga gccaccgcac ccagccttaa attttttttt aagggaaatc 12540
aaacccagtg atattgggcc agtacagtgg ctcacacctg taattccacc actttgggag 12600
gctgaggcag gtgaatcacc tgaggtcagg agttcgagac cagcccggca aacatggcga 12660
aaccccgtct ctactaaaaa taagaaaatt agccgggcgt agtggcatgc acctgtaatc 12720
tcagctactc gggaagctga ggcatgagaa tcgcttgaac ctgggagcag gacgttgcag 12780
tgaaccgata tcacaccact gcactccagc ctgggtgaca gagcaagact ctgtctcaaa 12840
aaaaaaaaga aaaaaaaatc cagtgatact tactttttaa atttttattt acttattttt 12900
tgctttaagt tgaatcttta aacttatctt tatttttgag acacagtctc actctgtcgc 12960
ccaggctgga gtgcagtggt acaaccacag ctcagtgcag cgttgacctc ctgggctcaa 13020
gccatcctcc cgcctcagcc tcccgagtag ctgggactac aggcgcacac aaccatgtcc 13080
agcttatttt tgtatttttt gtagagacag ggtcccactg tgttgccctg gcttgttctg 13140
aactcctagg ctcaagtgat ccccccgcct caccctccca aagtgctggg attacaggca 13200
tgagccacca catccagact tcactttttt gtttaatgtc gcaaatggca taaggaatgg 13260
gattcaatgg ggacacattt ataaacgttg cagcagctcc tagaacttgc ctatccttgt 13320
aaacttctct aggtgattgc taattacttc tttttttttt tttttttttg agacggagtc 13380
tcactctgtc gcccaggctg gagtacagtg gcgcaatctc gtctcactgc aaactccacc 13440
tcccgggttc acgccattct cctgcctcag cctcccgagt agctgggact acaggcaccc 13500

gccaccacgc ccggctaatt ttttgtattt tttttagta gaggtggggt ttcactgtgt 13560
tatccaggat ggtcttgatc tcctgacctc gtgatccacc tgcctcagcc tcccaaagtg 13620
ctgggattac aggcgtgagc caccatgccc agcccgctaa ttatttcaat ttgaccttga 13680
cactgagcct gccaagtagg ttcaagcatt ttgatggccc ctttacaggt tgggaaagct 13740
aatttatctg tccaaggccg aattctgaaa ctgagtctta actgccaaaa attcttatca 13800
tcaatttctt cttctgggtt gggcacagtg gctcatgcct gtaaagccag caatttgaga 13860
ggcatcatga tgcaagagga agaggattga gtgaagctag gagtttggga ccagcctggg 13920
caacatagtg agaccccatc tataaaaaaa aattaaaaat tagttgggca tggtggtgca 13980
ctcctgtggt cctagctatt caggaggctg aggtgggagg attccttgag cccagggttg 14040
acgctgcaga gagctgtgat cacgccactg cagtccagcc tgagtgacag ctggaaataa 14100
tgataaataa ataataaata attatttaaa aaattataat aaaaataatt aaaaaattat 14160
tttccctgat taatcttttt ttttgtcctt ctgagagttc aatttgtccc ttttctgcct 14220
ggtctcctag gtttccctaa aatcctgctg agaggttagc actgcctgcc aaagtcagtt 14280
tgcaaaatcc cagagaaatc cagcttattc ctgggggaac cgccaagact gcccagccct 14340
gtgtggggtt caggcaagtt tctcacatgt gcctttttgg caagaggcct ctggcaaccc 14400
catgagtccc caaagagact caattctaaa agttggtctc caccagctct ctgtggctta 14460
ggggttcaag ttcaactgtg aaagccctgt tttgttttga ttttgctttg agggagagga 14520
aaccgccctt ctgtttgttc aactccttct cctaagggga gaaatcaata tttacgtcca 14580
gactccaggt atccgtacaa ttgatttttc agatgtttat actcagccaa aggcgggatc 14640
ccacaaaaca aaaaatattt ttttggctgt acttttgtga agattttatt taaattcctg 14700
attgatcagt gtctattagg tgatttggaa taacaatgta aaaacaatat acaacgaaag 14760
gaagctaaaa atctatacac aattcctaga aaggaaaagg caaatataga aagtggcgga 14820
agttcccaac atttttagtg ttttcctttt gaggcagaga ggacaatggc attaggctat 14880
tggaggatct tgaaaggctg ttgttatcct tctgtggaca acaacagcaa aatgttaaca 14940
gttaaacatc gagaaatttc aggaggatct ttcagaagat gcgtttccaa ttttgagggg 15000
gcgtcagctc ttcaccggag acccaaatac aacaaatcaa gtcgcctgcc ctggcgacac 15060
tttcgaagga ctggagtggg aatcagagct tcacgggtta aaaagccgat gtcacatcgg 15120
ccgttcgaaa ctcctcctct tgcagtgagg tgaagacatt tgaaaatcac cccactgcaa 15180
actcctcccc ctgctagaaa cctcacattg aaatgctgta aatgacgtgg gccccgagtg 15240
caatcgcggg aagccagggt ttccagctag gacacagcag gtcgtgatcc gggtcgggac 15300
actgcctggc agaggctgcg agc atg ggg ccc tgg ggc tgg aaa ttg cgc 15350
met gly pro trp gly trp lys leu arg
-21 -20 -15
tgg acc gtc gcc ttg ctc ctc gcc gcg gcg ggg act gca g gtaaggcttg 15400
trp thr val ala leu leu leu ala ala ala gly thr ala v
-10 -5 -1 1
ctccaggcgc cagaataggt tgagagggag cccccggggg gcccttggga atttattttt 15460
ttgggtacaa ataatcactc catccctggg agacttgtgg ggtaatggca cggggtcctt 15520
cccaaacggc tggagggggc gctggagggg ggcgctgagg ggagcgcgag ggtcgggagg 15580
agtctgaggg atttaaggga aacggggcac cgctgtcccc caagtctcca cagggtgagg 15640
gaccgcatct tctttgagac ggagtctagc tctgtcgccc aggatggagt gcagtggcac 15700
gatctcagct cactgcaacc tccgcctccc gggtttaagc gagtctcctc tctcagcctc 15760

ccgaatagct gggattacag gcgcccaacc accacgcccg cctaattttt gtatttttag 15820
tagagacggg ttttcaccat tttggccagg ctggtctcga accccgacct caggtgatct 15880
gcccaaaagt gctgggatta caggcgtcag ccaccgcgcc cggccgggac cctctcttct 15940
aactcggagc tgggtgtggg gacctccagt cctaaaacaa gggatcactc ccacccccgc 16000
cttaagtcct tctgggggcg agggcgactg gagacccgga tgtccagcct ggaggtcacc 16060
gcgggctcag gggtcccgat ccgctttgcg cgaccccagg gcgccactgc catcctgagt 16120
tgggtgcagt cccgggattc cgccgcgtgc tccgggacgg gggccacccc ctcccgcccc 16180
tgcccccgcc cctttggccc gccccccgaa ttccattggg tgtagtccaa caggccaccc 16240
tcgagccact cccccttgtcc aatgtgaggc ggtggaggcg gaggcggcgg tcgggaggac 16300
ggggcttgtg tacgagcggg gcggggctgg cgcggaagtc tgagcctcac cttgtccggg 16360
gcgaggcgga tgcaggggag gcctggcgtt cctccgcggt tcctgtcaca aaggcgacga 16420
caagtcccgg gtccccggag ccgcctccgc gacatacacg agtcgccctc cgttatcctg 16480
ggccctcctg gcgaagtccc cggtttccgc tgtgctctgt ggcgacacct ccgtccccac 16540
cttgtcctgg ggggcgccct cgccccacca gccccgatca agttcacaga ggggcccccg 16600
gccaccctca aggcctcggt tccttacgag gttgaaacgt tgcctcagaa tctccccgcc 16660
cctccttggt ctgcagccga gatcttcagc cacggtgggg cagctatccc ccgggaccga 16720
ccccctgggg tggcctcgct tcttcagagg ctgtgaatgg cttcggttca gctgtccaag 16780
cggcgatttt tcctctgggt gaaatggatt agattttaga tttccacaag aggctggtta 16840
gtgcatgatc ctgagttaga gctttttagg tggctttaaa ttagttgcag agagacagcc 16900
tcgccctaga caacagctac atggcccttt ccctcctgag aaccagccta gcctagaaaa 16960
ggattgggat tgcctgatga acacaaggat tgcaggaaac ttttttttta attggcaagg 17020
gggttggctt tgactggatg gagagctttg aactgccttg aaattcacgc tgtaactaac 17080
acaccagttt cctctgggag gccagagagg gagggagggt gtaatgaaat acggatgatt 17140
gttcttttat ttttatttac ttatttattt tttaactttt tgtagagatg aggtctcgct 17200
tggttgctca ggctggtctt gaactcctgg cctcaagcga tcctcctacc tcagcctccc 17260
aaagtgttgg gattacagga gtgagccacc gcgccccacc ggggatgatg atgattgcaa 17320
acattctgcc actcagtttt acaaaagaaa gagaggcact ggattaatgt gtatctcact 17380
caccaatcaa cctcttcctt aagagaaaat gttaaggaag tcttaggcaa ggccttgttt 17440
gttcatcact ttagtttctc tctcccggga tggctgagaa tgtgatgttt cctctgttgt 17500
caaggagact acacccctga tgttttcctc cagacttctg agagctggtg tgtgtttcta 17560
gcactttcta gctgcaccac ctcacgctgt agctggcttc aaggcatatc caggggggag 17620
tttcttgtcc atttccttta caaagggaag ttgttggaat ctgaaccgca agccttcact 17680
tagaccaaaa tcaggcaaca gcggtgagcg cagctccaaa cgtgtcaatg actcacccaa 17740
atttgagtaa gggagttggc tgctttaacg agccgcaggg tgattccctt gtcatttccg 17800
gaaataccta tcttccaggg aacactggga aaaaacaggg agacctttgt tgagacagaa 17860
aacctgtagg ggaattctgt tcctcattcc tgctcttatc tgtagacttc ctccctgata 17920
agatccaatt ctagatgggt cggttgctcc ttgctttgat gggtgctttg atggctttta 17980
ttattattat tattattatt attattattt tgatgggctt tttgatgtcc cttttccttc 18040
cacactctgt cccaactgtc aagcaaatag ccttttgttg ctaagagact gcagatgtaa 18100
ccgaccagca gcaaacagtg agtcaggctc tctcttccgg aagcaaaatc aattgctgag 18160
atcactctgg ggaaaatacc caccttattt ggaaagaagc actgatcaat tgatgtctat 18220
tttttttttt tttgagttgg agtctcgccc tgtcacccag gctggagtgc aatggcataa 18280

tctcgcctca ctgcaatccc cgcctcccgg gttccagcaa ttctcctgcc tcagcctcct 18340
gagtagctgg aattataggc gcctgccaca acacccggct aatttttgta tttgtagtag 18400
agatggggtt tcaccacgtt ggccaggctg gtctcgaact cctgacctcg tgatccaccc 18460
gcctcagcct cccaaagtcc aaggattgca ggcgtgaccc actgtgccag ccaatcaatt 18520
gatttctcat tcattttcag ctggctctgt tcccttaagc caggggattt tcgtttgttt 18580
gtttcccctt caaggaaatg attctagcta cagttttgat ttccttgtac aactgttttc 18640
agtagcacag ggaaagaaaa catcgaaagc attcaccacc tcatttgtgt gctgggggaa 18700
aaagcagaaa tgtgtattct ctttttttgt ttcgatgacc ttgttcctga cttgttactc 18760
gtgacttgag agatcagagg gctagaggac tagaatttat agaggtgttt tttttgtttg 18820
tttatttttg ttcgagttgc ccaggctgga gtgcagtggc gcaatctcgg ctcactgcaa 18880
cctctgcctc ccaggttcaa gcgattcttc ggcctcagcc tcctgagtag ctggaactac 18940
aggcgcccgc caccacaccc agctaatttt tgtatttttc agtagagatg ggatttcacc 19000
atattggtca agctggcctc gaactcctga cctcgtgatc cacccgcctc agtttcccaa 19060
agtgctggga gtacaggcgt gagccgccgt gcccggcctt tttgtgtttt tgtgtttttg 19120
agaggagctc attgcttttt aggcttccct agcgtgagaa aatctgggga tccatgctct 19180
agtttacttc cttttttttt ttttttttga gatggagtct cgcttagatt gcctaatctc 19240
agctcattgc aacttctgcc tccggggttc aagggattct cgtgtctcag cctcctgggt 19300
agctaggata cgggcacccg ctaccatgcc tggctaattt tgtactttta gtagagacag 19360
ggtttcgcca cgttggccag gctggtctcg aactcctgac ctcaggtgag ccgcctgcct 19420
tggcctccca aagtgctgag attacaggcg tgagccaccg cgcttggcct aatttgcttt 19480
tcctgaaatt caaatggtct aatatgaaaa acgccaacct tgcttgaaag aataagaaag 19540
aggtgcggtt tcgttgggcc gttgatgttt ggaacaggac tggttttgtc cccttgctcg 19600
gaaagggcag caactgtgag gacagctccc tgacgtgctc tcactcagca ctgttccgtt 19660
cctgagcact gtccccacta gctaggccaa gggagctcat ttggcaggca actgctgtct 19720
ggctgcgcct gtggcagtaa aatctgcctt tatttttggg aggcagggtc ttgccctgtc 19780
gctcaggctg aagtgtgcag ttatagctca ctgcagcctc cagcttctgt actcaactga 18940
tcctcctctc tcagcctcct gagtagctgg gactatacgc acgtgttacc actcccacct 19900
cagtttgttt gtttatttat ttatttattt atttattgag atggagtttt gctcttgctg 19960
cccaggctgg agtgcaatgg cgcgatctcg gctcaccgca acctccacct cctggttcaa 20020
gcgattctcc tgcctcagcc tcctgagtag ctgggattac aggcatgcac caccacgccc 20080
ggctaatttt gtatttttcg tagagatggg gtttctccac attggttcag gctgttctcg 20140
aactcccaac ctcaggtgat ccacccgcct cagcctccca aagtgctggg attataggcg 20190
tgagcccccg aacccggcca ctcccagcta agtttaaatt ttttgtttgt ttgttcgttt 20260
gtttttattt tttgagacag agtctcccgc ccaggctgga gcgcagatca ctgcatcctt 20320
gacctcccag gcttaagcca tcctccccac tcagcctccc aagtagctgg gattacaggt 20380
gtgtgccact atgcttggct aagttgtgta ttttttgtag agatggggtt caagggattc 20440
tcgctttgtt gcctcggttg gtctcaaact cctgggctca agcagtcctc cctcctcagc 20500
ctcccaaggt gctggggaaa tccacttttg aaacattgtc tggagagttg cccaggtggt 20560
agatcacaga aataggtcat cgtggggtcc ttcccatggg tgcagtcttg agccacctgt 20620
ggccagcaaa tatttggaga ataatagtca ggggagagct tgaggtccag ggaaaggttt 20680
tgtttttctt cagggaaagg tttttattgt tctttatccc tccttaaagg accttcaggt 20740
gttactgaca ttcccggtct acccagtggc acatttagtt tgtaagctgg gccctcgtac 20800

agaggtaggg aggtgagagc attggattag tggtcaccaa agctgcggtc acctagtggg 20860
gtgatcagag gctcctccct taagatcttg attgccaacg cctctggccc aactttcctt 20920
tttatttatc gcaagcctcc tggaatctca attgcttttt gcccacccgg tgtgtcagca 20980
caagaaatga gtcatttcct cctttaagca cagttgaaat tgagctgtga gtcagtgagg 21040
tgtgtacgat attgtcaaag cggggtgtgt acagtattga cagatctgta gttgggcaag 21100
agaattatca gagtttgtga ccacagcaga ttccaaagct cgactcattt tcttctctct 21160
tccttccctt ttttcttttc tttttttttt tttttttgac agagtctcgc tctgttgccc 21220
aggctggagt gcagtggcac aatctgggct cactgcagcc cctgcctcct gggttcaaat 21280
gattctcatg tttcagcctc ccgagtagct gcaattacag gcattcgggt tcaagtgatt 21340
ctcctgcctc agccacctga gcagctggga ttacaggcgc ccgccaccac gcccggctaa 21400
tttttgtatt tttagtagag acggggtttc accatgttgg ccaggctggt ctcgaactcc 21460
tgaactcagg tgatccgccc acttcggcct cccaaagtgc tgagattaca gacgtgagtc 21520
accgcgccca gcctgttctg ttctttaatt ctcaaaacac cctctaggaa gtagagactg 21580
ccattctccc ccattttaca gatcaggaaa ctgagtccca gaaggattta gtcagttacc 21640
caagttgttc tagttaaatg gcctggaaag ccagtgaagc ccaggattgt ctatctaacc 21700
cccttactac tctaactttc agggaatcca catgaatgtg ctgggtcaac catcaaagtt 21760
gaaatggata aagggggctg gatgcggtgg ctgatgcctg taatcctagc actttgggag 21820
gccgagatgg gtgggtggat tgcttgagcc caagagtttg agaccagcct gggcaacata 21880
gtgagacacc tgtctctgca aaaaataaat aaaaagttag ctgagtgtga tggtgcaccc 21940
ctctagtcac agctgttgag ttaggcttag gcaggaggat cgcatgaacc tgggaggtgg 22000
aggcggccgt gagcctcagt catgccactg cactccaacc tgggcaacag agtgaaagcc 22060
ggtgtccgaa agagaaagaa aaaaagacat agatacatct tttaaagtta ggttgtatgt 22120
taattaccta caactcagtt tcaactgtgc ttaaaggagg aaatgactca tttcttgcta 22180
catatcaaat tagcccaaaa tgtagtggct taaaacaaca catttatgat ttctcagttt 22240
ttgcgtgtca ggaatttgga agcagcacag ctagacggtt ccagctcagg gtctctcatg 22300
aagttgcaat caaaatattg gcaggagaga aaaacatatt ttcagaagct gcaggcatag 22360
gaagacttgg ctggggttga aggatccact tccaagatgg cgcactcagt ggctcttggc 22420
tggaggcctc agttccctgc tgcgtggagc tctccctcca gctgcttgag tggactcatg 22480
acatgcagct ggcctcccct ggagcagtcg atccaacaat gagcatggcc atgaactagg 22540
ctcagaagcc actccctgtc gtctctacat tttcctatca gaagcaagtc attaaaagtc 22600
cagtgccact ccaggggaga cgaattaggc tctgccttct gaaaggatta tcacagaaga 22660
tgcggtccta tattcttttt ttaaaattat tctttttttt attttgtaga gatggggtct 22700
tggtatgttg cctaggccag tctggaattc ctgggctcaa acaatcctgt ctctgcctcc 22780
caaagtgttg ggattacagg catgagccac tgcacctggt catgtggtca tattttcttt 22840
ttcttttttt tttttttttg agacagagtc tctgtcgccc aggctggagt atggtggcgt 22900
gatctcagtt cactgcagcc tccgcctccc gggttcaagc gattctcctg cctcagcctc 22960
ctgagtagct gggattacag gcgcccgcca acatgcccag ctaatttttt tagtagagat 23020
ggggtttcac catgttagcc aggatggtct cgatctcctg atttggtgat ccgcccacct 23080
tggcctccca aagtttcaac catcgatcag aacttattga tgtacttatg tagctaggca 23140
cggtggcgcg tgcctgtaat cccagctact tggaagggtt aaggcaggag aatcgcttga 23200
acctgggagg cagaggttac agtgagtcaa gatcatacca ttgcactcca gtctgggcaa 23260
cagaatgaga ctctgtctca aaaacaaaaa acaaaccctt gtatgtgatt ttcctggata 23320

gcatctgtta catcttcaca aagataaaaa gtcagacttg gctgggcatg gtggctcaca 23380
cctgtaatcc cagcactgag aggctgaggc aggcagatca cttgaggtca ggaatttgag 23440
accaggctgg gcagcatggt gaaaccccgt ctctacaaaa aatacaaaaa ttagccgggt 23500
gtggtgtcac gcacctgtat tcccaagcta ctcaggaagc taaggcagga gaatcacttg 23560
aacccagagg tggaggtttg cagtgagttg agattgtgcc attgcactcc agcctgggcg 23620
acagagtgag actctgtgtc aaaaataaaa taaaataaaa ttttaaaaaa ggcagatttt 23680
tttttcttct tggtattgtt accttattat agtaataata agtgcatagt gcatgctgag 23740
ataagcaatc ataatttgtt attgcggccg ggcatggtgg ctccagccta taatcccagc 23800
actttggtca ggagttcaag gccagcctgg ccaatatagt gaaactccat ctctactaaa 23860
atacaagaaa ttacctgggc atggtggcag ttgctggtga tccccagcta cttgggaggc 23920
tgaggcagga gaatcgcttg aacctgggaa gcagaggttg cagtgagcca agattgcacc 23980
actgcactcc agcctgggtg acagagtgag actctgtctg aaaataataa taataataat 24040
ttgttattgc ttttattgcc ttagtttaca tagggaatca aagtttatac tttgatttat 24100
aaaagttgct ttgattctag ttcacagaac cagaatcttt catataaagg tattagaggg 24160
cccagtgtgg tggctcatgc ctgtaatccc agcatattgg gaggctgagg agggaggatc 24220
actttaggag tttgaggcca gcctaggcaa catagtgaga ccttgtctct acaaaaaatt 24280
ccaacattag ctgggcatgg tggcatgtgc ctgtagtccc atttatttgg ggggctgagg 24340
caggaggatc acttgagccc acgaggttca atccaggttg cagtaagcca tgatcctgcc 24400
actgcactcc agtttgggta acagagcgaa gctatgtctc aaaaaaagaa aaaaaaagta 24460
ttctaaatcc aaatttaata tataaaacta aatgcaggcc aagtgtggtg gcatatacct 24520
ataatcacaa cactttggga ggctgaggtg ggaggattgc ttgagcccaa gagttcaaga 24580
ccagcctagg taacacagta agaccccatc tctacaaaaa gtagaaaaat tagcctggca 24640
tggtggtgag tgcttttaat cccaactact tagggggctg agatgggaag attgcttgag 24700
cctcagagtt tgaggctgca gtgggccgtg atcgctccac tgatcgctct aaagtgagac 24760
cctgtctcaa aaaaaaagaa aatagaagaa aactaaatac attcaataag actttgatct 24820
cttttccaag gtgtaaatat attttgggaa attttccagt tactttgttc tcattttaat 24880
gtaataatct aagtcttggt tttctaagga aaagttttct cttattatat cttttgttaa 24940
tgtttctctc ccatttcttt tgatctgatc ttcagataca tgattatctt cactgctaaa 25000
tttgtgttct ctggcctcta catttataat ttctcataat tctttatcta agtatttctt 25060
ccctacctac tgaagaaaac tcaagttttc ttccacctta atgattatgc tgtgtctgtg 25120
agttttcttc atgactcttt acagtacaag tttttgtttt ttgttttttt aatggtcaga 25180
tggatagaac aacacaggtt ttgtttgttt tgttttaact tttaaaaaaa ttataataga 25240
taaagggtct cactacgttg tccaggctga tctcatactc ctgggctcaa gcaatccacc 25300
cacctctgcc tcccaaagtg ctgggattac agtcatgagc caacatgcct gggcagtaca 25360
ggtttttttt gagacggagt tttgttcttg ttgccgaggc tggagtgcaa tggcacaatc 25420
ttggctcacc acaaagtctg cctcccaggt tcaagtgatt ctcctgcctc agcctcctga 25480
gtagctggga ttacaggcat gtgccaccac gcccagctaa ttttgtattt ttagtagaga 25540
cggggtttca ccatgttggc caggctggtt tcgaactgct gacctcaggt gatctgccca 25600
cctcggcctc ccaaagtgct gggattacag gcatgagcca ccatgcccag ctgtagtaca 25660
ggttttaata tgctaaatac tcttcctttc tttattaatg tgcatggaag ttctaatatt 25720
tttttcccat accccagaga gtccatattt tggaatcaac aacactagcc tttgttgaca 25780
agtgtctctc ttgggttcct tctttgtgtc ctccactgaa ttttggggtt cataaaattt 25840

catttgttgt gcttgcttaa ttccctggga atcagactgt tcctgatcgg atgacatttc 25900
tggttaattc tttagttggc aggaaataga cacaggaaac gtggtcagtt tctgattctg 25960
gcgttgagag accctttctc cttttcctct ctctcag tg ggc gac aga tgc gaa 26014
al gly asp arg cys glu
5
aga aac gag ttc cag tgc caa gac ggg aaa tgc atc tcc tac aag tgg 26062
arg asn glu phe gln cys gln asp gly lys cys ile ser tyr lys trp
10 15 20
gtc tgc gat ggc agc gct gag tgc cag gat ggc tct gat gag tcc cag 26110
val cys asp gly ser ala glu cys gln asp gly ser asp glu ser gln
25 30 35
gag acg tgc t gtgagtcccc tttgggcatg atatgcattt attttgtaa 26160
glu thr cys l
40
tagagacagg gtctcgccat gttggccagg ctggtcttga atttctggtc tcaagtgatc 26220
cgctggcctc ggcctcccaa agtgctggga ttacaggcac cacgcctggc ctgtgacacg 26280
attcttaacc cctttttgat gatggcggct ggaaaagtgg ccagtggatt ttgatgtatt 26340
caatcatgaa ttaggaggtg gggagagaat gaattattgg agctttcctt aaagccatta 26400
aatggctcta ttgttttttc aattgatgtg aatttcacat aacatgaaat taaccagctc 26460
agtggcatta atacatctgc aatgctgtgt ggccaccacc tctatcttgt tccaaaactt 26520
tgcataacct aatgtctttt tttttttttt tttttgagac ggagtctcgt tccatcaccc 26580
aggctggagt gcagtggtgt gatctcagct cactgcaacc tccgcctccc aggttcacgc 26640
catcctcctg cctcagcctc ccgagtagct gggactacag gcaccctcca ccacatccgg 26700
ctaatttttt gtatctttag tagagatggg gtttcaccat gttagccggg atggtctcga 26760
tctcctgacc tcgtgatcca cctgcctccg cctcccaaag tgctggcatt acaggcgtga 26820
gccaccatgc ccggcctatt ttttttttta agagatggag tctaattctg ttgcccaggc 26880
tggagtccag tggtaccatc atacttcact gcagccttga cctcttgggc tcaagtgatt 26940
ctcttgcctc gaactcccaa agtattggga ttacaggtgt gagccaccgc actcagccta 27000
atgtccagtt tttaacaagc tccatttaaa tgccctccgt tttgacccat aaaggggtag 27060
gcttggccgg gcacaatggc ttgtgtctgt agtcccagct acttgggagg ctgaggcaga 27120
aaggcagaaa gattgcttta taaagcccag gagtttgagg gccacctggg tggcatagct 27180
agacctcatc tctaaaaaat aagtaataaa taaatatttg tttttgtttt tttctttttc 27240
ttttcttttt tttttttttt tgagacggag tcttgctctg ttgcccaggc tggagtgcag 27300
tggcgcgatc tcagctcact gcaagctgtg cctcctgggt tcatgccatt ctcctgcctc 27360
agcctcccga gtagctggga ctacaggcgc ccactaccac gcccagctaa ttttttgtat 27420
ttttagtaga gatggggttt caccacgtta gccaggatgg tctcaatctc ctgacctcgt 27480
gatccgccag ctttggcctc ccaaagtgtt gggattacag gcgtgagcca ctgagcccgc 27540
cccatatgta tgtatatata tattttttta aaatgggaga ccaggcatgg tggctcatgc 27600
ctagaatccc agcactttgg gaagctgagg taggcggatc acttgaggcc atgagtttga 27660
gaccagcctg ctcaacatga tgaaacttct atctctacta aaaaaaaaag tgggattagg 27720
tcaggcacgg tggctcacac ctgtaatccc agcactttca gaggccgagg caggaggatc 27780
atgaggtcag gagatcgaga ccatcctggc taacacggtg aaaccccgtc tctactaaaa 27840

aaatacaaaa aattagccag gcgtggtggc gggtgcctgt agtcccagct actcaggagg 27900
ctgaggcagg agaatggcgt gaacccggga ggcggagctt gcagtgagcc aagatcgtgc 27960
cactgtactc cagcctgggc gacagagcaa gactctgtct caaaaaaaaa aaaaaaagtg 28020
ggattgacat tctcttcaaa gttctggggt tttcctttgc aaagacagga ttggcaaggc 28080
cagtgggtct tttttgtgtg tgtgtgtgtg acggagtctc actctgccac ccaggctgga 28140
gtgcaatggc aggatctcgg ctcaccgcaa cctcctcctc ccaggttaaa gtgattctcc 28200
tgcctcagcc tcccgagtag ctgggactac aggtgcccgc caccacaccc aactaatttt 28210
tgtattttta gtagagacag ggtttcacta tattggccag gctggtcttg aacccctgac 28320
ctcacgtgat ccacccgcct tggcctccca aagtgctggg attacaggcg tgagccactg 28380
tgctcggcct cagtgggtct ttcctttgag tgacagttca atcctgtctc ttctgtag tg 28440
eu
tct gtc acc tgc aaa tcc ggg gac ttc agc tgt ggg ggc cgt gtc aac 28488
ser val thr cys lys ser gly asp phe ser cys gly gly arg val asn
45 50 55
cgc tgc att cct cag ttc tgg agg tgc gat ggc caa gtg gac tgc gac 28536
arg cys ile pro gln phe trp arg cys asp gly gln val asp cys asp
60 65 70 75
aac ggc tca gac gag caa ggc tgt c gtaagtgtgg ccctgccttt 28581
asn gly ser asp glu gln gly cys p
80
gctattgagc ctatctgagt cctggggagt ggtctgactt tgtctctacg gggtcctgct 28641
cgagctgcaa ggcagctgcc ccgaactggg ctccatctct tgggggctca taccaagcct 28701
cttccgccct tcaaatcccc ccttgaccag gaggcattac aaagtgggga tggtgctacc 28761
tcttcgggtt tgtcacgcac agtcagggag gctgtccctg ccgagggcta gccacctggc 28821
acacacactg gcaagccgct gtgattcccg ctggtcgtga tccccgtgat cctgtgatcc 28881
ccgccccgtg aggctgaaca catagtgacg cttgctagcc aagcctcaat gacccacgta 28941
acatgaaggg ggaaaagcca gaaagttctg ccaaggagca aggccaagaa tcccgaaggg 29001
aaatggactt tgaagctggg cgtcttcttg gctgtcttaa tacaagtggc acatccaaat 29061
ccaaaacccc gaaattcaaa gtcttgagca cccgaaattc tgaaacgtct tgagcactga 29121
cctttagaag gaaatgctta ttggagcatt ttggatttcg gatttttacc actgagtgtg 29181
gagtcctaat taggaaaaaa accaggctga ccgaaccaaa ggaaagcaat aaaagaaggc 29241
agatagggtc aggcacggtg gctcacccct gtaatcccag ccttttgaga ggctgaggcg 29301
ggtggatcac ttgaggtcag gagttcgaga gcagcctggc caacacggtg aaaccccatc 29361
tctactgaaa atacaaaaac tagccaggta tggtggcgtc tgcctgtaat cccagctact 29421
cgggaggctg agacaggaga atcacttgaa cctgggaggc agaggttgca gtgagccaat 29481
atcacgccat tgcactccag cctgggggac aagagcgaaa ttctgtctca aaaaaaaaga 29541
agaagaaggc cgacaaacta tgtaactctg cctttctcca tggtccagaa cacacagccc 29601
tcctgcgtaa ataactcctt atcttcctgc tcccagctat catcagacac ctcggctgat 29661
agaaaattgc aagttagctc actgcaacct cggcattata agtactgcac aaagccctct 29721
tcagcgcaca gcacaagcac cattctataa aatctccagc aagcggccag gtgcagtggc 29781
tcatacctgt aatcccagca ttttgggaga ctgaggcggg cggatcacct gaggtcagga 29841
gtttgagacc agcctggcca acatggtgaa accccgtctc tattaaaaat acaaaaaaat 29901

tagccaggcg tggtggcagg tgcctgtaat cccagctact tggaaggctg aggcaggaga 29961
atcgcttgaa cccgggaggt ggaagttgca gtgagccgag atcttgccat cgcactccag 30021
cctgggggac aagagtgaga cttcgtctca aaaaaaaaaa aaaaaattcc cagcaagcct 30081
ttgtcttctg gcagtcagct cctctcttgc tgacctgctc attgctttct tgcaaggtat 30141
tttcctacct actttctgga ataaatctgt ctttctgtac ttacaactac cttttttaaa 30201
atttctttct tttttgagat ggagtctcac tctgtttgcc caggctggag ttcagtggtg 30261
caatctcagc tcactgcaac ctctacctac tgggttcaag cgattctcct gcctcagctt 30321
cccgagtagc tgggattaca ggcgtgcacc agcacgcagg ctaatttttg tatttttagt 30381
agagacgggg tttcaccatg ttggccaagg tggtcttgaa ctcctgacct caagtgatcc 30441
tcccacctca gcctcccaaa gcgctaggat tacggccatg agccactgag gccggctgca 30501
cctacaactg tcttgataaa ttcttacccc cacaccactg gtccagatag tcagtgctca 30561
cccacaacat taaggatatt ccaaatttga aacattccaa aatcagaaaa atattccaac 30621
tctgaaaata ttccaaaatc caaaaaaatt caaaatccaa aacacttctg gtcccaagca 30681
ttttagagaa gggatactca acccaaaata aggacagcaa ttctataaat tgtgctacca 30741
tcttgcaggt ctcagtttaa cagctttaca cctattagcg caccagtgct catagcagtg 30801
ctgggaaatg tgtacagatg aggaaactga ggcaccgaga gggcagtggt tcagagtcca 39861
tggcccctga ctgctcccca gcccgccttt ccaggggcct ggcctcactg cggcagcgtc 30921
cccggctata gaatgggctg gtgttgggag acttcacacg gtgatggtgg tctcggccca 30981
tccatccctg cag cc ccc aag acg tgc tcc cag gac gag ttt cgc tgc 31029
ro pro lys thr cys ser gln asp glu phe arg cys
85 90 95
cac gat ggg aag tgc atc tct cgg cag ttc gtc tgt gac tca gac cgg 31077
his asp gly lys cys ile ser arg gln phe val cys asp ser asp arg
100 105 110
gac tgc ttg gac ggc tca gac gag gcc tcc tgc ccg gtg ctc acc tgt 31125
asp cys leu asp gly ser asp glu ala ser cys pro val leu thr cys
115 120 125
ggt ccc gcc agc ttc cag tgc aac agc tcc acc tgc atc ccc cag ctg 31173
gly pro ala ser phe gln cys asn ser ser thr cys ile pro gln leu
130 135 140
tgg gcc tgc gac aac gac ccc gac tgc gaa gat ggc tcg gat gag tgg 31221
trp ala cys asp asn asp pro asp cys glu asp gly ser asp glu trp
145 150 155
ccg cag cgc tgt agg ggt ctt tac gtg ttc caa ggg gac agt agc ccc 31269
pro gln arg cys arg gly leu tyr val phe gln gly asp ser ser pro
160 165 170 175
tgc tcg gcc ttc gag ttc cac tgc cta agt ggc gag tgc atc cac tcc 31317
cys ser ala phe glu phe his cys leu ser gly glu cys ile his ser
180 185 190

agc tgg cgc tgt gat ggt ggc ccc gac tgc aag gac aaa tct gac gag 31365
ser trp arg cys asp gly gly pro asp cys lys asp lys ser asp glu
195 200 205
gaa aac tgc g gtatgggcgg ggccagggtg ggggcggggc gtcctatcac 31415
glu asn cys a
210
ctgtccctgg gctcccccag gtgtgggaca tgcagtgatt taggtgccga agtggatttc 31475
caacaacatg ccaagaaagt attcccattt catgtttgtt tctttttttt cttttctttc 31535
tttattttgt ttttgagatg gagtctcact ctgtgatttt tttcatctct aaatttccta 31595
catccatatg gccaccatga ggccccaggc tggccgatgg ttgctgttag cttattggga 31655
aatcactgtt tggaaggtgc tggttgtttt ttgttgtttg ttgtttttgt ttttgttttt 31715
gttttgagac ggagtctcgc tctgtcgcca gggtggagtg cagtggcgcg atcagctcac 31775
tgcaacctcc gcttcctggg ttcaagccat tctcctgcct cagcctccca agtagcgcgg 31835
attacaggca tgtgccacca cctccggcta tttttttttc tatttagtag agatggggtt 31895
tcaccatgtt agtcaggctg gtcatgaact cttgacctca ggtgatccac ccgcctcggc 31955
ctcccaaagt gctgggatta caggcgtgca ctgctgcacc cagccttttt ttgttttttt 32015
gagacagggt cttgctgtca cccaggttga agtaaggtgg cacgattatg gctcactgcg 32075
gccttgatct ccttggctca agcgatcctc tcacttcagc ctctcaagca gttggaacca 32135
caggctgtac caccaagcct ggccaatttt tttgtacaga cacaggctgg tcttgaactc 32195
ctgggctcaa gcaatcctcc tgccttggcc tcccaaagtg ctgggattcc aggcatgagc 32255
cgctgcaccc ggcaaaaggc cctgcttctt tttctctggt tgtctcttct tgagaaaatc 32315
aacacactct gtcctgtttt ccag ct gtg gcc acc tgt cgc cct gac gaa 32365
la val ala thr cys arg pro asp glu
215
ttc cag tgc tct gat gga aac tgc atc cat ggc agc cgg cag tgt gac 32413
phe gln cys ser asp gly asn cys ile his gly ser arg gln cys asp
220 225 230 235
cgg gaa tat gac tgc aag gac atg agc gat gaa gtt ggc tgc gtt aat 32461
arg glu tyr asp cys lys asp met ser asp glu val gly cys val asn
240 245 250
g gtgagcgctg gccatctggt tttccatccc ccattctctg tgccttgctg 32512
v
cttgcaaatg atttgtgaag ccagagggcg cttccctggt cagctctgca ccagctgtgc 32572
gtctgtgggc aagtgacttg acttctcaga gcctcacttc cttttgtttt gagacggagt 32632
ctcgctctga cacccaggct ggagtgctgt ggcacaatca cagctcacgg cagcctctgc 32692
ctctgatgtc cagtgattct cctgcctcag cctcccgagt agctgagatt aaaggcgtat 32752
accaccacgc ccggctaatt ttttgtattt ttattagaga cagggtttct ccatgttggc 32812
caggctggtc ttgaactcct ggtctcaggt gatccacccg cctcggcctc ccaaagtgct 32872
aggattacag gtgtgagcca ctgcgccagg cctaattttt ttgtattttt agtagagatg 32932
cggttttgcc atattgccca ggctggtctc gaactcctgg gctcaagcga tctgcctgcc 32992
ttggcctccc aaagtgctgg gattacaggc acaaaccacc gtgcccgacg cgttttctta 33052
atgaatccat ttgcatgcgt tcttatgtga ataaactatt atatgaatga gtgccaagca 33112

aactgaggct cagacacacc tgaccttcct ccttcctctc tctggctctc acag tg aca 33271
al thr
ctc tgc gag gga ccc aac aag ttc aag tgt cac agc ggc gaa tgc atc 33219
leu cys glu gly pro asn lys phe lys cys his ser gly glu cys ile
255 260 265
acc ctg gac aaa gtc tgc aac atg gct aga gac tgc cgg gac tgg tca 33267
thr leu asp lys val cys asn met ala arg asp cys arg asp trp ser
270 275 280 285
gat gaa ccc atc aaa gag tgc g gtgagtctcg gtgcaggcgg cttgcagagt 33319
asp glu pro ile lys glu cys g
290
ttgtggggag ccaggaaagg gactgagaca tgagtgctgt agggttttgg gaactccact 33379
ctgcccaccc tgtgcaaagg gctccttttt tcattttgag acagtctcgc acggtcgccc 33439
aggctggagc gcaatggcgc gatcttggct caccacaacc tccggctccc aggttcaagc 33499
gattcttctg cctcagcctc ctgagtagct gggattacag ctgaatgcca ccttgctggg 33559
ctaatttttg tatttttagt agagatgggg tttcaccatg ttggccaggc tggcctcgaa 33619
ctcctgacct cgagtgatct gcccgcctcc tgaagtgctg ggattacagg cgtgagccac 33679
ctcgtcctgg tgagggtttt tttttttccc caaccctctg tggtggatac tgaaagacca 33739
tattaggata actgtacagt atagagaagg cagtggcaag ttttctctgt catataccag 33799
agtgggcttg ggcatggtgg catactcctg tagtctcagc taatcaggag gctgaggaag 33859
gaggatcgct tgggcccagg agttggagac tgtagtgagc tgtgatcaca ccaccacact 33919
tcaatctggg caacagagca agagacccta tctctaaaaa aaagtaagta tttcggacac 33979
tgtgggccat acggtctctg gtgcagtttc tcaacatggc tgttgggtga acacaaccac 34039
gcacagaacg caaaccaata cacgtggctg tgggcccaga aaatgttatt tatggacacc 34099
aaaattggaa tttcatataa ctgttttgtg tcatgaaaat gatttccctt tttattttta 34159
tttttcttct caagtattta aatatgtaaa agccattttt aggcctggca ggatggttca 34219
cagctgtaat cccagcactt tgggaggtcg aggcgggagg atcacgaggt caggagatcg 34279
agaccatcct ggccaacaca gtgaaacccc gtctctacta aaaatacaaa aaattaacca 34339
ggcttggtgg cgcgcgtctg tagtcccagc tgctcaggag gctgaggcag gagaatcgct 34399
tgaatgcagg aggcggaggt tgtagtgagc cgaggttgca ccactgcact ccagcctgag 34459
cgacagagtg agagtccgcc tcaaacaaaa aaatgtttgc ccatgctggt cttgaactcc 34519
tgggctcaag ctatctgcct gccttggtct cccaaagttc tgggattaca ggcatgagct 34579
acagcgcccg gacttttgtt gttttatatc tatatatcta tatataactt gttttatgta 34639
tatatataac ttgttttata tatatacata aactgcagta aaaaacatgt aacataaaat 34699
ttaccttctc aaaccttatt aagtgcacag ttctgtgcca ttagcaaatt cacactgttg 34759
tacaacatca caaccaccat ctccagaact tttttttttt tttttattct ttttgagaca 34819
gagtctcact cgtcgcacgg gctggagtgc agtggtgcga tctcggttca ctgcaacctc 34879
cacctaccag gttcaagcaa ttctcctgcc tcagccccct cagtagctgg gattacaggt 34939
gcccgtccta ccacgcccag ctaatttttg tattttcagt agagactgac tgggtttcac 34999
catgttggcc aggctggtct cgaactcctg acctcaagtg atcctcccac ctcagcctcc 35059
caaagtgctg ggaatacagg catgagccac tgcgcccggc cccagaactc ttttatcttc 35119
ccaaactgaa gctctgtccc catgaaacac tcactctcca tcccctcccc aactcctggc 35179

acccaccatt ctactttctg tccctatgaa tgtgatggct ctagggacct cctctgagtg 35239
gaatcagaca gcattttcct tttttgactg gcttatttca ctgagccaag tgcggtggca 35299
cacgcctgta atcccaaaac tttgggagac cgaggcgggc gcatcaccag aggacaggag 35359
nncgagacca gcccggccaa cagggggaaa ccccatcact agggagcctg cagaaagaaa 35419
gccaccacat ggcctgctgg agccacacaa tcccagcaaa acagggacgc taaacgtagg 35479
agaaacacac aaccccagga ggcggaggtc gcagtgagcc gagatcgtgc cattacactc 35539
cagcctgggc aacaagagtg aaactccgtc tctcctaaaa atacaaaaaa attagctggg 35599
catggtggca catgcctgta gtcccagcta cttgggaggc tgaggcagga gaatcacttg 35659
aacccgggag gtggaggttg taatgagcca aggttggcgg cgaagggatg ggtaggggcc 35719
cgagagtgac cagtctgcat cccctggccc tgcgcag gg acc aac gaa tgc ttg 35773
ly thr asn glu cys leu
295
gac aac aac ggc ggc tgt tcc cac gtc tgc aat gac ctt aag atc ggc 35821
asp asn asn gly gly cys ser his val cys asn asp leu lys ile gly
300 305 310
tac gag tgc ctg tgc ccc gac ggc ttc cag ctg gtg gcc cag cga aga 35869
tyr glu cys leu cys pro asp gly phe gln leu val ala gln arg arg
315 320 325 330
tgc gaa g gtgatttccg ggtgggactg agccctgggc cccctctgcg cttcctgaca 35926
cys glu a
tggcaaccaa acccctcatg cctcagtttc cccatctgtt aagtgtgctt gaaagcagtt 35986
aggagggttt catgagattc cacctgcatg gaaaactatc attggctggc cagagtttct 36046
tgcctctggg gattagtaat taagaaattt caggccgggt gcgtaatccc tgtaatccca 36106
acaccttggg acgccgaggc gggcagatca cctgaggtcg ggagttccag a[?]cagcctga 36166
ccaacatgga gaaaccccgt ctctactaaa aatacaaaat tagccgggct tggtggtgca 36226
tgcctataat cccagctact caggaggctg aggcaggaga atcacttgaa cctgggaggt 36286
ggaggttgtg gtgagccaag atcgtgccat tgcactccag cctgggcaac aagagtgaaa 36346
ctccatccaa aaaaaaaaga aaagaaaaga aaaaaaagaa aagaaatttc agctgacaca 36406
gcttcacact cttggttggg ttcccgtggt gaatgatgag gtcaggtgat gactggggat 36466
gacacctggc tgtttccttg attacatctc ccgagaggct gggctgtctc ctggctgcct 36526
tcgaaggtgt gggttttggc ctgggcccca tcgctccgtc tctagccatt ggggaagagc 36586
ctccccacca agcctctttc tctctcttcc ag at atc gat gag tgt cag gat 36638
sp ile asp glu cys gln asp
335
ccc gac acc tgc agc cag ctc tgc gtg aac ctg gag ggt ggc tac aag 36686
pro asp thr cys ser gln leu cys val asn leu glu gly gly tyr lys
340 345 350 355
tgc cag tgt gag gaa ggc ttc cag ctg gac ccc cac acg aag gcc tgc 36734
cys gln cys glu glu gly phe gln leu asp pro his thr lys ala cys
360 365 370

aag gct gtg g gtgagcacgg gaaggcggcg ggtggggcg gcctcacccc 36784
lys ala val g
375
ttgcaggcag cagtggtggg ggagtttcat cctctgaact ttgcacagac tcatatcccc 36844
tgaccgggag gctgtttgct cctgagggct ctggcagggg agtctgccgc cctgttagga 36904
cttgggcttg ccagggggat gcctgcatat gtcctagttt ttgggaatat ccagttaacg 36964
gaaccctcag ccctactggt ggaacaggaa ccggctttcc tttcagggac aacctgggga 37024
gtgacttcaa ggggttaaag aaaaaaaatt agctgggcat ggtgccacac acctgtggtc 37084
ccagctactc agaaggctga ggcgggagga ttgcttgagg gcaggaggat tggttgatcc 37144
tcccacctca gcctccggag tagctgggac ctcaggtgca tgccactatg cctggctaat 37204
tttctttttt cttttttttt ttttttcgag acggagtctc gctctgttgc ccaggctgga 37264
gtgcagtggc aggatctcgg ctcactgcaa gctccgcctc ccgggttcac gccattctcc 37324
tgcctcagcc tccccagtag ctgggactac aggagcccgc cactgcacca ggccaatttt 37384
tttgtatttt tagtagagac ggggtttcac tgtgttagcc aggatggtct cgatctcctg 37444
acttcgtgat ccgcccacct cggccttcca aagtgctcgg attacaggcg tgagccactg 37504
cgcccagccg ctaattttca tatttttagt aaaaacaggg tttcaccatg ttggccaggc 37564
tagtcttgaa ctcctgaacc caagtgatcc tcctgccttg gcctcccaaa gtgctgggat 37624
tacagacacc acacctggct attattattt tttagagaca gggtgctgct ctatcttcca 37684
gcctgtagtg cagtgcagcc tccatcatag ctcgctgcag ccttgacctc ctgggttcac 37744
gtgatcgtcc cgcctaagcc tctggaggag ctgggagtac tggcatgtgc caccatgcct 37804
ggttaatttt tttttttttt tttttgagac agagtctcat tctgtcaccc aggctggagt 37864
gcggtggtgc gatcttggct tactgaaacc tccacctccc aggttccagc aattctcctg 37924
cctcacccct ctgagtagct gggattacag gttccggcta ccaaacctgg ctagtttttg 37984
tatgtttagt agagacaggg tttcaccatg ttggtgaggc tggtctcgat tctcccgcct 38044
cagcctccca aagtgctggg attacaggct tgagccaccg tgcctggctt tttttttttt 38104
tttttttttt gtggcaataa ggtctcattg tcttgcccag gctagcctta tgctcctagc 38164
ctcaagtgat cctcctccct cagcctccca aagtgctggg attacaggtg ggcgccactg 38224
tgcctgttcc cgttgggagg tcttttccac cctctttttc tgggtgcctc ctctggctca 38284
gccgcaccct gcaggatgac acaaggggat ggggaggcac tcttggttcc atcgacgggt 38344
cccctctgac cccctgacct cgctccccgg accccccag gc tcc atc gcc tac ctc 38399
ly ser ile ala tyr leu
375 380
ttc ttc acc aac cgg cac gag gtc agg aag atg acg ctg gac cgg agc 38447
phe phe thr asn arg his glu val arg lys met thr leu asp arg ser
385 390 395
gag tac acc agc ctc atc ccc aac ctg agg aac gtg gtc gct ctg gac 38495
glu tyr thr ser leu ile pro asn leu arg asn val val ala leu asp
400 405 410
acg gag gtg gcc agc aat aga atc tac tgg tct gac ctg tcc cag aga 38543
thr glu val ala ser asn arg ile tyr trp ser asp leu ser gln arg
415 420 425

atg atc tgc ag gtgagcgtcg cccctgcctg cagccttggc ccgcaggtga 38594
met ile cys se
430
gatgagggct cctggcgctg atgcccttct ctcctcctgc ctcag c acc cag ctt 38649
r thr gln leu
435
gac aga gcc cac ggc gtc tct tcc tat gac acc gtc atc agc aga gac 38697
asp arg ala his gly val ser ser tyr asp thr val ile ser arg asp
440 445 450
atc cag gcc ccc gac ggg ctg gct gtg gac tgg atc cac agc aac atc 38745
ile gln ala pro asp gly leu ala val asp trp ile his ser asn ile
455 460 465
tac tgg acc gac tct gtc ctg ggc act gtc tct gtt gcg gat acc aag 38793
tyr trp thr asp ser val leu gly thr val ser val ala asp thr lys
470 475 480
ggc gtg aag agg aaa acg tta ttc agg gag aac ggc tcc aag cca agg 38841
gly val lys arg lys thr leu phe arg glu asn gly ser lys pro arg
485 490 495
gcc atc gtg gtg gat cct gtt cat gg gtgcgtatcc acgacgctga 38887
ala ile val val asp pro val his gl
500 505
gggctgcaga gggaatggag ggagcaggaa ggagcttcag gaactggtta gtgggctggg 38947
catggtggct caaagcacct gtaatcccag cactttggga ggccaaggtg ggtggatcat 39007
caagaccagc ctgaccaaca tggtgaaacc tcgtctctac taaaaataca aaaattagcc 39067
gggtgtggtg gtgggcacct gtaatcccag ctgctcggga ggctgaggca ggagaatcac 39127
ttgaacctgg gagatggagg ttgcagtgag ccaagacagc cccactgcac tccagcctgg 39187
gtgacagagt gagactccgt ctcaaaaaaa aaaaaaaaaa ctaaacaaaa aactggttag 39247
tggctagaca acaggatggt atcttccaag cccatggctg actcagcagc tcctgggtca 39307
agacactgtg acctgtgtcc cctggcagga agcatcgccc ctgccacctg cccggtgtac 39367
tctgtacctg tcaggtgaca tctgctacct aagcacgtga gaggtggcat ttcacagttt 39427
cagtgtggtg ctgacaaccc gggacgcaca ctgtccttgc agctacaatc aggaggtgaa 39487
tgttgggttt ccagcagaga acactggaga aggcacactt ggtgtctgga agggaaaagc 39547
agggaagaga gcatcatcag atgcctgcgg gtgaaggtgg gcccgctatg gccagcgtcc 39607
ctttttattt ttatttattt atttatttga gatggaatct cgctctgtcg cccagactgt 39667
agtgcagtgg tgcgatcacg gctcactgca agctccgcct cacaggttca cgccattctc 39727
ctgcctcagc ctcccgagta gctgggacta caggcacccg ccaccacgcc cggttaattt 39787
tttgcatttt tattagagac ggggtttcac cgcgttagcc aggatggtct aaatctcctg 39847
accctgtgat ccacccgcct cggcctccct aagtgcttgg attacaagcg tgagccacca 39907
cgcccggccc cctttttatt ttttattttt tgagacggag tctcgctctg tcgcccaggc 39967
tagattgcag tggcgtgatc tcggctcact gcagcctccg cctcccaggt tcaagtgatt 40027
ctcctgcctc aacctcccaa ctaattagga ttacaagcat gtaccaccat gcctgactaa 40087
ttttttgtat ttttagtaga gactgggttt caccatgttg gctaggctgg tctcgaaccc 40147

ttagcctcaa gtaatctgcc tgcctcagcc tcccaaacag cggggattac aggcatgagc 40207
cactgtgccc aacccaaccc tggatctctt ttaaacaaga caatgctcgc tgttgccaca 40267
gaacaatggg tggggtacat gtggcccagt gtgtttggcc acataactgc caggccagag 40327
ggaaagagac tctcagactg tctccactca gatacaaatg tgtgtgttgt gtgcgtgtgt 40387
tctggtctca tatttgtttg ttttgagaca gggtgtcgct ctgtcactga gtctggagtg 40447
cagtggcgca atcagagttc actgcagcct caaactcttg ggctcagttg attctcccac 40507
ttcagcctcc caagtagctg gaactacagg tgaacaccac tgtgcccagc taatttattt 40567
tatttttagt agagatgagg tctcactatg ttgcccaggc tggtcttgac ctcctagcct 40627
caagcaatcc tcctgccttg gtctcccaaa gtgctgggat tacacgtgcg agccattgcg 40687
catggcttgt gttcttgtgt ttcttccttt ttctttcgag atggcgtctc agtctgccac 40747
ccaggctgga gtgcagtggt gtgatcatag ctcactgtag cctcaacttc ctgggctcaa 40807
gcaatcctct tgatttcagc ctcccgggcc tggccagcat ggtgaaaccc cgtctctact 40867
aaaaatacaa aaatgtagcc aggcgtggtg gtgggcgcct gtaatcccag ctacaccaga 40927
ggctgaggca ggagaatcgc ttgagcctgg aaggtggagg ttgcagcaag ccaagatcgt 40987
gccactgcac tccagcctgg gcaacagaga cagactctgt ctcaaaaaaa aaaaaaaaaa 41047
acccaaacaa gccacatttg gagtttgggg ttcccagcag gactatttcc caagcctgag 41107
cctggctgtt tcttccagaa ttcgttgcac gcattggctg ggatcctccc ccgccctcca 41167
gcctcacagc tattctctgt cctcccacca g c ttc atg tac tgg act gac tgg 41220
y phe met tyr trp thr asp trp
510 515
gga act ccc gcc aag atc aag aaa ggg ggc ctg aat ggt gtg gac atc 41268
gly thr pro ala lys ile lys lys gly gly leu asn gly val asp ile
520 525 530
tac tcg ctg gtg act gaa aac att cag tgg ccc aat ggc atc acc cta 41316
tyr ser leu val thr glu asn ile gln trp pro asn gly ile thr leu
535 540 545
g gtatgttcgc aggacagccg tcccagccag ggccgggcac aggctggagg 41367
a
acagacgggg gttgccaggt ggctctggga caagcccaag ctgctccctg aaggtttccc 41427
tctttctttt ctttgttttt tctttttttg agatgaggtc ttggtctgtc acccaggctg 41487
gagtgcactg gcgcaatcgt agctcactgc agcctccacc tcccaggctc aagtgatcct 41547
cctgcctcac cctcctgagt agctgagatt acagacacgt gccaccacgg cagactaatt 41607
ttattttatt tttgggaaga gacaaagtct tgttatgttg gcctggctgg tctcaaactc 41667
agggtgcaag cgatcctccc gcctcagcct tccaaactgc tgggattaca ggcgtgggcc 41727
accgtaccca gcctccttga agtttttctg acctgcaact cccctacctg cccattggag 41787
agggcgtcac aggggagggg ttcaggctca catgtggttg gagctgcctc tccaggtgct 41847
tttctgctag gtccctggca gggggtcttc ctgcccggag cagcgtggcc aggccctcag 41907
gaccctctgg gactggcatc agcacgtgac ctctccttat ccacttgtgt gtctag 41963
at ctc ctc agt ggc cgc ctc tac tgg gtt gac tcc aaa ctt cac tcc 42010
sp leu leu ser gly arg leu tyr trp val asp ser lys leu his ser
550 555 560

atc tca agc atc gat gtc aac ggg ggc aac cgg aag acc atc ttg gag 42058
ile ser ser ile asp val asn gly gly asn arg lys thr ile leu glu
565 570 575
gat gaa aag agg ctg gcc cac ccc ttc tcc ttg gcc gtc ttt gag 42103
asp glu lys arg leu ala his pro phe ser leu ala val phe glu
580 585 590
gtgtggctta cgtacgagat gcaagcactt aggtggcgga tagacacaga ctatagatca 42163
ctcaagccaa gatgaacgca gaaaactggt tgtgactagg aggaggtctt agacctgagt 42223
tatttctatt ttcttctttc tttttttttt tttttttgag acagagtttt gctctcgttt 42283
cccaggctgg agggcaatgg catgatctcg gctcaccgca acctccacct cccaggttca 42343
agtgattctc ctgtctcagg ctccccagta gctgggatta caggcatgca ccaccaccat 42403
gcccggctaa ttttgtattt ttagtagaga cggagtttct ccatgttggt caggctggtc 42463
tcgaactccc gacctcaggt gatctgcctg cctcggcctc ccaaagtgct gggattacag 42523
acttgagcca ccgcgcccag ctatttctgt ttctttcttt tcttcttctt cttttttttt 42583
ttctaagaga caggatctca ctctgtcccc aggcaggagt gcagtgctgt gatcatagct 42643
cactgcagcc ttaacctcct gggctcaagt gatcttccca cctcagcctc ccaagtagct 42703
ggaactacag gtgcacacca ccatgcccag ctcatttttg tatttttttt ttttttgaga 42763
cagtctcgtt ctgtcacccc ggctggagtg cagtggtaca atcttggctc actgcaacct 42823
ctgcctccca ggttcaagcg attctcctgc ctcagcctcc tgagtagttg agattacagg 42883
catgtgtgcc atcatacctg gctgattttt gtattttttt ttagagatgg ggtctcagta 42943
tgttgaccag gcttgtctta aactcccggc ctcaagtgat cctcccactt cagtctccca 43003
aagtgctggg attacaggca tgagccactg cggccggttt gttttctttt ttttttcgtt 43063
ttttggagac ggaatttcac ctttgttgcc caggatggag tgcaatggca cgatatcgcc 43123
tcaccacaac ctctgcctcc tgggttcaaa ccattttcct gcctcagcct tcttagtagc 43183
tgggattaca agcatgtgcc accacgcccg gctgattttg tatttttagt agagatgggg 43243
tttctccatg ttggccaggc tggtctcgaa ctcctgacct caggtcattc gcccacctct 43303
gcctcccaaa gtgctgggat tacaggcgtg agccaccgtg cccggtggtt tgtattcttt 43363
ttactgagag tcgtgaaagg cagtgatcct ctgtcacatg tgatcttggc tctcagggga 43423
catttggcaa tttctagaga ttttttggtt gtcacaagtc aatggggaag actgttggca 43483
tttagtgggt agaggctggt gacgctgctg aacacccaga acagggaagt agcaggccct 43543
agatagagcc atcgtgggga aaccctgctc taaggaaatg gcgctatttt ataaccccac 43603
gttcctggca tgattaccaa cagccaaaag tggagtcccc ccaagtgtgt tcgtccattt 43663
gcattgcagt aaaggaatag ctgaggccgg gtaatttata aagaaaagag atttaaactg 43723
ggtatggcag tttatgccta taatcccaga actttgggag gctgaggcag gaggatcgct 43783
tgagtccagg agtgtgagac cgagaccagc ctggccaaca tgacgaaact ctgtctctac 43843
aaaaaataca aaaagtaggc caggcacggt ggttcacgcc tgtaatccca gcactttggg 43903
aggccgaggc gggcggatca cgaggtcagg agatcgagac catcctggct aacacggtga 43963
aaccccgtct ctactaaaaa tacaaaaaca aaattagccg ggtgtggtgg caggcgcctg 44023
tagtcccagc tactcgggag gctgaggcgg gagaatggcg tgaacccggg aggcggagct 44083
tgcagtgagc caagatcgcg ccactgcact ccagcctggg tgaccgagtt gagactccgt 44143
ctcaaaaaaa aaaaaaaaaa aaaaaataca aaaagtagcc aggtgtggtg gcaggcacct 44203
gtaatcctgg gttctcgaga ccgaggcatg agaattgcct gaccccagga ggtggaggct 44263

gcagtgagcc aagatcatgc cactgcactc cagcctgggc gacagagtgg gactctgtct 44323
caaaaaacaa caaaaaaaaa gttctggaaa tggatggtgg tgatggtgat acttccacaa 44383
cagcgtgaat ctgcttaagg ccaccgaact gtgcactcac aaatagtcga gatggtacat 44443
tttatgttat gtgtatttca ccacaattaa aaactagttg tgggccaggt gtggtggttc 44503
atgcctgtaa tcccagcact ttgggaggtc agagggaggt ggatcatgag gtcagcagtt 44563
cgagaccagc caggccaaca tggtgaaacc ccatctctac taaaaataca aaaattagcc 44623
aggcgtggtg gcacatgcct gtagtcccag ctacttgaga ggctgaagca ggagaatcgc 44683
ttgaacctgg gaggctaaga ttgcagtgag ccgagatcgt gccactgcac tccagcctgg 44743
acgacagagt gagacttcgt ctcaaaaaaa aaaccaaaaa aaaaattagc tgtgggtcag 44803
gcactgtggc tcacgcctgt aatcccagca ctttgggaga ccgaggtagg tggatggcct 44863
gaggtcagga gttcgaatcc agcctggcca acatggtgaa agcccgtctc tactaaaaat 44923
acaaaaaatt agtcaggtat gttggcacac ctgtaatccc agctactcgg gaggctgaag 44983
caagagaatc gtttgaaccc aggaggtgga cgttgcagtg agccgagatt gggccactgt 45043
actccagcct gggcaacaaa agtgaaactc tgtctgaaac aaacaaacaa acaaacaaac 45103
agacaaacaa aaaaactagt tgtggagaga gggtggcctg tgtctcatcc cagtgtttaa 45163
cgggatttgt catcttcctt gctgcctgtt tag gac aaa gta ttt tgg aca gat 45217
asp lys val phe trp thr asp
595 600
atc atc aac gaa gcc att ttc agt gcc aac cgc ctc aca ggt tcc gat 45265
ile ile asn glu ala ile phe ser ala asn arg leu thr gly ser asp
605 610 615
gtc aac ttg ttg gct gaa aac cta ctg tcc cca gag gat atg gtt ctc 45313
val asn leu leu ala glu asn leu leu ser pro glu asp met val leu
620 625 630
ttc cac aac ctc acc cag cca aga g gtaagggtgg gtcagcccca 45358
phe his asn leu thr gln pro arg g
635 640
cccccccaac cttgaaacct ccttgtggaa actctggaat gttctggaaa tttctggaat 45418
cttctggtat agctgatgat ctcgttcctg ccctgactcc gcttcttctg ccccag 45474
ga gtg aac tgg tgt gag agg acc acc ctg agc aat ggc ggc tgc cag 45521
ly val asn trp cys glu arg thr thr leu ser asn gly gly cys gln
645 650 655
tat ctg tgc ctc cct gcc ccg cag atc aac ccc cac tcg ccc aag ttt 45569
tyr leu cys leu pro ala pro gln ile asn pro his ser pro lys phe
660 665 670
acc tgc gcc tgc ccg gac ggc atg ctg ctg gcc agg gac atg agg agc 45617
thr cys ala cys pro asp gly met leu leu ala arg asp met arg ser
675 680 685
tgc ctc aca g gtgtggcaca cgccttgttt ctgcgtcctg tgtcctccaa 45667
cys leu thr g
690
ctgccccctc ctgagcctct ctctgctcat ctgtcaaatg ggtacctcaa ggtcgttgta 45727

aggactcatg agtcgggata accatacttt tcttggatgg acacatcagc accgggcttg 45787
acatttaccc agttcccctt tgatgcctgg tttcctcttt cccggccccc tgaagaggtg 45847
atctgatttc tgacaggagc cctgagggag gaaatggtcc cctttgttga cttttctttt 45907
tctttatttt tttcttttga gatttgctgt cacccagcct ggaatgcagt ggtgccatct 45967
tggctcactg ctacctctcc cactgggttc aagcaattct cctgcctcag cctcccaagt 46027
agctgggatt acaagcatgc gccaccatgc ctggctaagt tttgtatttt tagtacagac 46087
agggtttctc catggtggcc aggctggtct tgaactcctg acctcaggtg atcctcccac 46147
ctctgcctcc cgaagtgcta cgattacagg catgagccac cgcgcccatc cccctttgtt 46207
gacttttctc atcctctgag aaagtctcag ttgaggccag cacctccctc aagtgaattg 46267
aatctccctt ttgaacaaca acaaataaca atatgaccca gacgtggtgg ctcacacctg 46327
tggtcccagc tactcgggag gctgaggtgt gaggattgct tgagcccagg aggtcaaggc 46387
tacagagagc tataatcaca ccacttcact ccagcctggg ggacaaagtg aaaccctgtc 46447
tgaaaaaaac aaaaaaagaa aaaggaaaaa gaaacaatac gatcacaaag tagatattca 46507
tagtgtttat tttcagtact cttttttttt tttttttttt tttttgagac ggagtcttgc 46567
tctgttgccc aggctggagt gcagtggcac gatcttggct cactgcagcc tctgcctccc 46627
aggttcaagc gcttggctca ctgcaacctc cgcctcctgg gttcaagcgc ttcttctgcc 46687
tcagcctccc cagtagctgg gactataggc acgtcccact acgcccagct aattttttgt 46747
attttttagt agagatgggg tttcactatg ttagccagga tggtctcgat ctcctgacct 46807
cgtgatctgc ctgccttggg ctcccaaagt gttgggatta tgggcatgag ccactgcacc 46867
tggccttttt tttttttttt tttgagatgg agtttcgctc ttgttgccca ggctggagtg 46927
caatggtgtg atctcggctc actgcaacct ctgcctcctg ggttcaagca attctcctgc 46987
ctcagcctcc cgagtagctg ggattacagg cacctgccac cacgcctggc taatttttgt 47047
acttttagta gagacggggt ttctccatgt tggtcaggct ggtctcaaac tcctgacctc 47107
aggtgatcca cccacctcgg cctcccaaag ttctgggatt acagacatga gc[?]accg[?]g[?] 47167
ctggccgtgt ctggcctttt ttagttattt cttttttttt tttttttttt tttgagacag 47227
agtcttactc cgtcgcccag gctggagtgc agcggtgcga tgtctgcgca ctgcaagctc 47287
cgccccctgg gttcatgcca ttctcctgcc tcagccttct gagtagctgg gactgcaggc 47347
gcctgccact acgcccggct acttttttgt atatttagta gagatggagt ttcactgtgt 47407
tagccaggat ggtctcgatc tcctgacttt gtgatccgcc cgcctcggcc tcccaaagtg 47467
ctgggattac aggcgtgagc caccatgcca ggcttttttt tttttttttt tttttgagac 47527
ggagtcttgc tctgtcgccc aggctggagt gcagtgccat gatctcagct cactgcaagc 47587
tccacttccc aggctcacgc cattctccag cctcagcctc ccaagtagct gagactacag 47647
gggcccgcca ccacactcgg ctaatttttt tgtattttta gtagagacgg ggtttcacca 47707
tgttagccag gctggtcttg aactcctaac ctcaggcgat tcacctgcct cggcctccca 47767
aagtgctggg attaaaggta tgagccacct cgcctggtgt gagccacctc gcccagcctg 47827
agccacctca cccagcctaa gccactgtgc ctggcctgat tttggacttt ttaaaaattt 47887
tattaataat tatttttggg tttctttttt ttgagacagg gtcttactct gtcatccagg 47947
ccatcctgtc tgtctgtcat cccagtgatg ggatcatacc ttgctgcagc ctctacctcc 48007
tgggctcaag cgatcctccc ccctcagcct cctgagtagc tgggagtaca ggtgtgcacc 48067
accacacctg gctaattttt tttttttttt ttgtatatag agatggtatt ttgccatgtt 48127
gaccaggcta gtcttaaact cctggactca ctcaagagat cctcctgcct tggcctccca 48187
aggtcatttg agactttcgt cattaggcgc acacctatga gaagggcctg caggcacgtg 48247

gcactcagaa gacgtttatt tattctttca g ag gct gag gct gca gtg gcc acc 48301
lu ala glu ala ala val ala thr
695 700
cag gag aca tcc acc gtc agg cta aag gtc agc tcc aca gcc gta agg 48349
gln glu thr ser thr val arg leu lys val ser ser thr ala val arg
705 710 715
aca cag cac aca acc acc cga cct gtt ccc gac acc tcc cgg ctg cct 48397
thr gln his thr thr thr arg pro val pro asp thr ser arg leu pro
720 725 730
ggg gcc acc cct ggg ctc acc acg gtg gag ata gtg aca atg tct cac 48445
gly ala thr pro gly leu thr thr val glu ile val thr met ser his
735 740 745
caa g gtaaagactg ggccctccct aggcccctct tcacccagag acgggtccct 48499
gln a
750
tcagtggcca cgaacatttt ggtcacgaga tggagtccag gtgtcgtcct cactcccttg 48559
ctgaccttct ctcacttggg ccgtgtgtct ctgggccctc agtttcccta tctgtaaagt 48619
gggtctaata acagttcttg ccctctttgc aaggattaaa tgggccaaat catatgaggg 48679
gccaggtcct tcaggctcct ggttcccaaa gtcagccacg caccgtgtgg gtcccaaaat 48739
tttatcaagg cacattcgtt gcctcagctt caggcatctg cccaaaaagg ccaggactaa 48799
ggcaaggaga gggagggatt cctcagtact cagcttttca cagaggctcc aaaaggctaa 48859
ggaatccagt aacgttttaa cacaatttta caattttttt ttttgagacg gagttttgct 48919
cttgttgccc aggctggagt gcagtggcac gatctcggct cactgcaacc tctggctccc 48979
gggttcaagc gattctcctg cctcagtctc ccgagtagct gggattacag gcatgcgcca 49039
ccacgctcgg ctaattttgt atttttagta cagaaggggc ttctctgttg gtcaggctgg 49099
tcgtgaactc tcaacctcag gtgagccacc cgcctgagcc tcccaaagtg ctgggattac 49159
aggtgtgagc caccacgcct ggcctttttt ttgagacaga gtctcgctct cgcccatgct 49219
gtactgcagt gacgcagtct gggctcactg taacctccgc ttcccaggtt caagtgattc 49279
ttctgccgca gcctcccatg tagagtagct gggattacag gcacccgcca ccatgcctgg 49339
ctaattcttg catttttagt agagatgggg tttcacagtg ttggccaggc tggtctcaaa 49399
cttctgacct caagtcatct gcctgccttg gccctgccaa agtgctggga ttatagatgt 49459
gagccaccgc gcctggccta cagtttattc tttggtggct cacacctgta atctcagcac 49519
tttgggaggc caaggtggga gaatggcttg agcccaggag ttcaagtcca gcctgggcaa 49579
catagcaaga ccctatctct actacaaaat aaataataaa taaactaatt ttttttcttt 49639
taaaacccaa ctattcaaca tggcaatgca atatattaaa aaaatttttt ttttctttga 49699
aacggagtct ctcactgtca cccgggctgg agtgcagtgt cgccatcttg gctcactgca 49759
acctccgcct cccaggtcca agtgattctc ctgcttcagc ctcccgagta gctgggatta 49819
caggcaccca ccaccatacc cagctaatat ttttgtattt ttagtagaga tggggtttca 49879
ctatgttggg caggctggtc tggaactcct gacctcgtga tctgcccgag gatcggcggc 49939
ctcccaaagt gctggggatt gcaggcatga gccaccgtgc ccagccaaaa cttttttatt 49999
tttatttttt tgggacacgg tctcactgtg taccccagac tggagtgata gagtgctgtc 50059
atggctcact gcagcctcaa cctccctggg ctcaggtgat cttcctgctt cagtctccca 50119

ggtagctggg actacaggca tgagccacca cacccagcta atttttgaat tttttgtag 50179
agacagggtt tcaccttgtg gcccagactt gtctctaact ccagggctca agcgatctgc 50239
ccaccttggc ctcccaaagt gctgagatta atgcaattta aaaaattttt tggccaggcc 50299
tggtggctca tgcctgtatt cacaacacct tgggaggcaa aggtgggcag atcacttgag 50359
gtcaggagtt cgagactagc ctggccaaca tggtgaaacc ccctgtctac taaaaaaata 50419
caaaaattac ctgggcacag tggtgggtgc ctgtaatccc agctacttgg gatgctgagg 50479
gtggagaatt gcttgaacct gggaggcaga agttgcagta agccaagatc atgccactgg 50539
actccagcct cagtgacaga gcaaaactct gtctccaaaa aaattgtttt tttttttttt 50599
ttttcaaatc atcacactac agccaaggcc tggccactta cttttgtaaa taaagtttta 50659
ttggagccag tggaccagtg aggccgaatc ttgcaggtgt aagatcacag tctatccttg 50719
aaaattttga tattttgttc attgggtggt ttttcattaa tttaaatttt aaaaaataac 50779
atattaaagg ctggtgtgga ggtgcacgcc tgcagtccta gctactccca gaggctgagg 50839
cgggagactt gcttgagccc aagagttgaa gtccagcctg ggcaacatag cgagaccccc 50899
atctctaaaa ataaaaataa tgcattagaa tattattgga ttcctgggca gggcacagtg 50959
gctcacacct gtaatcccag cactttggga ggctgaggtg ggtggatcac ctgaggtcag 51019
gagtttgaga ccagcctggc caacatggtg aaaccccgtc tctactaaaa atacaaaaat 51079
tagccaggcg tggtggcagg tgcctgtaat cccagctact cgggaggctg aagcacgaga 51139
atcgcttgaa tccaggaggc ggaggttgca gtgagctgag attgcgccat tgcactccag 51199
cctggaggac aagagtgaaa ctccattccc ctctgcaaag aaaaggaata ttatcagatt 51259
cctaagcttt ttggctcccc ctttagtttg ggggctgggg tggtgagtgt ctgacctggc 51319
ctcactgtcc tccctggatg tgatgagacc caggtgtggg tcaggatgtc attcgtttgt 51379
ccaccagagg gcgcccaaac tgctttgagc tgctgggaaa tggtgctcct agacttttag 51439
caaacaaaca aaaaaaaatg gcacatcggc aaatttcaga ccattctttt tttttttttt 51499
tttggttcca gagtagctga aatctttgtt cagttacaag caggataaaa tggaaactgc 51559
ctgggagagg ctgagaaacc ttcttgcttg ggggaggtgg ggcactgcta gaattaatcg 51619
cttcacagac cagcccatcc aggactcctc aaatttggca aaaaagccat tcattcattc 51579
attcatttat gtagagacga gggggatctg gctatattgc ctagattggt ctcaaattcc 51739
tggcctcaag tgatcctcct gccttggtct actaatgtgc tgcgattaca ggcatgagcc 51799
accgtgccta gctctagtgg acttgaaatg ttgccttgcc cagggccctt atgttgaatg 51859
gcccaggtcc acttgtatgg ttctgtacca aggttaaccc catcccataa tgcctgggac 51919
agttgatgca ggacaatcag cttctgtgcc attcaacctc aggactgagc atgctgggca 51979
ttgtggggtc cgaaggtggc tcccctgtcc ccttcaaaat accctctttt tcttttcttc 52039
tttttttttt tttttttttt ttgagacgaa gtcttgctct gttgccccag ctagagtgca 52099
gtggtgcgat ctcagctccc cgcaacctct gcttcccggg ttcaggcgat tctcctgcct 52159
cagcctcctg agtagctggg attacaggtg cccaccgcca cagctggcta atttttgtat 52219
ttttagtaga gacagggttt caccgtgttg gccaggctgg tcttgaactc ctgacctcag 52279
gcaacctgcc cacctcagcc tcccaaagtg ctgggattac aggtttgagc cactgggcct 52339
ggcctttttt tttttttttt gagagggagt ctcactctgt tgcccaggct ggagtgcaat 52399
ggcgcgatct tgactcactg caactccatt tcccgggttc aagtgattct cctccctcag 52459
cctcccaagt agctgggatt acaggtgcat gccaccacgg ccagctaatt ttgtattttt 52519
agtagagaca gggtttcact atgttgatca tgctggtctc aaactcctga ccttaggtga 52579
tctgcccgcc ttagcctccc aaagtgttgg gattacaggt gtgagccacc gcgcccagac 52639

caaaatatgc tcattttaat aaaatgcaca agtaggttga caagaatttc acctgcaacc 52699
ttgtcaacca cctagaataa aagcctctgc agccctcccc taaagactca tcaatgtgag 52759
gctcaagaac cttcttaggc tgggctcggt ggctcatttc tgtaatccct gcactttgga 52819
aggctgaggc aggaggatct cttgaggcca ggagttcaag acaagcctgg gcaacatagc 52879
cagacctctg tttctatccc ccacaaaaag aaccttctta aaccggaatt gagtcctaca 52939
acctcgataa ctcacaaata agcccgtgtg gcctctcaca gacttgggaa gttctccaag 52999
tgtccaggga gatgtgccag gcgctttcct gccgtgacca ccgtcctctg cctgctccat 53059
ttcttggtgg ccttccttta gacctgggcc tcactcttgc ttctctcctg cag ct ctg 53117
la leu
750
ggc gac gtt gct ggc aga gga aat gag aag aag ccc agt agc gtg agg 53165
gly asp val ala gly arg gly asn glu lys lys pro ser ser val arg
755 760 765
gct ctg tcc att gtc ctc ccc atc g gtaagcgcgg gccggtcccc 53210
ala leu ser ile val leu pro ile v
770 775
cagcgtcccc caggtcacag cctcccgcta tgtgacctcg tgcctggctg gttgggcctg 53270
ttcacttttt ctcctggaca gggaacagcc ccactggtgt cctttatcac ccccacggcc 53330
tctcctggct tggggctgac agtgacaaga tcagacagct aaggggtcag atggaggatg 53390
tggagctggg tcccgtgctg tggaatagcc tcaccgagat ttgagtgcct tctggggaac 53450
tggttccctt gcagggggct gtgtggagag gcgcgctctc cctgcctcac ccatgctcat 53510
cctaactcgg ttaccatcac atctcttttt tctttttttc ttaaatttta agaaaaaaga 53570
aatttaattt ttttgagaga cagagtcttg ctctgtcacc caggctggag tgcagtggca 53630
ccatcatgcc tcgctgcagc ctcaatgtct gggctcaagc gatcctccca cctcagcctc 53690
ctgagtagct ggtgcaagcc actatacccc acttcctatt tcttaaaaag tcacagccct 53750
gtgtgtggct aatcctggac agaaatctag aagaagtcag ctacttctgg ggcgtggctc 53810
acccagtggg cttcaggtta gatatttctt atacttatga ggctgggtgt ggtggcttat 53870
gcctgtaatc ccagcacttt gggaggctga agtgggtgga ttgcttgggc tcaggagttc 53930
gagaccaacc tgggcaacat ggcgaaaccc tgtttctaga aaaggtacaa aaattagctg 53990
ggcaggtggc acgtgcctgt ggtaccagct acttgagggc ctgaggcagg aggatcgctt 54050
gaacctggga ggtcgaggtt gcagtgaact gagatcatgt cactgcactc cagcctggtg 54110
acagagcaag accccgtctc aaaaaaaaaa aaagaaagaa aaaaattctt atgcatagat 54170
ttgcctcttt tctgtttgtt tgttttgaga tggagtctcg ctctgtcgcc caggctggag 54230
tacagtggct caacctcggc tcactgcaac ctctgcctcc cgggttcaag caattctcct 54290
gcctcagcct cctgagtagc tgggactaca gcgcccgcca ccatgcccag ctaatttttg 54350
tatttttagt agagactgac tgggtttcat catgttggcc aggctggtct cgaactcttg 54410
acctcatgat ccgcccgcct cagcctccca aaatgctggg attacaggcg tgagccacca 54470
ggcccaggcc gcaaggcgat ctctaaacaa acataaaaga ccaggagtca aggttatggt 54530
acgatgcccg tgttttcact ccagccacgg agctgggtct ctggtctcgg gggcagctgt 54590
gtgacagagc gtgcctctcc ctacag tg ctc ctc gtc ttc ctt tgc ctg ggg 54642
al leu leu val phe leu cys leu gly
780

gtc ttc ctt cta tgg aag aac tgg cgg ctt aag aac atc aac agc atc   54690
val phe leu leu trp lys asn trp arg leu lys asn ile asn ser ile
785                 790                 795                 800
aac ttt gac aac ccc gtc tat cag aag acc aca gag gat gag gtc cac   54738
asn phe asp asn pro val tyr gln lys thr thr glu asp glu val his
                805                 810                 815
att tgc cac aac cag gac ggc tac agc tac ccc tcg gtgagtgacc        54784
ile cys his asn gln asp gly tyr ser tyr pro ser
            820                 825
ctctctagaa agccagagcc catggcggcc ccctcccagc tggaggcata tgatcctcaa   54844
gggaccaggc cgaggcttcc ccagccctcc agatcgagga cagcattagg tgaatgcttc   54904
tgtgcgctca ttcagaatgt cagcggacaa tggccttggt ggtgtagagg aatgttggat   54964
aagcaaatag agagctccat cagatggtga cagggcaaag aaagtcaaaa ggagttcaga   55024
ggccgggcgc ggtggctcat gcctgtaatc ccaggacttt gggaggccga ggctggcgga   55084
tcacctgaag tcaggagttt gagaccagct tggccatcat gacaaaaccc cgtctctatt   55144
aaaaatacaa aaaattagcc aggcgtggga gtgggcgcct gtaatcccag ctactcggga   55204
ggccgaggta gaaaaatcgc ttgaacctag gaggcagagg ttgcagtgag ccgagatcgc   55264
gccactgcat tccagcccgg gaggcaagag caaaactcca tctcaaaaaa aaaaaaaaaa   55324
ggagttcaga ggcccggcat ggtggttcac acatgtgatc ccagaacttg ggaggttgga   55384
ggcaggagaa tcacctgagc tcagagttca agaccagcct gggcagcaca gcaagacccc   55444
atctctgcaa aaaataaaaa tttagcccag tgtggtgatg agcgcctagt tccagctact   55504
agggaggcta aggcaggagg attgcttgag gctaaggtag gagattgaga ctgcagtgac   55564
ttgtgattgc gtcactgcgc tccagcctgg gtgacagagc aagcccttgt ctcttaaaaa   55624
aaaaaaaaaa ttcaaagaag ggtttccaga gggccaggag ggaggaaggg agaggaggtg   55684
ttttattttt ttgcttttat tttttatttt gagacagagt ctctctctgt cacccaggtt   55744
ggagtgcagt gctgtgatct tggctcactg caacttctgc ctcctgggtt caagcaattc   55804
ttatgcctca gcctcagcct cctgagtagc tgggattaca acactatgcc cgggtaattt   55864
ttgtattttt agtagagacg aggtttcgcc atgttgccca gactggtctc gaactcctga   55924
cctcaagtga tccacccgcc ttggcctccc cacgtgctgg gattgcaggc gtgagccact   55984
gcgcccgcct tgatctttac acaaggggtt tagggtaggt agccttctct gaaccaggag   56044
aacagcctgt gcgaaggccc tgaggctgga ccgtgcctgt tgggtttgag gccgttgtag   56104
ctggagcaaa cagagagagg ggtaaaaagg caggaggcta ccaggcaggt tgtgcagagc   56164
cttgtgggcc actggggagg actttggctt ttgccctgag agcggtggga agtgactgaa   56224
tccggtactc accgtctccc tctggcggct cctgggggaa catgcttggg gatcaggctg   56284
ggggaggctg ccaggcccag gaggtgagaa gtaggtggcc tccagccgtg tttcctgaat   56344
gctggactga tagtttccgc tgtttaccat ttgttggcag aga cag atg gtc agt   56399
                                            arg gln met val ser
                                                    830
ctg gag gat gac gtg gcg tgaacatctg cctggagtcc cgtccctgcc          56447
leu glu asp asp val ala
    835             839
cagaaccctt cctgagacct cgccggcctt gttttattca aagacagaga agaccaaagc   56507

attgcctgcc agagctttgt tttatatatt tattcatctg ggaggcagaa caggcttcgg 56567
acagtgccca tgcaatggct tgggttggga ttttggtttc ttcctttcct cgtgaaggat 56627
aagagaaaca ggcccggggg gaccaggatg acacctccat ttctctccag gaagttttga 56687
gtttctctcc accgtgacac aatcctcaaa catggaagat gaaaggggag gggatgtcag 56747
gcccagagaa gcaagtggct ttcaacacac aacagcagat ggcaccaacg ggaccccctg 56807
gccctgcctc atccaccaat ctctaagcca aacccctaaa ctcaggagtc aacgtgttta 56867
cctcttctat gcaagccttg ctagacagcc aggttagcct ttgccctgtc acccccgaat 56927
catgacccac ccagtgtctt tcgaggtggg tttgtacctt ccttaagcca ggaaagggat 56987
tcatggcgtc ggaaatgatc tggctgaatc cgtggtggca ccgagaccaa actcattcac 57047
caaatgatgc cacttcccag aggcagagcc tgagtcactg gtcaccctta atatttatta 57107
agtgcctgag acacccggtt accttggccg tgaggacacg tggcctgcac ccaggtgtgg 57167
ctgtcaggac accagcctgg tgcccatcct cccgacccct acccacttcc attcccgtgg 57227
tctccttgca ctttctcagt tcagagttgt acactgtgta catttggcat ttgtgttatt 57287
attttgcact gttttctgtc gtgtgtgttg ggatgggatc ccaggccagg gaaagcccgt 57347
gtcaatgaat gccggggaca gagaggggca ggttgaccgg gacttcaaag ccgtgatcgt 57404
gaatatcgag aactgccatt gtcgtcttta tgtccgccca cctagtgctt ccacttctat 57467
gcaaatgcct ccaagccatt cacttcccca atcttgtcgt tgatgggtat gtgtttaaaa 57527
catgcacggt gaggccgggc gcagtggctc acgcctgtaa tcccagcact ttgggaggcc 57587
gaggcgggtg gatcatgagg tcaggagatc gagaccatcc tggctaacac gtgaaacccc 57647
gtctctacta aaaatacaaa aaattagccg ggcgtggtgg cgggcacctg tagtcccagc 57707
tactcgggag gctgaggcag gagaatggtg tgaacccggg aagcggagct tgcagtgagc 57767
cgagattgcg ccactgcagt ccgcagtctg gcctgggcga cagagcgaga ctccgtctca 57827
aaaaaaaaaa acaaaaaaaa accatgcatg gtgcatcagc agcccatggc ctctggccag 57887
gcatggcgag gctgaggtgg gaggatggtt tgagctcagg catttgaggc tgtcgtgagc 57947
tatgattatg ccactgcttt ccagcctggg caacatagta agaccccatc tcttaaaaaa 58007
tgaatttggc cagacacagg tgcctcacgc ctgtaatccc agcactttgg gaggctgagc 58067
tggatcactt gagttcagga gttggagacc aggcctgagc aacaaagcga gatcccatct 58127
ctacaaaaac caaaaagtta aaaatcagct gggtacggtg gcacgtgcct gtgatcccag 58187
ctacttggga ggctgaggca ggaggatcgc ctgagcccag gaggtggagg ttgcagtgag 58247
ccatgatcga gccactgcac tccagcctgg gcaacagatg aagaccctat ttcagaaata 58307
caactataaa aaaataaata aatcctccag tctggatcgt ttgacgggac ttcaggttct 58367
ttctgaaatc gccgtgttac tgttgcactg atgtccggag agacagtgac agcctccgtc 58427
agactcccgc gtgaagatgt cacaagggat tggcaattgt ccccagggac aaaacactgt 58487
gtccccccca gtgcagggaa ccgtgataag cctttctggt ttcggagcac gtaaatgcgt 58547
ccctgtacag atagtgggga ttttttgtta tgtttgcact ttgtatattg gttgaaactg 58607
ttatcactta tatatatata tatacacaca tatatataaa atctatttat ttttgcaaac 58667
cctggttgct gtatttgttc agtgactatt ctcgggcccc tgtgtagggg gttattgcct 58727
ctgaaatgcc tcttctttat gtacaaagat tatttgcacg aactggactg tgtgcaacgc 58787
tttttgggag aatgatgtcc ccgttgtatg tatgagtggc ttctgggaga tgggtgtcac 58847
tttttaaacc actgtataga aggtttttgt agcctgaatg tcttactgtg atcaattaaa 58907
tttcttaaat gaaccaattt gtctaaactc gatgcacgtt cttctgttcg cgcgcttctt 58967
tttgtttttt tttttttcct gagatggagc ctggctctgt cacccctggc tggagtgcag 59027

tggcatgatc tcggcttact gcaagctccg cctcccaggt tcaagcaatt ctcctgcctc 59087
agcctcccta gtagctagga ttacaggtga gtgccaccac gcctggccaa tttttttttt 59147
tttttttttt ttgagacaga gtctcgctct gtcacccagg ctggagtgca gtggtgtgat 59207
ctcggctcac tgcaagctct gcctcccagg ttaatgccat tctcctgtct cagcctcctg 59267
agtagctggg gccacaggcg cctgccacca cgcccggcta attttttttt gtacttcttt 59327
tagtacagac ggggtttcac catgttagcc aggatggtct cgatctcctg accttgtgat 59387
ccacctgctt cggcctccca aagtgctgag attacaggcg tgagccaccg cgggtggcca 59447
acgctaattt ttttgttttt ttagatggag tcttgctctg tcgcccaggc tggagtgcag 59507
tggcgtgatc tctgcctact gcaagctccg cctcccgggt tcatgccatt ctcctgcctc 59567
agcctcctga gtaactggga ctacaggcac ccgccaccac gcccggctaa ttttttgtat 59627
ttttagtaga gacagggttt caccgtgtta gccaggatgg tcttgatctc ctgaccttgt 59687
gatccacccg tctcggcctc ccaaagtgct gggattagag gtgtgagcca ccacacctgg 59747
cctagcctgg ctaatttttg tatttttggt agagacgggg tttcaccatg ttggtcaggc 59807
tggtcttgaa cttctgacct caggtaatct gcctgcctca gtctcccaaa gtgctgggat 59867
tacaggtgtg agccaccgcg cctggcctca cttccttctg tcatctgttt gtggattgga 59927
ctccccagga gaaggaccca gaaggggaag actcccagaa ctccgggcaa gatgcaatct 59987
ccgtgggctg cca 60000

<210> SEQ ID NO.: 2
   <211> 24
   <212> polynucleotide
   <213> artificial sequence
<220>
   <221> oligonucleotide
   <223> Ex1F
<400>
   cacattgaaa tgctgtaaat gacg
<210> SEQ ID NO.: 3
   <211> 24
   <212> polynucleotide
   <213> artificial sequence
<220>
   <221> oligonucleotide
   <223> Ex1R
<400>
   ctattctggc gcctggagca agcc
<210> SEQ ID NO.: 4
   <211> 24
   <212> polynucleotide
   <213> artificial sequence
<220>
   <221> oligonucleotide
   <223> Ex2F
<400>
   ttgagagacc ctttctcctt ttcc
<210> SEQ ID NO.: 5
   <211> 20
   <212> polynucleotide
   <213> artificial sequence
<220>
   <221> oligonucleotide
   <223> Ex2R
<400>
   gcatatcatg cccaaagggg
<210> SEQ ID NO.: 6
   <211> 24
   <212> polynucleotide
   <213> artificial sequence
<220>
   <221> oligonucleotide
   <223> Ex3F
<400>
   ttcctttgag tgacagttca atcc
<210> SEQ ID NO.: 7
   <211> 24
   <212> polynucleotide
   <213> artificial sequence
<220>
   <221> oligonucleotide
   <223> Ex3R
<400>
   gataggctca atagcaaagg cagg
<210> SEQ ID NO.: 8
   <211> 24
   <212> polynucleotide
   <213> artificial sequence
<220>
   <221> oligonucleotide
   <223> Mut191-2F
<400>
   acagttcaat cctgtctctt ctct
<210> SEQ ID NO.: 9
   <211> 10
   <212> polynucleotide
   <213> artificial sequence
<220>
   <221> oligonucleotide
   <223> Ex4AF
<400>
   gtggtctcgg ccatccatcc
<210> SEQ ID NO.: 10
   <211> 20
   <212> polynucleotide
   <213> artificial sequence
<220>
   <221 > oligonucleotide
   <223> Ex4ARF
<400>
   agccatcttc gcagtcgggg
<210> SEQ ID NO.: 11
   <211> 12
   <212> polynucleotide
   <213> artificial sequence
<220>
   <221> oligonucleotide
   <223> Mut 509insCR
<400>
   cgagccatct tcgcagtcgg ag
<210> SEQ ID NO.: 12
   <211> 20
   <212> polynucleotide
   <213> artificial sequence
<220>
   <221> oligonucleotide
   <223> Ex4BF
<400>
   cccccagctg tgggcctgcg
<210> SEQ ID NO.: 13
   <211> 20
   <212> polynucleotide
   <213> artificial sequence
<220>
   <221> oligonucleotide
   <223> Ex4BR
<400>
   cgcccccacc ctgccccgcc
<210> SEQ ID NO.: 14
   <211> 20
   <212> polynucleotide
   <213> artificial sequence
<220>
   <221> oligonucleotide
   <223> Ex6F
<400>
   tcctccttcc tctctctggc
<210> SEQ ID NO.: 15
   <211 > 20
   <212> polynucleotide
   <213> artificial sequence
<220>
   <221> oligonucleotide
   <223> Ex6R
<400>
   tctgcaagcc gcctgcaccg
<210> SEQ ID NO.: 16
   <211> 23
   <212> polynucleotide
   <213> artificial sequence
<220>
   <221> oligonucleotide
   <223> MutC255GF
<400>
   ctctggctctc acagtgacac gc
<210> SEQ ID NO.: 17
   <211> 20
   <212> polynucleotide
   <213> artificial sequence
<220>
   <221> oligonucleotide
   <223> Mut E291 XR
<400>
   gcaccgagac tcaccgcaat
<210> SEQ ID NO.: 18
   <211> 20
   <212> polynucleotide
   <213> artificial sequence
<220>
   <221> oligonucleotide
   <223> Ex7F
<400>
   ggcgaaggga tgggtagggg
<210> SEQ ID NO.: 19
   <211> 20
   <212> polynucleotide
   <213> artificial sequence
<220>
   <221> oligonucleotide
   <223> Ex7R
<400>
   gttgccatgt caggaagcgc
<210> SEQ ID NO.: 20
   <211> 20
   <212> polynucleotide
   <213> artificial sequence
<220>
   <221> oligonucleotide
   <223> Ex9F
<400>
   cccctgacct cgctccccgg
<210> SEQ ID NO.: 21
   <211> 20
   <212> polynucleotide
   <213> artificial sequence
<220>
   <221> oligonucleotide
   <223> Ex9R
<400>
   gctgcaggca ggggcgacgc
<210> SEQ ID NO.: 22
   <211> 20
   <212> polynucleotide
   <213> artificial sequence
<220>
   <221> oligonucleotide
   <223> Ex10F
<400>
   atgcccttct ctcctcctgc
<210> SEQ ID NO.: 23
   <211> 24
   <212> polynucleotide
   <213> artificial sequence
<220>
   <221> oligonucleotide
   <223> Ex10R
<400>
   agccctcagc gtcgtggata
<210> SEQ ID NO.: 24
   <211> 20
   <212> polynucleotide
   <213> artificial sequence
<220>
   <221> oligonucleotide
   <223> Mut1432delGF
<400>
   gggacatcca ggcccccgcc
<210> SEQ ID NO.: 25
   <211> 20
   <212> polynucleotide
   <213> artificial sequence
<220>
   <221> oligonucleotide
   <223> Ex11F
<400>
   tcctcccccg ccctccagcc
<210> SEQ ID NO.: 26
   <211> 20
   <212> polynucleotide
   <213> artificial sequence
<220>
   <221> oligonucleotide
   <223> Ex11R
<400>
   gctgggacgg ctgtcctgcg
<210> SEQ ID NO.: 27
   <211> 20
   <212> polynucleotide
   <213> artificial sequence
<220>
   <221> oligonucleotide
   <223> Ex13F
<400>
   gtcatcttcc ttgctgcctg
<210> SEQ ID NO.: 28
   <211> 30
   <212> polynucleotide
   <213> artificial sequence
<220>
   <221> oligonucleotide
   <223> Ex13R
<400>
   ttccacaagg aggtttcaag gttggggggg
<210> SEQ ID NO.: 29
   <211> 13
   <212> polynucleotide
   <213> artificial sequence
<220>
   <221> oligonucleotide
   <223> MutH635NR
<400>
   acctcttggc tgggtcaggt tct

<210> SEQ ID NO.: 30
   <211> 20
   <212> polynucleotide
   <213> artificial sequence
<220>
   <221> oligonucleotide
   <223> Ex14F
<400>
   aaatttctgg aatcttctgg
<210> SEQ ID NO.: 31
   <211> 20
   <212> polynucleotide
   <213> artificial sequence
<220>
   <221> oligonucleotide
   <223> Ex14R
<400>
   gcagagagag gctcaggagg
<210> SEQ ID NO.: 32
   <211> 22
   <212> polynucleotide
   <213> artificial sequence
<220>
   <221> oligonucleotide
   <223> Ex15F
<400>
   gaagggcctg cagcacgtgg ca
<210> SEQ ID NO.: 33
   <211> 19
   <212> polynucleotide
   <213> artificial sequence
<220>
   <221> oligonucleotide
   <223> Ex15R
<400>
   tagggagggc ccagtcttt
<210> SEQ ID NO.: 34
   <211> 20
   <212> polynucleotide
   <213> artificial sequence
<220>
   <221> oligonucleotide
   <223> Ex17F
<400>
   gggtctctgg tctcgggggc
<210> SEQ ID NO.: 35
   <211> 22
   <212> polynucleotide
   <213> artificial sequence
<220>
   <221> oligonucleotide
   <223> Ex17R
<400>
   ggctctggct ttctagagag gg
<210> SEQ ID NO.: 36
   <211> 23
   <212> polynucleotide
   <213> artificial sequence
<220>
   <221> oligonucleotide
<400>
   cgggtcggga cactgcctgg cag
<210> SEQ ID NO.: 37
   <211> 23
   <212> polynucleotide
   <213> artificial sequence
<220>
   <221> oligonucleotide
<400>
   cgggtcggga ccctgcctgg cag
<210> SEQ ID NO.: 38
   <211> 23
   <212> polynucleotide
   <213> artificial sequence
<220>
   <221> oligonucleotide
<400>
   ctgccaggca gtgtcccgac ccg
<210> SEQ ID NO.: 39
   <211> 23
   <212> polynucleotide
   <213> artificial sequence
<220>
   <221> oligonucleotide
<400>
   ctgccaggca gggtcccgac ccg
<210> SEQ ID NO.: 40
   <211> 23
   <212> polynucleotide
   <213> artificial sequence
<220>
   <221> oligonucleotide
<400>
   atgcatttcc cgtcttggca ctg
<210> SEQ ID NO.: 41
   <211> 23
   <212> polynucleotide
   <213> artificial sequence
<220>
   <221> oligonucleotide
<400>
   gatgcatttc cctcttggca ctg
<210> SEQ ID NO.: 42
   <211> 25
   <212> polynucleotide
   <213> artificial sequence
<220>
   <221> oligonucleotide
<400>
   gatgcatttc ccgtcttggc actgg
<210> SEQ ID NO.: 43
   <211> 25
   <212> polynucleotide
   <213> artificial sequence
<220>
   <221> oligonucleotide
<400>
   agatgcattt ccctcttggc actgg
<210> SEQ ID NO.: 44
   <211> 25
   <212> polynucleotide
   <213> artificial sequence
<220>
   <221> oligonucleotide
<400>
   tgtctcttct gtagtgtctg tcacc
<210> SEQ ID NO.: 45
   <211> 25
   <212> polynucleotide
   <213> artificial sequence
<220>
   <221> oligonucleotide
<400>
   gtctcttctg tctgtgtctg tcacc
<210> SEQ ID NO.: 46
   <211> 27
   <212> polynucleotide
   <213> artificial sequence
<220>
   <221> oligonucleotide
<400>
   ctgtctcttc tgtagtgtct gtcacct
<210> SEQ ID NO.: 47
   <211> 27
   <212> polynucleotide
   <213> artificial sequence
<220>
   <221> oligonucleotide
<400>
   tgtctcttct gtctgtgtct gtcacct
<210> SEQ ID NO.: 48
   <211> 23
   <212> polynucleotide
   <213> artificial sequence
<220>
   <221> oligonucleotide
<400>
   ggccgtgtca accgctgcat tcc
<210> SEQ ID NO.: 49
   <211> 21
   <212> polynucleotide
   <213> artificial sequence
<220>
   <221> oligonucleotide
<400>
   gccgtgtcaa ccgctgcatt c
<210> SEQ ID NO.: 50
   <211> 25
   <212> polynucleotide
   <213> artificial sequence
<220>
   <221> oligonucleotide
<400>
   aggaatgcag cgtttgacac ggccc
<210> SEQ ID NO.: 51
   <211> 27
   <212> polynucleotide
   <213> artificial sequence
<220>
   <221> oligonucleotide
<400>
   gaggaatgca gcgtttgaca cggcccc
<210> SEQ ID NO.: 52
   <211> 23
   <212> polynucleotide
   <213> artificial sequence
<220>
   <221> oligonucleotide
<400>
   agctgtgggg gccgtgtcaa ccg
<210> SEQ ID NO.: 53
   <211> 23
   <212> polynucleotide
   <213> artificial sequence
<220>
   <221> oligonucleotide
<400>
   agctgtgggg gcgtgtcaac cgc
<210> SEQ ID NO.: 54
   <211> 23
   <212> polynucleotide
   <213> artificial sequence
<220>
   <221> oligonucleotide
<400>
   cggttgacac ggcccccaca gct
<210> SEQ ID NO.: 55
   <211> 23
   <212> polynucleotide
   <213> artificial sequence
<220>
   <221> oligonucleotide
<400>
   gcggttgaca cgcccccaca gct
<210> SEQ ID NO.: 56
   <211> 21
   <212> polynucleotide
   <213> artificial sequence
<220>
   <221> oligonucleotide
<400>
   caaggctgtc gtaagtgtgg c
<210> SEQ ID NO.: 57
   <211> 23
   <212> polynucleotide
   <213> artificial sequence
<220>
   <221> oligonucleotide
<400>
   gcaaggctgt cgtaagtgtg gcc
<210> SEQ ID NO.: 58
   <211> 23
   <212> polynucleotide
   <213> artificial sequence
<220>
   <221> oligonucleotide
<400>
   caaggctgtc gttaagtgtg gcc
<210> SEQ ID NO.: 59
   <211> 21
   <212> polynucleotide
   <213> artificial sequence
<220>
   <221> oligonucleotide
<400>
   aaggctgtcg ttaagtgtgg c
<210> SEQ ID NO.: 60
   <211> 25
   <212> polynucleotide
   <213> artificial sequence
<220>
   <221> oligonucleotide
<400>
   gacaacgacc ccgactgcga agatg
<210> SEQ ID NO.: 61
   <211> 25
   <212> polynucleotide
   <213> artificial sequence
<220>
   <221> oligonucleotide
<400>
   gacaacgacc cccgactgcg aagat
<210> SEQ ID NO.: 62
   <211> 23
   <212> polynucleotide
   <213> artificial sequence
<220>
   <221> oligonucleotide
<400>
   acaacgaccc cgactgcgaa gat
<210> SEQ ID NO.: 63
   <211> 23
   <212> polynucleotide
   <213> artificial sequence
<220>
   <221> oligonucleotide
<400>
   acaacgaccc ccgactgcga aga
<210> SEQ ID NO.: 64
   <211> 23
   <212> polynucleotide
   <213> artificial sequence
<220>
   <221> oligonucleotide
<400>
   gcggccactc atccgagcca tct
<210> SEQ ID NO.: 65
   <211> 23
   <212> polynucleotide
   <213> artificial sequence
<220>
   <221> oligonucleotide
<400>
   gcggccactc acccgagcca tct
<210> SEQ ID NO.: 66
   <211> 25
   <212> polynucleotide
   <213> artificial sequence
<220>
   <221> oligonucleotide
<400>
   tgcggccact catccgagcc atctt

<210> SEQ ID NO.: 67
   <211> 25
   <212> polynucleotide
   <213> artificial sequence
<220>
   <221> oligonucleotide
<400>
   tgcggccact cacccgagcc atctt
<210> SEQ ID NO.: 68
   <211> 23
   <212> polynucleotide
   <213> artificial sequence
<220>
   <221> oligonucleotide
<400>
   ccagctggcg ctgtgatggt ggc
<210> SEQ ID NO.: 69
   <211> 23
   <212> polynucleotide
   <213> artificial sequence
<220>
   <221> oligonucleotide
<400>
   ccagctggcg ccgtgatggt ggc
<210> SEQ ID NO.: 70
   <211> 25
   <212> polynucleotide
   <213> artificial sequence
<220>
   <221> oligonucleotide
<400>
   tccagctggc gctgtgatgg tggcc
<210> SEQ ID NO.: 71
   <211> 25
   <212> polynucleotide
   <213> artificial sequence
<220>
   <221> oligonucleotide
<400>
   tccagctggc gccgtgatgg tggcc
<210> SEQ ID NO.: 72
   <211> 25
   <212> polynucleotide
   <213> artificial sequence
<220>
   <221> oligonucleotide
<400>
   ctgcaaggac aaatctgacg aggaa
<210> SEQ ID NO.: 73
   <211> 25
   <212> polynucleotide
   <213> artificial sequence
<220>
   <221> oligonucleotide
<400>
   ctgcaaggac aactgcggta tgggc
<210> SEQ ID NO.: 74
   <211> 27
   <212> polynucleotide
   <213> artificial sequence
<220>
   <221> oligonucleotide
<400>
   actgcaagga caaatctgac gaggaaa
<210> SEQ ID NO.: 75
   <211> 27
   <212> polynucleotide
   <213> artificial sequence
<220>
   <221> oligonucleotide
<400>
   actgcaagga caactgcggt atgggcg
<210> SEQ ID NO.: 76
   <211> 25
   <212> polynucleotide
   <213> artificial sequence
<220>
   <221> oligonucleotide
<400>
   caaatctgac gaggaaaact gcggt
<210> SEQ ID NO.: 77
   <211> 25
   <212> polynucleotide
   <213> artificial sequence
<220>
   <221> oligonucleotide
<400>
   caaatctgac gacaaatctg acgag
<210> SEQ ID NO.: 78
   <211> 27
   <212> polynucleotide
   <213> artificial sequence
<220>
   <221> oligonucleotide
<400>
   acaaatctga cgaggaaaac tgcggta
<210> SEQ ID NO.: 79
   <211> 27
   <212> polynucleotide
   <213 > artificial sequence
<220>
   <221> oligonucleotide
<400>
   acaaatctga cgacaaatct gacgagg
<210> SEQ ID NO.: 80
   <211> 23
   <212> polynucleotide
   <213> artificial sequence
<220>
   <221> oligonucleotide
<400>
   gggtccctcg cagagtgtca ctg
<210> SEQ ID NO.: 81
   <211> 23
   <212> polynucleotide
   <213> artificial sequence
<220>
   <221> oligonucleotide
<400>
   gggtccctcg ccgagtgtca ctg
<210> SEQ ID NO.: 82
   <211> 25
   <212> polynucleotide
   <213> artificial sequence
<220>
   <221> oligonucleotide
<400>
   tgggtccctc gcagagtgtc actgt
<210> SEQ ID NO.: 83
   <211> 25
   <212> polynucleotide
   <213> artificial sequence
<220>
   <221> oligonucleotide
<400>
   tgggtccctc gccgagtgtc actgt
<210> SEQ ID NO.: 84
   <211> 23
   <212> polynucleotide
   <213> artificial sequence
<220>
   <221> oligonucleotide
<400>
   aacccatcaa agagtgcggt gag
<210> SEQ ID NO.: 85
   <211> 23
   <212> polynucleotide
   <213> artificial sequence
<220>
   <221> oligonucleotide
<400>
   aacccatcaa atagtgcggt gag
<210> SEQ ID NO.: 86
   <211> 25
   <212> polynucleotide
   <213> artificial sequence
<220>
   <221> oligonucleotide
<400>
   gaacccatca aagagtgcgg tgagt
<210> SEQ ID NO.: 87
   <211> 25
   <212> polynucleotide
   <213> artificial sequence
<220>
   <221> oligonucleotide
<400>
   gaacccatca aatagtgcgg tgagt
<210> SEQ ID NO.: 88
   <211> 20
   <212> polynucleotide
   <213> artificial sequence
<220>
   <221> oligonucleotide
<400>
   tcactctcgg gcccctacca
<210> SEQ ID NO.: 89
   <211> 21
   <212> polynucleotide
   <213> artificial sequence
<220>
   <221> oligonucleotide
<400>
   tcactctcgg acccctaccc a
<210> SEQ ID NO.: 90
   <211> 20
   <212> polynucleotide
   <213> artificial sequence
<220>
   <221> oligonucleotide
<400>
   cactctcggg cccctaccc
<210> SEQ ID NO.: 91
   <211> 19
   <212> polynucleotide
   <213> artificial sequence
<220>
   <221> oligonucleotide
<400>
   cactctcgga cccctaccc
<210> SEQ ID NO.: 92
   <211> 25
   <212> polynucleotide
   <213> artificial sequence
<220>
   <221> oligonucleotide
<400>
   acgagtgcct gtgcgccgac ggctt
<210> SEQ ID NO.: 93
   <211> 25
   <212> polynucleotide
   <213> artificial sequence
<220>
   <221> oligonucleotide
<400>
   acgagtgcct gtacgccgac ggctt
<210> SEQ ID NO.: 94
   <211>23
   <212> polynucleotide
   <213> artificial sequence
<220>
   <221> oligonucleotide
<400>
   cgagtgcctg tgcgccgacg gct
<210> SEQ ID NO.: 95
   <211> 23
   <212> polynucleotide
   <213> artificial sequence
<220>
   <221> oligonucleotide
<400>
   cgagtgcctg tacgccgacg gct
<210> SEQ ID NO.: 96
   <211> 24
   <212> polynucleotide
   <213> artificial sequence
<220>
   <221> oligonucleotide
<400>
   gcgaagatgc gaaggtgatt ccgg
<210> SEQ ID NO. : 97
   <211> 25
   <212> polynucleotide
   <213> artificial sequence
<220>
   <221> oligonucleotide
<400>
   ggcccagcga agatttccgg gtggg
<210> SEQ ID NO.: 98
   <211> 27
   <212> polynucleotide
   <213> artificial sequence
<220>
   <221> oligonucleotide
<400>
   agcgaagatg cgaaggtgat ttccggg
<210> SEQ ID NO.: 99
   <211> 27
   <212> polynucleotide
   <213> artificial sequence
<220>
   <221> oligonucleotide
<400>
   tggcccagcg aagatttccg ggtggga
<210> SEQ ID NO.: 100
   <211> 21
   <212> polynucleotide
   <213> artificial sequence
<220>
   <221> oligonucleotide
<400>
   tgaagaagag gtaggcgatg g
<210> SEQ ID NO.: 101
   <211> 21
   <212> polynucleotide
   <213> artificial sequence
<220>
   <221> oligonucleotide
<400>
   cggttggtga agacgatgga g
<210> SEQ ID NO.: 102
   <211> 23
   <212> polynucleotide
   <213> artificial sequence
<220>
   <221> oligonucleotide
<400>
   gtgaagaaga ggtaggcgat gga
<210> SEQ ID NO.: 103
   <211> 23
   <212> polynucleotide
   <213> artificial sequence
<220>
   <221> oligonucleotide
<400>
   ccggttggtg aagacgatgg agc
<210> SEQ ID NO.: 104
   <211> 25
   <212> polynucleotide
   <213> artificial sequence

<220>
   <221> oligonucleotide
<400>
   ctccatcgcc tacctcttct tcacc
<210> SEQ ID NO.: 105
   <211> 25
   <212> polynucleotide
   <213> artificial sequence
<220>
   <221> oligonucleotide
<400>
   ctccatcgcc taactcttct tcacc
<210> SEQ ID NO.: 106
   <211> 27
   <212> polynucleotide
   <213> artificial sequence
<220>
   <221> oligonucleotide
<400>
   gctccatcgc ctacctcttc ttcacca
<210> SEQ ID NO.: 107
   <211> 27
   <212> polynucleotide
   <213> artificial sequence
<220>
   <221> oligonucleotide
<400>
   gctccatcgc ctaactcttc ttcacca
<210> SEQ ID NO.: 108
   <211> 25
   <212> polynucleotide
   <213> artificial sequence
<220>
   <221> oligonucleotide
<400>
   tgccggttgg tgaagaagag gtagg
<210> SEQ ID NO.: 109
   <211> 25
   <212> polynucleotide
   <213> artificial sequence
<220>
   <221> oligonucleotide
<400>
   gtgccggttg gtgagaagag gtagg
<210> SEQ ID NO.: 110
   <211> 27
   <212> polynucleotide
   <213> artificial sequence
<220>
   <221> oligonucleotide
<400>
   gtgccggttg gtgaagaaga ggtaggc
<210> SEQ ID NO.: 111
   <211> 27
   <212> polynucleotide
   <213> artificial sequence
<220>
   <221> oligonucleotide
<400>
   cgtgccggtt ggtgagaaga ggtaggc
<210> SEQ ID NO.: 112
   <211> 25
   <212> polynucleotide
   <213> artificial sequence
<220>
   <221> oligonucleotide
<400>
   caatagaatc tactggtctg acctg
<210> SEQ ID NO.: 113
   <211> 25
   <212> polynucleotide
   <213> artificial sequence
<220>
   <221> oligonucleotide
<400>
   caatagaatc tagtggtctg acctg
<210> SEQ ID NO.: 114
   <211> 27
   <212> polynucleotide
   <213> artificial sequence
<220>
   <221> oligonucleotide
<400>
   gcaatagaat ctactggtct gacctgt
<210> SEQ ID NO.: 115
   <211> 27
   <212> polynucleotide
   <213> artificial sequence
<220>
   <221> oligonucleotide
<400>
   gcaatagaat ctagtggtct gacctgt
<210> SEQ ID NO.: 116
   <211> 25
   <212> polynucleotide
   <213> artificial sequence
<220>
   <221> oligonucleotide
<400>
   ggcccccgac gggctggctg tggac
<210> SEQ ID NO.: 117
   <211> 25
   <212> polynucleotide
   <213> artificial sequence
<220>
   <221> oligonucleotide
<400>
   ggcccccgac ggctggctgt ggact
<210> SEQ ID NO.: 118
   <211> 25
   <212> polynucleotide
   <213> artificial sequence
<220>
   <221> oligonucleotide
<400>
   gtccacagcc agcccgtcgg gggcc
<210> SEQ ID NO.: 119
   <211> 25
   <212> polynucleotide
   <213> artificial sequence
<220>
   <221 > oligonucleotide
<400>
   agtccacagc cagccgtcgg gggcc
<210> SEQ ID NO.: 120
   <211> 25
   <212> polynucleotide
   <213> artificial sequence
<220>
   <221> oligonucleotide
<400>
   gcgggagttc cccagtcagt ccagt
<210> SEQ ID NO.: 121
   <211> 25
   <212> polynucleotide
   <213> artificial sequence
<220>
   <221> oligonucleotide
<400>
   gcgggagttc cctagtcagt ccagt
<210> SEQ ID NO.: 122
   <211> 23
   <212> polynucleotide
   <213> artificial sequence
<220>
   <221> oligonucleotide
<400>
   cgggagttcc ccagtcagtc cag
<210> SEQ ID NO.: 123
   <211> 23
   <212> polynucleotide
   <213> artificial sequence
<220>
   <221> oligonucleotide
<400>
   cgggagttcc ctagtcagtc cag
<210> SEQ ID NO.: 124
   <211> 25
   <212> polynucleotide
   <213> artificial sequence
<220>
   <221> oligonucleotide
<400>
   ctgtccccag aggatatggt tctct
<210> SEQ ID NO.: 125
   <211> 25
   <212> polynucleotide
   <213> artificial sequence
<220>
   <221> oligonucleotide
<400>
   ctgtccccag agaatatggt tctct
<210> SEQ ID NO.: 126
   <211> 23
   <212> polynucleotide
   <213> artificial sequence
<220>
   <221> oligonucleotide
<400>
   tgtccccaga ggatatggtt ctc
<210> SEQ ID NO.: 127
   <211> 23
   <212> polynucleotide
   <213> artificial sequence
<220>
   <221> oligonucleotide
<400 >
   tgtccccaga gaatatggtt ctc
<210> SEQ ID NO.: 128
   <211> 23
   <212> polynucleotide
   <213> artificial sequence
<220>
   <221> oligonucleotide
<400>
   tggttctctt ccacaacctc acc
<210> SEQ ID NO.: 129
   <211> 23
   <212> polynucleotide
   <213> artificial sequence
<220>
   <221> oligonucleotide
<400>
   tggttctctt caacaacctc acc
<210> SEQ ID NO.: 130
   <211> 25
   <212> polynucleotide
   <213> artificial sequence
<220>
   <221> oligonucleotide
<400>
   atggttctct tccacaacct caccc
<210> SEQ ID NO.: 131
   <211> 25
   <212> polynucleotide
   <213> artificial sequence
<220>
   <221> oligonucleotide
<400>
   atggttctct tcaacaacct caccc
<210> SEQ ID NO.: 132
   <211> 25
   <212> polynucleotide
   <213> artificial sequence
<220>
   <221> oligonucleotide
<400>
   gctgaccttt agcctgacgg tggat
<210> SEQ ID NO.: 133
   <211> 25
   <212> polynucleotide
   <213> artificial sequence
<220>
   <221> oligonucleotide
<400>
   agctgacctt tagctgacgg tggat
<210> SEQ ID NO.: 134
   <211> 27
   <212> polynucleotide
   <213> artificial sequence
<220>
   <221> oligonucleotide
<400>
   agctgacctt tagcctgacg gtggatg
<210> SEQ ID NO.: 135
   <211> 27
   <212> polynucleotide
   <213> artificial sequence
<220>
   <221> oligonucleotide
<400>
   gagctgacct ttagctgacg gtggatg
<210> SEQ ID NO.: 136
   <211 > 23
   <212> polynucleotide
   <213> artificial sequence
<220>
   <221> oligonucleotide
<400>
   tgctcctcgt cttcctttgc ctg
<210> SEQ ID NO.: 137
   <211> 23
   <212> polynucleotide
   <213> artificial sequence
<220>
   <221> oligonucleotide
<400>
   tgctcctcgg ggtctttgcc tgg
<210> SEQ ID NO.: 138
   <211> 25
   <212> polynucleotide
   <213> artificial sequence
<220>
   <221> oligonucleotide
<400>
   gtgctcctcg tcttcctttg cctgg
<210> SEQ ID NO.: 139
   <211> 25
   <212> polynucleotide
   <213> artificial sequence
<220>
   <221> oligonucleotide
<400>
   gtgctcctcg gggtctttgc ctggg
<210> SEQ ID NO.: 140
   <211> 25
   <212> polynucleotide
   <213> artificial sequence
<220>
   <221> oligonucleotide
<400>
   gactcacagc acgtctcctg ggact
<210> SEQ ID NO.: 141
   <211> 25
   <212> polynucleotide
   <213> artificial sequence
<220>
   <221> oligonucleotide
<400>
   gactcacagc acatctcctg ggact

<210> SEQ ID NO.: 142
   <211> 23
   <212> polynucleotide
   <213> artificial sequence
<220>
   <221> oligonucleotide
<400>
   actcacagca cgtctcctgg gac
<210> SEQ ID NO.: 143
   <211> 23
   <212> polynucleotide
   <213> artificial sequence
<220>
   <221> oligonucleotide
<400>
   actcacagca catctcctgg gac
<210> SEQ ID NO.: 144
   <211> 23
   <212> polynucleotide
   <213> artificial sequence
<220>
   <221> oligonucleotide
<400>
   ccatcgtggc agcgaaactc gtc
<210> SEQ ID NO.: 145
   <211> 23
   <212> polynucleotide
   <213> artificial sequence
<220>
   <221> oligonucleotide
<400>
   atgcacttcc cacgtcctgg gag
<210> SEQ ID NO.: 146
   <211> 21
   <212> polynucleotide
   <213> artificial sequence
<220>
   <221> oligonucleotide
<400>
   catcgtggca gcgaaactcg t
<210> SEQ ID NO.: 147
   <211> 21
   <212> polynucleotide
   <213> artificial sequence
<220>
   <221> oligonucleotide
<400>
   tgcacttccc acgtcctggg a
210> SEQ ID NO.: 148
<211> 20
   <212> polynucleotide
   <213> artificial sequence
<220>
   <221> oligonucleotide Ex8F
<400>
   cattggggaa gagcctcccc
210> SEQ ID NO.: 149
<211> 20
   <212> polynucleotide
   <213> artificial sequence
<220>
   <221> oligonucleotide Ex8R
<400>
   gcctgcaagg ggtgaggccg
210> SEQ ID NO.: 150
<211> 20
   <212> polynucleotide
   <213> artificial sequence
<220>
   <221> oligonucleotide Ex12F
<400>
   actggcatca gcacgtgacc
210> SEQ ID NO.: 151
<211> 20
   <212> polynucleotide
   <213> artificial sequence
<220>
   <221> oligonucleotide Ex12R
<400>
   cgtgtgtcta tccggccacc
210> SEQ ID NO.: 152
<211 > 20
   <212> polynucleotide
   <213> artificial sequence
<220>
   <221> oligonucleotide Ex16F
<400>
   gcgctttcct gccgtgacca
210> SEQ ID NO.: 153
<211> 23
   <212> polynucleotide
   <213> artificial sequence
<220>
   <221> oligonucleotide Ex16R
<400>
   cctgtccagg agaaaaagtg aac
210> SEQ ID NO.: 154
<211> 23
   <212> polynucleotide
   <213> artificial sequence
<220>
   <221> oligonucleotide MutI771TF
<400>
   cagtagcgtg agggctctgt caa
210> SEQ ID NO.: 155
<211> 19
   <212> polynucleotide
   <213> artificial sequence
<220>
   <221> oligonucleotide Mut2389+4A>GR
<400>
   ctgggggacc ggccggcgc
210> SEQ ID NO.: 156
<211> 23
   <212> polynucleotide
   <213> artificial sequence
<220>
   <221> oligonucleotide
<400>
   tgtcaagctg ggtgctgagg cag
210> SEQ ID NO.: 157
<211> 23
   <212> polynucleotide
   <213> artificial sequence
<220>
   <221> oligonucleotide
<400>
   tgtcaagctg gttgctgagg cag
210> SEQ ID NO.: 158
<211> 21
   <212> polynucleotide
   <213> artificial sequence
<220>
   <221> oligonucleotide
<400>
   gtcaagctgg gtgctgaggc a
210> SEQ ID NO.: 159
<211> 21
   <212> polynucleotide
   <213> artificial sequence
<220>
   <221> oligonucleotide
<400>
   gtcaagctgg ttgctgaggc a
210> SEQ ID NO.: 160
<211> 23
   <212> polynucleotide
   <213> artificial sequence
<220>
   <221> oligonucleotide
<400>
   ggtccctcgc agagtgtcac tgt
210> SEQ ID NO.: 161
<211> 23
   <212> polynucleotide
   <213> artificial sequence
<220>
   <221> oligonucleotide
<400>
   ggtccctcgc actgtgagag cca
210> SEQ ID NO.: 162
<211> 21
   <212> polynucleotide
   <213> artificial sequence
<220>
   <221> oligonucleotide
<400>
   gtccctcgca gagtgtcact g
210> SEQ ID NO.: 163
<211> 21
   <212> polynucleotide
   <213> artificial sequence
<220>
   <221> oligonucleotide
<400>
   gtccctcgca ctgtgagagc c
210> SEQ ID NO.: 164
<211> 21
   <212> polynucleotide
   <213> artificial sequence
<220>
   <221> oligonucleotide
<400>
   ccgtcggggg cctggatgtc t
210> SEQ ID NO.: 165
<211> 21
   <212> polynucleotide
   <213> artificial sequence
<220>
   <221> oligonucleotide
<400>
   cccgtcgggg tctggatgtc t
210> SEQ ID NO.: 166
<211> 23
   <212> polynucleotide
   <213> artificial sequence
<220>
   <221> oligonucleotide
<400>
   cccgtcgggg gcctggatgt ctc
210> SEQ ID NO.: 167
<211> 23
   <212> polynucleotide
   <213> artificial sequence
<220>
   <221> oligonucleotide
<400>
   gcccgtcggg gtctggatgt ctc
210> SEQ ID NO.: 168
<211> 25
   <212> polynucleotide
   <213> artificial sequence
<220>
   <221> oligonucleotide
<400>
   ccggttggtg aagaagaggt aggcg
<210> SEQ ID NO.: 169
   <211> 25
   <212> polynucleotide
   <213> artificial sequence
<220>
   <221> oligonucleotide
<400>
   cggttggtga agaaagaggt aggcg
<210> SEQ ID NO.: 170
   <211> 27
   <212> polynucleotide
   <213> artificial sequence
<220>
   <221> oligonucleotide
<400>
   gccggttggt gaagaagagg taggcga
<210> SEQ ID NO.: 171
   <211> 27
   <212> polynucleotide
   <213> artificial sequence
<220>
   <221> oligonucleotide
<400>
   ccggttggtg aagaaagagg taggcga
<210> SEQ ID NO.: 172
   <211> 23
   <212> polynucleotide
   <213> artificial sequence
<220>
   <221 > oligonucleotide
<400>
   actggaagct ggcgggacca cag
<210> SEQ ID NO.: 173
   <211> 23
   <212> polynucleotide
   <213> artificial sequence
<220>
   <221> oligonucleotide
<400>
   gcactggaag ctgggaccac agg
<210> SEQ ID NO.: 174
   <211> 23
   <212> polynucleotide
   <213> artificial sequence
<220>
   <221> oligonucleotide
<400>
   ctgtggtccc gccagcttcc agt
<10> SEQ ID NO.: 175
<211> 23
   <212> polynucleotide
   <213> artificial sequence
<220>
   <221> oligonucleotide
<400>
   cctgtggtcc cagcttccag tgc
<210> SEQ ID NO.: 176
   <211> 21
   <212> polynucleotide
   <213> artificial sequence
<220>
   <221> oligonucleotide
<400>
   gcgggagttc cccagtcagt c
<210> SEQ ID NO.: 177
   <211> 21
   <212> polynucleotide
   <213> artificial sequence
<220>
   <221> oligonucleotide
<400>
   gcgggagttc accagtcagt c

<210> SEQ ID NO.: 178
   <211> 23
   <212> polynucleotide
   <213> artificial sequence
<220>
   <221> oligonucleotide
<400>
   ggcgggagtt ccccagtcag tcc
<210> SEQ ID NO.: 179
   <211> 23
   <212> polynucleotide
   <213> artificial sequence
<220>
   <221> oligonucleotide
<400>
   ggcgggagtt caccagtcag tcc
<210> SEQ ID NO.: 180
   <211> 23
   <212> polynucleotide
   <213> artificial sequence
<220>
   <221> oligonucleotide
<400>
   ccccatcggt aagcgcgggc cgg
<210> SEQ ID NO.: 181
   <211> 23
   <212> polynucleotide
   <213> artificial sequence
<220>
   <221> oligonucleotide
<400>
   ccccatcggt aggcgcgggc cgg
<210> SEQ ID NO.: 182
   <211> 23
   <212> polynucleotide
   <213> artificial sequence
<220>
   <221> oligonucleotide
<400>
   ccggcccgcg cttaccgatg ggg
<210> SEQ ID NO.: 183
   <211> 23
   <212> polynucleotide
   <213> artificial sequence
<220>
   <221> oligonucleotide
<400>
   ccggcccgcg cctaccgatg ggg
<210> SEQ ID NO.: 184
   <211> 23
   <212> polynucleotide
   <213> artificial sequence
<220>
   <221> oligonucleotide
<400>
   gaaaagaggc tggcccaccc ctt
<210> SEQ ID NO.: 185
   <211> 23
   <212> polynucleotide
   <213> artificial sequence
<220>
   <221> oligonucleotide
<400>
   gaaaagaggc ttctccttgg ccg
<210> SEQ ID NO.: 186
   <211> 21
   <212> polynucleotide
   <213> artificial sequence
<220>
   <221> oligonucleotide
<400>
   aaaagaggct ggcccacccc t
<210> SEQ ID NO.: 187
   <211> 21
   <212> polynucleotide
   <213> artificial sequence
<220>
   <221> oligonucleotide
<400>
   aaaagaggct tctccttggc c
<210> SEQ ID NO.: 188
   <211> 25
   <212> polynucleotide
   <213> artificial sequence
<220>
   <221> oligonucleotide
<400>
   cgccttcccg tgctcaccca cagcc
<210> SEQ ID NO.: 189
   <211> 25
   <212> polynucleotide
   <213> artificial sequence
<220>
   <221> oligonucleotide
<400>
   cgccttcccg tgttcaccca cagcc
<210> SEQ ID NO.: 190
   <211> 25
   <212> polynucleotide
   <213> artificial sequence
<220>
   <221> oligonucleotide
<400>
   ggctgtgggt gagcacggga aggcg
<210> SEQ ID NO.: 191
   <211> 25
   <212> polynucleotide
   <213> artificial sequence
<220>
   <221> oligonucleotide
<400>
   ggctgtgggt gaccacggga aggcg
<210> SEQ ID NO.: 192
   <211> 25
   <212> polynucleotide
   <213> artificial sequence
<220>
   <221> oligonucleotide
<400>
   actatctcca ccgtggtgag cccag
<210> SEQ ID NO.: 193
   <211> 25
   <212> polynucleotide
   <213> artificial sequence
<220>
   <221> oligonucleotide
<400>
   actatctcca ccatggtgag cccag
<210> SEQ ID NO.: 194
   <211> 25
   <212> polynucleotide
   <213> artificial sequence
<220>
   <221> oligonucleotide
<400>
   ctgggctcac cacggtggag atagt
<210> SEQ ID NO.: 195
   <211> 25
   <212> polynucleotide
   <213> artificial sequence
<220>
   <221> oligonucleotide
<400>
   ctgggctcac catggtggag atagt
<210> SEQ ID NO.: 196
   <211> 25
   <212> polynucleotide
   <213> artificial sequence
<220>
   <221> oligonucleotide
<400>
   gggctctgtc cattgtcctc cccat
<210> SEQ ID NO.: 197
   <211> 25
   <212> polynucleotide
   <213> artificial sequence
<220>
   <221> oligonucleotide
<400>
   gggctctgtc cactgtcctc cccat
<210> SEQ ID NO.: 198
   <211> 25
   <212> polynucleotide
   <213> artificial sequence
<220>
   <221> oligonucleotide
<400>
   atggggagga caatggacag agccc
<210> SEQ ID NO.: 199
   <211> 25
   <212> polynucleotide
   <213> artificial sequence
<220>
   <221> oligonucleotide
<400>
   atggggagga cagtggacag agccc
<210> SEQ ID NO.: 200
   <211> 25
   <212> polynucleotide
   <213> artificial sequence
<220>
   <221> oligonucleotide
<400>
   tgcaacatgg ctagagactg ccggg
<210> SEQ ID NO.: 201
   <211> 25
   <212> polynucleotide
   <213> artificial sequence
<220>
   <221> oligonucleotide
<400>
   tgcaacatgg ctggagactg ccggg
<210> SEQ ID NO.: 202
   <211> 23
   <212> polynucleotide
   <213> artificial sequence
<220>
   <221> oligonucleotide
<400>
   gcaacatggc tagagactgc egg
<210> SEQ ID NO.: 203
   <211> 23
   <212> polynucleotide
   <213> artificial sequence
<220>
   <221> oligonucleotide
<400>
   gcaacatggc tggagactgc cgg
<210> SEQ ID NO.: 204
   <211> 23
   <212> polynucleotide
   <213> artificial sequence
<220>
   <221> oligonucleotide
<400>
   tgctgatgac ggtgtcatag gaa
<210> SEQ ID NO.: 205
   <211> 23
   <212> polynucleotide
   <213> artificial sequence
<220>
   <221> oligonucleotide
<400>
   tgctgatgac gatgtcatag gaa
<210> SEQ ID NO.: 206
   <211> 21
   <212> polynucleotide
   <213> artificial sequence
<220>
   <221> oligonucleotide
<400>
   gctgatgacg gtgtcatagg a
<210> SEQ ID NO.: 207
   <211> 21
   <212> polynucleotide
   <213> artificial sequence
<220>
   <221> oligonucleotide
<900>
   gctgatgacg atgtcatagg a
<210> SEQ ID NO.: 208
   <211> 23
   <212> polynucleotide
   <213> artificial sequence
<220>
   <221> oligonucleotide
<400>
   tccaaacttc actccatctc aag
<210> SEQ ID NO.: 209
   <211> 23
   <212> polynucleotide
   <213> artificial sequence
<220>
   <221> oligonucleotide
<400>
   tccaaacttc agtccatctc aag
<210> SEQ ID NO.: 210
   <211> 23
   <212> polynucleotide
   <213> artificial sequence
<220>
   <221> oligonucleotide
<400>
   cttgagatgg agtgaagttt gga
<210> SEQ ID NO.: 211
   <211> 23
   <212> polynucleotide
   <213> artificial sequence
<220>
   <221> oligonucleotide
<400>
   cttgagatgg actgaagttt gga
<210> SEQ ID NO.: 212
   <211> 25
   <212> polynucleotide
   <213> artificial sequence
<220>
   <221> oligonucleotide
<400>
   gccaagtgga ctgcgacaac ggctc
<210> SEQ ID NO.: 213
   <211> 25
   <212> polynucleotide
   <213> artificial sequence
<220>
   <221> oligonucleotide
<404>
   gccaagtgga ctacgacaac ggctc
<210> SEQ ID NO.: 214
   <211> 25
   <212> polynucleotide
   <213> artificial sequence
<220>
   <221> oligonucleotide
<400>
   gagccgttgt cgcagtccac ttggc
<210> SEQ ID NO.: 215
   <211> 25
   <212> polynucleotide
<113> artificial sequence
<220>
   <221> oligonucleotide
<400>
   gagccgttgt cgtagtccac ttggc
<210> SEQ ID NO.: 216
   <211> 25
   <212> polynucleotide
   <213> artificial sequence
<220>
   <221> oligonucleotide
<400>
   ctgctggcca gggacatgag gagct

<210> SEQ ID NO.: 217
   <211> 25
   <212> polynucleotide
   <213> artificial sequence
<220>
   <221> oligonucleotide
<400>
   ctgctggcca ggtacatgag gagct
<210> SEQ ID NO.: 218
   <211> 25
   <212> polynucleotide
   <213> artificial sequence
<220>
   <221> oligonucleotide
<400>
   agctcctcat gtccctggcc agcag
<210> SEQ ID NO.: 219
   <211> 25
   <212> polynucleotide
   <213> artificial sequence
<220>
   <221> oligonucleotide
<400>
   agctcctcat gtacctggcc agcag
<210> SEQ ID NO.: 220
   <211> 25
   <212> polynucleotide
   <213> artificial sequence
<220>
   <221> oligonucleotide
<400>
   ctcgccgcgg cggggactgc aggta
<210> SEQ ID NO.: 221
   <211> 25
   <212> polynucleotide
   <213> artificial sequence
<220>
   <221> oligonucleotide
<400>
   ctcgccgcgg cgaggactgc aggta
<210> SEQ ID NO.: 222
   <211> 25
   <212> polynucleotide
   <213> artificial sequence
<220>
   <221> oligonucleotide
<400>
   tacctgcagt ccccgccgcg gcgag
<210> SEQ ID NO.: 223
   <211> 25
   <212> polynucleotide
   <213> artificial sequence
<220>
   <221> oligonucleotide
<400>
   tacctgcagt cctcgccgcg gcgag
<210> SEQ ID NO.: 224
   <211> 25
   <212> polynucleotide
   <213> artificial sequence
<220>
   <221> oligonucleotide
<400>
   gaccatcttg gaggatgaaa agagg
<210> SEQ ID NO.: 225
   <211> 25
   <212> polynucleotide
   <213> artificial sequence
<220>
   <221> oligonucleotide
<400>
   gaccatcttg gacgatgaaa agagg
<210> SEQ ID NO.: 226
   <211> 25
   <212> polynucleotide
   <213> artificial sequence
<220>
   <221> oligonucleotide
<400>
   cctcttttca tcctccaaga tggtc
<210> SEQ ID NO.: 227
   <211> 25
   <212> polynucleotide
   <213> artificial sequence
<220>
   <221> oligonucleotide
<400>
   cctcttttca tcgtccaaga tggtc
<210> SEQ ID NO.: 228
   <211> 27
   <212> polynucleotide
   <213> artificial sequence
<220>
   <221> oligonucleotide
<400>
   gttttcctcg tcagatttgt ccttgca
<210> SEQ ID NO.: 229
   <211> 27
   <212> polynucleotide
   <213> artificial sequence
<220>
   <221> oligonucleotide
<400>
   gttttcctcg tcacatttgt ccttgca
<210> SEQ ID NO.: 230
   <211> 25
   <212> polynucleotide
   <213> artificial sequence
<220>
   <221> oligonucleotide
<400>
   ttttcctcgt cagatttgtc cttgc
<210> SEQ ID NO.: 231
   <211> 25
   <212> polynucleotide
   <213> artificial sequence
<220>
   <221> oligonucleotide
<400>
   ttttcctcgt cacatttgtc cttgc
<210> SEQ ID NO.: 232
   <211> 25
   <212> polynucleotide
   <213> artificial sequence
<220>
   <221> oligonucleotide
<400>
   ttgtccttgc agtcggggcc acta
<210> SEQ ID NO.: 233
   <211> 25
   <212> polynucleotide
   <213> artificial sequence
<220>
   <221> oligonucleotide
<400>
   ttgtccttgc agacggggcc accat
<210> SEQ ID NO.: 234
   <211> 23
   <212> polynucleotide
   <213> artificial sequence
<220>
   <221> oligonucleotide
<400>
   tgtccttgca gtcggggcca cca
<210> SEQ ID NO.: 235
   <211> 23
   <212> polynucleotide
   <213> artificial sequence
<220>
   <221> oligonucleotide
<400>
   tgtccttgca gacggggcca cca
<210> SEQ ID NO.: 236
   <211> 25
   <212> polynucleotide
   <213> artificial sequence
<220>
   <221> oligonucleotide
<400>
   agcccagtag cgtgagggct ctgtc
<210> SEQ ID NO.: 237
   <211> 25
   <212> polynucleotide
   <213> artificial sequence
<220>
   <221> oligonucleotide
<400>
   agcccagtag cgagagggct ctgtc
<210> SEQ ID NO.: 238
   <211> 25
   <212> polynucleotide
   <213> artificial sequence
<220>
   <221> oligonucleotide
<400>
   gacagagccc tcacgctact gggct
<210> SEQ ID NO.: 239
   <211> 25
   <212> polynucleotide
   <213> artificial sequence
<220>
   <221> oligonucleotide
<400>
   gacagagccc tctcgctact gggct
<210> SEQ ID NO.: 240
   <211> 25
   <212> polynucleotide
   <213> artificial sequence
<220>
   <221> oligonucleotide
<400>
   tcgccttgct cctcgccgcg gcggg
<210> SEQ ID NO.: 241
   <211> 25
   <212> polynucleotide
   <213> artificial sequence
<220>
   <221> oligonucleotide
<400>
   tcgccttgct ccccgccgcg gcggg
<210> SEQ ID NO.: 242
   <211> 25
   <212> polynucleotide
   <213> artificial sequence
<220>
   <221> oligonucleotide
<400>
   cccgccgcgg cgaggagcaa ggcga
<210> SEQ ID NO.: 243
   <211> 25
   <212> polynucleotide
   <213> artificial sequence
<220>
   <221> oligonucleotide
<400>
   cccgccgcgg cggggagcaa ggcga
<210> SEQ ID NO.: 244
   <211> 25
   <212> polynucleotide
   <213> artificial sequence
<220>
   <221> oligonucleotide
<400>
   acggctacag ctacccctcg gtgag
<210> SEQ ID NO.: 245
   <211> 25
   <212> polynucleotide
   <213> artificial sequence
<220>
   <221> oligonucleotide
<400>
   cggctacagc taccccctcg gtgag
<210> SEQ ID NO.: 246
   <211> 25
   <212> polynucleotide
   <213> artificial sequence
<220>
   <221> oligonucleotide
<400>
   ctcaccgagg ggtagctgta gccgt
<210> SEQ ID NO.: 247
   <211> 25
   <212> polynucleotide
   <213> artificial sequence
<220>
   <221> oligonucleotide
<400>
   ctcaccgagg gggtagctgt agccg
<210> SEQ ID NO.: 248
   <211> 25
   <212> polynucleotide
   <213> artificial sequence
<220>
   <221> oligonucleotide
<400>
   cccaggagac gtgctgtgag tcccc

<210> SEQ ID NO.: 249
   <211> 25
   <212> polynucleotide
   <213> artificial sequence
<220>
   <221> oligonucleotide
<400>
   cccaggagac gtactgtgag tcccc
<210> SEQ ID NO.: 250
   <211> 25
   <212> polynucleotide
   <213> artificial sequence
<220>
   <221> oligonucleotide
<400>
   ggggactcac agcacgtctc ctggg
<210> SEQ ID NO.: 251
   <211> 25
   <212> polynucleotide
   <213> artificial sequence
<220>
   <221> oligonucleotide
<400>
   ggggactcac agtacgtctc ctggg
<210> SEQ ID NO.: 252
   <211> 25
   <212> polynucleotide
   <213> artificial sequence
<220>
   <221> oligonucleotide
<400>
   ctccccatcg gtaagcgcgg gccgg
<210> SEQ ID NO.: 253
   <211> 25
   <212> polynucleotide
   <213> artificial sequence
<220>
   <221> oligonucleotide
<400>
   ctccccatcg gtcagcgcgg gccgg
<210> SEQ ID NO.: 254
   <211> 25
   <212> polynucleotide
   <213> artificial sequence
<220>
   <221> oligonucleotide
<400>
   ccggcccgcg cttaccgatg gggag
<210> SEQ ID NO.: 255
   <211> 25
   <212> polynucleotide
   <213> artificial sequence
<220>
   <221> oligonucleotide
<400>
   ccggcccgcg ctgaccgatg gggag
<210> SEQ ID NO.: 256
   <211> 25
   <212> polynucleotide
   <213> artificial sequence
<220>
   <221> oligonucleotide
<400>
   ccagtacatg aagctggtgg gaga
<210> SEQ ID NO.: 257
   <211> 25
   <212> polynucleotide
   <213> artificial sequence
<220>
   <221> oligonucleotide
<400>
   tcttgatctt ggcctgggga cagag
<210> SEQ ID NO.: 258
   <211> 23
   <212> polynucleotide
   <213> artificial sequence
<220>
   <221> oligonucleotide
<400>
   cagtacatga agctggtggg agg
<210> SEQ ID NO.: 259
   <211> 23
   <212> polynucleotide
   <213> artificial sequence
<220>
   <221> oligonucleotide
<400>
   cttgatcttg gcctggggac aga

## Description

### Field of the invention

The present invention relates an "in vitro" method for detecting individuals who are predisposed to the disease named Familial Hypercholesterolemia, and more particularly to a method for detecting the presence or absence of the mutation [313+1 inst in the LDL-r gene represented by SEQ ID NO:1] associated with Familial Hypercholesterolemia

### Background of the invention

According to the WHO definition, atherosclerosis is a variable combination of changes in the intimae of the arteries involving focal accumulation of lipids and complex compounds with blood and its constituents, accompanied by fibrous tissue formation, calcification and associated changes in the media.

Atherosclerosis may be considered as a special form of arteriosclerosis with pathogenic significant deposition of lipids in the arterial wall. Most forms of arteriosclerosis involve fatty degeneration of vascular wall, the terms "arteriosclerosis" and "atherosclerosis" may be used synonymously (Assmann G. in "Lipid Metabolism and Atherosclerosis" Schattauer Verlag GMbH, Stuttgart 1982:1).

Lipids are almost completely insoluble in aqueous solutions. Lipoproteins represent the functional unit of transport for water-insoluble lipids in the blood. Lipoproteins are divided into various categories according their density, as determined by ultracentrifugation (Havel RJ et al. J Clin Invest 1955, 34:1345). Low density-lipoproteins (LDL) (d=1.019-1.063 g/mL) transport the bulk of the cholesterol in the blood. They are composed of about 75% lipid (primarily cholesterol, cholesteryl esters and phospholipids) and 25% protein. Approximately 70% of the total cholesterol is transported in the blood by LDL particles.

Hypercholesterolemia (i.e., elevation of plasma low density lipoprotein-cholesterol (LDL-c)) is one of the most critical risk factors in the onset and the progress of atherosclerosis. More than half of all deaths in Western society are related to atherosclerosis cardiovascular diseases (Murray CJL and Lopez AD. Lancet 1997; 349:1269-1276).

Familial hypercholesterolemia (FH: MIM#143890) is an autosomal co-dominant inherited disease of lipoprotein metabolism caused by low density lipoprotein receptor (LDL-r) gene mutations, a cell-surface protein that mediates the specific uptake and degradation of plasma LDL (Goldstein JL, Brown MS Ann Rev Cell Biol 1985; 1:1-39). The penetrance of FH is almost 100% meaning that half of the offspring of an affected parent has a severely elevated plasma cholesterol level from birth onwards, with males and females equally affected (Goldstein JL, Brown MS. The metabolic basis of inherited disease, Scriver CR, Beaudet AL, Sly WS, Valle D, eds. McGraw Hill New York 6th edition, 1989; 1215-1250). FH affected individuals display arcus lipoides corneae, tendon xanthomas and premature symptomatic coronary heart disease (Scientific Steering Committee on behalf of the Simon Broome register Group. Atherosclerosis 1999; 142: 105-115). FH is one of the most common inherited disorders with frequencies of heterozygote and homozygote estimated to be 1/500 and 1/1,000,000, respectively.

In certain populations, a small number of mutations predominate due to founder effects and therefore, the frequency of heterozygous FH is higher, these populations include French Canadians (Leitersdorf E et al. J Clin Invest 1990; 85:1014-1023), Christian Lebanese (Lehrman MA et al. J Biol Chem 1987; 262:401-410) Druze (Landsberger D et al. Am J Hum Genet 1992; 50: 427-433) Finns (Koivisto UM et al. J Clin Invest 1992; 90:219-228) South African Afrikaner (Kotze MJ et al. Ann Hum Genet 1991; 55:115-121), and Ashkenazi Jews of Lithuanian descent (Meiner V et al. Am J Hum Genet 1991; 49:443-449).

FH heterozygous patients display a twofold increase in plasma cholesterol (generally above the 95^{th} percentile value for population). In patients with FH the age-standardised and sex-standardised mortality ratios are four to five times higher than in the general populations (Scientific Steering Committee on behalf of the Simon Broome Register Group. Atherosclerosis1999; 142: 105-115). Patients who have inherited two mutant at the LDL-r locus are termed "FH homozygotes" or "FH compound heterozygotes". Few or no functional LDL-r is synthesized in such individuals. This abnormalities lead to a six-fold to eight-fold elevation in plasma LDL-c levels as early as the intrauterine gestation, as a consequence, coronary heart disease typically occurs before the age of 20 years (Goldstein JL et al. N Engl J Med 1983; 309:288-296). If individuals with heterozygous or homozygous FH could be diagnosed before they develop symptomatic disease, they could be treated preventively to reduce their risk of myocardial infarction.

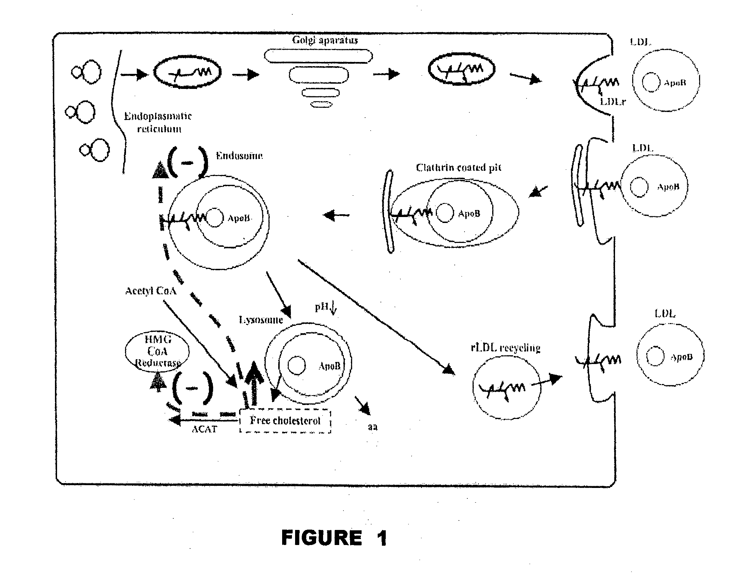

The LDL-r is a ubiquitous trans-membrane glycoprotein of 839 amino acids that mediates the transport of LDL into cells via endocytosis (Goldstein J and Brown M J Biol Chem 1974; 249:5153-5162) (Figure 1).

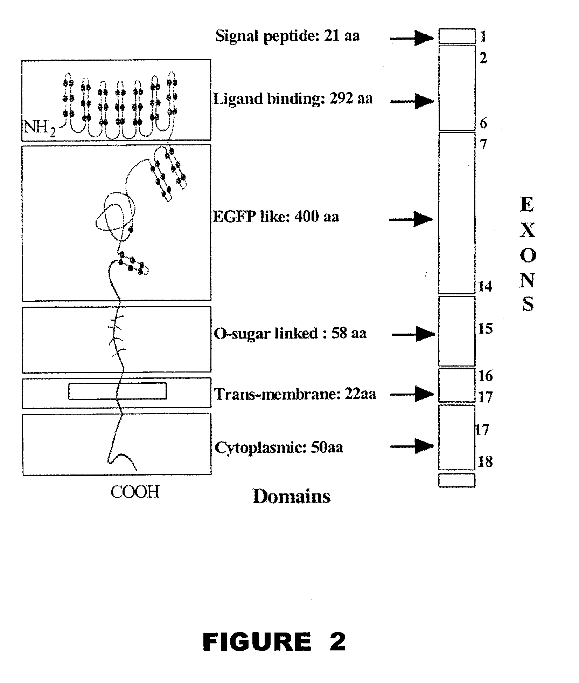

The LDL-r gene lies on the short arm of chromosome 19p13.1-13.3 (Yamamoto T et al. Cell 1984;39: 27-38), spans 45,000 base pairs (bp). It contains 18 exons and 17 introns encoding the six functional domains of the mature protein: Signal peptide, ligand-binding domain, epidermal growth factor (EGF) precursor like, O-linked sugar, trans-membrane and cytoplasmic domain (Sundhof T et al. Science 1985; 228:893-895) (Figure 2).

The LDL-r production is tightly regulated by a sophisticated feedback mechanism that controls the transcription of the LDL-r gene in response to variations in the intracellular sterol concentration and the cellular demand for cholesterol (Sudhof TC et al J Biol Chem 1987; 262:10773-10779). DNA motifs essential for transcriptional regulation of the LDL-r gene is located within 177 bp of the proximal promoter (Sudhof TC et al. J Biol Chem 1987; 262: 10773-10779). This region contains all the cis-acting elements for basal expression and sterol regulation and includes three imperfect direct repeats of 16 bp each, repeats 1 to 3. Repeat 1 and 3 containing binding sites for the transcriptional factor Sp1 and contribute to the basal expression of the gene but requires the contribution of the repeat 2 for strong expression (Dawson PA et al. J Biol Chem 1988; 263; 3372-3379). Repeat 2 contains a 10 bp regulatory element, SRE-1, (Smith JR et al. J Biol Chem 1990; 265:2306-2310) that enhances transcription when the intracellular sterol concentration is low through interaction with a transcriptional factor designated as SREBP-1. To date, several naturally-occurring mutations have been mapped to the transcriptional regulatory elements of the LDL-r gene (Hobbs HH, et al. Hum Mutat 1992; 1:445-466; Koivisto UM, et al ProcNatl Acad Sci USA, 1994; 91:10526-10530), Mozas P, et al J Lipid Res 2002; 43:13-18, http.//www.ucl.ac.uk/fh; http.//www.umd.necker.fr).

Exon 1 encodes the signal peptide; a hydrophobic sequence of 21 amino acid. This peptide is cleaved from the protein during the translocation into the endoplasmic reticulum. Several frameshift, missense and nonsense mutation has been described in this exon (http.//www.uci.ac.uk/fh; http.//www.umd.necker.fr)

Exons 2 to 6 encode the ligand binding domain, which consists of seven tandem repeats of 40 amino acid each. The structure of the ligand binding domain has been partially elucidated (Jeon H et al. Nature Struc Biol 2001; 8:499-5049). There are a cluster of negatively charged amino acids, Asp-X-Ser-Asp-Glu in each repeat and six cysteine residues that forms three disulfide bonds.

The second domain of the human LDL-r consist a 400 amino acid sequence, encoded by exons 7 to 14. This sequence shows a 33% of homology of the human epidermal growth factor precursor (EGFP). Like the ligand binding domain, this region also contains three repeats of 40 amino acids with cysteine-rich sequences. The first two repeats, designated A and B, are contiguous and separated from the third repeat, C, by a 280 amino acid sequence that contains five copies of the conserved motif YWTD (Tyr-Trp-Thr-Asp). The EGFP like domain is required for the acid-dependent dissociation of the LDL particles from the LDL-r and clathrin coat pits that takes place in the endosome during receptor recycling. Of the all mutations described to date, approximately 55% are located in the EGFP-homology region of the LDL-r and 35% among the YWTD repeats (http.//www.ucl.ac.uk/fh).

The third domain of the LDL-r, is a region rich in threonine and serine residues, that is encoded by exon 15. The function of this domain is unknown, but has been observed that this region serve as attachment sites for O-linked carbohydrate chains. This region show minimal sequence conservation among six species analyzed (Goldstein et al. In The Metabolic and Molecular Basis of Inherited Disease. Sciver CR, Beaudet AL, Sly WS, Valle D. 7th Edition. McGraw Hill, 1995: 1981-2030). It is thought that this domain play a role in the stabilization of the receptor.

The trans-membrane domain that contains 22 hydrophobic amino acids is coded by exon 16 and the 5'end of exon 17. This domain is essential for anchor the LDL-r to the cell membrane.

The cytoplasmic domain of the LDL-r, that comprises 50 amino acid residues, is encoded by the remainder 3' region of the exon 17 and the 5' end of the exon 18. This domain contains two sequence signals for targeting the LDL-r to the surface and for localizing the receptor in coated pits (Yokode M, et al. J Cell Biol 1992;117: 39-46). This domain is the most conserved region of the LDL-r, which is more than 86% identical among six species (Goldstein et al. in The Metabolic and Molecular Basis of Inherited Disease. Sciver CR, Beaudet AL, Sly WS, Valle D, 7th Edition. McGraw Hill, 1995:1981-2030).

The naturally-occurring LDL-r mutations have been classified into 5 classes, including null alleles, transport defective alleles, binding defective alleles, internalization-defective alleles and recycling-defective alleles (Hobbs HH, et al. Hum Mutat 1992; 1:445-466). In many cases each category is associated with mutations in regions of the gene that code for one particular domain of the protein.

The heterogeneity observed in FH patients in relation to plasma LDL-c levels and coronary heart disease has been suggested due to differences in the nature of the mutation in the LDL-r gene and several studies have been published in support of this (Koivisto PVI et al, Sun XM et al. Arterioscler Thromb Vas Biol 1993; 13:1680-1688, Kotze MJ et al. Arterioscler Thromb Vas Biol 1993; 13:1460-1468; Gudnason V et al. Arterioscler Thromb Vas Biol 1997;17:3092-3101). On the other hand, in FH heterozygotes, the LDL-c lowering response to hydroxy-methylglutaryl coenzyme A (HMGCoA) reductase inhibitors may depend on the nature of the mutation in the LDL-r gene (Leisterdorf E et al. Circulation 1993; 87:35-44; Jeenah M et al. Atherosclerosis 1993; 98:51-58, Sijbrands EJG et al. Atherosclerosis 1998;136: 247-254).

The primary ligand for the receptor is LDL, which contains a single copy of apolipoprotein B-100 (Goldstein J and Brown M J Biol Chem 1974;249:5153-5162). This apolipoprotein posses short segments that are rich in basic amino acids and that bind to the receptor negatively charged cluster (Boren J et al. J Clin Inves 1998; 101: 1084-1093). Several mutations located in the apolipoprotein B gene alter the functional activity of the protein and decrease its binding to the LDL receptor, delaying the clearance of LDL particles and leading to accumulation of LDL cholesterol in the circulation. To date, four mutations causing FDB have been identified, all of them located in the LDL-r binding domain of the apo B-100 protein (residues 3130-3630): R3480W, R3500Q, R3500W and R3531C (Soria L et al. Proc Natl Acad Sci USA 1989; 86: 587-591; Pullinger CR,et al. J Clin Invest 1995; 95:1225-1234; Gaffney D, et al. Arterioscler Thromb Vasc Biol 1995; 15:1025-1029; Boren J, et al. J Biol Chem 2001; 276; 9214-9218). The CGG-to-CAG mutation at codon for amino acid 3500, resulting in a glutamine substitution for arginine (R3500Q), is the most frequent alteration causing Familial Defective apolipoprotein B-100 (FDB). Patients heterozygous for the apoB-3500 mutation are usually hypercholesterolemic, although serum cholesterol concentrations can vary from those found in FH to only modest elevations (Tybjaerg-Hansen A, et al. Atherosclerosis 1990; 80:235-242; Hansen PS, et al. Arterioscl Throm Vasc Biol 1997; 17:741-747). Since clinical and biochemical diagnosis often are inaccurate, only molecular diagnosis analyzing LDL receptor and apo B genes could distinguish between FDB and FH.

Clinical diagnosis of FH is based in laboratory findings, physical signs (xanthomas) and patient's history. By weighing the occurrence of clinical signs a diagnostic scoring table can be constructed for FH and this clinical diagnosis method has been recommended by WHO (Familial hypercholesterolemia. Report of a second WHO consultation. The International MedPed FH Organization, Geneva 1998).

Some patients with hypercholesterolemia may not have diagnosed by clinical criteria as FH because the family history is misleading or inaccurate. Other patients may not be diagnosed with FH because they have only moderate elevation of plasma cholesterol concentration and have not signs of tissue cholesterol deposition as xanthomas, arcus lipoids corneae or xanthelasmas. It has been demonstrated that the best available cut-off point to diagnose the FH in relatives by total plasma cholesterol concentration is the age-specific and the sex-specific 90^{th} percentile (Umans-Eckenhausen MAW et al. Lancet 2001; 357:165-168. However 18 % of FH patients have total plasma cholesterol concentration below this percentile, and, on the other hand, the proportion of false positives was also 18% when the 90^{th} percentile was used as cut-off point. Therefore, this percentage of patients would have been missed if only cholesterol concentration had been used as diagnostic criteria.

It was found that more than 50% of FH patients were not receiving lipid lowering therapy and dietary counseling because they were not aware of their condition (Williams RR et al. Am J Cardiol 1993; 72:18D-24D).

The elucidation of the molecular basis of FH has made diagnosis at the DNA level feasible in the vast majority of cases. Demonstration of an underlying defect in the LDL-r gene constitutes in fact the definite confirmation of the diagnosis (Familial hypercholesterolemia. Report of a second WHO consultation. The International MedPed FH Organization, Geneva 1998). Although accurate diagnosis of FH is possible by means of molecular methods, their use in heterogeneous populations is limited at present owing to mutational heterogeneity of the LDL-r gene.

In application PCT WO-88/03175 (Biotechnology Research Partners, Ltd.) a method is claimed for the diagnosis of atherosclerosis, based on detection of the presence or absence of various polymorphisms in the gene region of the apolipoprotein AO-CIII-AIV, or in the genes apoB, apocl, apoAII, as well as in the LDL receptor gene. Specifically for this gene, utilization of the polymorphisms Cfr131 and BstEII is presented.

Another document of interest is Japanese patent JP-10099099 which refers to the use of a mutation in the codifier triplet of the amino acid 109, specifically the insertion of a C, for the diagnosis of abnormalities in the LDL receptor gene, although familial hypercholesterolemia is not specifically mentioned.

Finally, U.S. Patents US-4.745.060 and US-4.966.837, both of the University of Texas, present methods for the diagnosis of familial hypercolesterolemia on the basis of mutations in the LDL receptor gene. However, what is claimed in the first of them are sequences corresponding to the "normal" gene, presenting a particular example of a mutation that is defined by the restriction map change with Xba I. In the second patent, on its part, the use of various restriction enzymes is claimed (Eco RI, Asp 718, Taq I, Bam HI, Xba I, Inf.I, Bgl II, Cla I, Eco RV, Kpn I, Pvu II, Sph I, Sst I, Sst II, Stu I, Xho I, Nde I and Nsi I) in a method for determining mutations in the LDL-r gene, based on observing the alteration of the restriction model with these enzymes compared to the model corresponding to the normal gene.

The closest patent document to the invention is WO02/06467, in which a method is described, for the detection of errors in the lipidic metabolism based on a series of mutations and polymorphisms of the LDL-r gene. However, none of the mutations or polymorphisms described in said patent coincides with those claimed in the present application.

### Detailed description

The nomenclature of the mutations and polymorphisms is defined in
- Antoranakis S. E. and the Nomenclature Working Group, Recommendations for Nomenclature Systems for Human Gene Mutations. Human Mutation 11:1-3; 1998
- Dunnen JT, Antoranakis S.E. Mutation Nomenclature Extensions and Suggestions to describe Complex Mutations: A Discussion. Human Mutation 15: 7-12, 2000.

Similarly the concept of the polymorphisms is defined in
- Harris H. The Principles of Human Biochemical Genetics 3rd Edition. Amsterdam. North-Holland 1980.
- Beauder AL, Scriver CL, Sly WS, Valle D. Genetics, Biochemistry and Molecular Basis of Variant Human Phenotypes, in The Metabolic and Molecular Bases of Inherited Disease. Editores Beaudet AL, Scriver CR, Sly WS, Valle D 7th Edition. Page 53, MacGraw Hill. New York 1995.

There has been detected, isolated and characterized a whole series of new mutations which are detailed below. Similarly, a whole series of mutations and polymorphisms already described, have combined with them to analyze the likelihood of an individual developing familial hypercholesterolemia. All of the mutations and polymorphisms herein described which relate to development of familial hypercholesterolemia are produced in the gene sequence SEQ ID NO: 1 corresponding to the low density lipoproteins receptor gene (LDL-r). That is to say, all of the mutations are produced in the same gene, are used in the same testing device, extra-corporeally and in vitro, the likelihood of developing the same disease, which supports the unitary nature of the invention.

In Table I all of the new mutations detected are detailed, according to the nomenclature scientifically approved and detailed in the publications mentioned above. Likewise, they are provided with an alpha-numerical code.

In Table II mutations are detailed, already described and known, whose use in combination with the mutations of Table I, in testing devices in vitro for diagnosis of the familial hypercholesterolemia, is herein described. Similarly, in analogous manner to that mentioned for the known mutations, in Table III polymorphisms are detailed.

The amino acid mutations are represented in one-letter codes which have their equivalence according to Table IV.

**TABLE I**

| **MUTATION** | **ID** |
|---|---|
| (-23)A>C | M002 |
| 1054 del11 | M006 |
| 108delC | M008 |
| 1197del9 | M009 |
| 1207delT | M010 |
| 1432delG | M012 |
| 191-2delAinsCT | M016 |
| 2184delG | M020 |
| 231delC | M022 |
| 2399del5/ins4 | M024 |
| 313+1insT | M027 |
| 338del16 | M029 |
| 509insC | M030 |
| 675del15 | M032 |
| 684dup12 | M034 |
| 941-39C>T | M041 |
| C195R | M046 |
| C255G | M0100 |
| C319Y | M050 |
| D157G | M059 |
| D630N | M063 |
| E291X | M068 |
| H635N | M096 |
| N59K | M074 |
| T41M | M097 |
| W515X | M098 |
| Y379X | M092 |
| Y421X | M093 |
| T433N | M105 |
| 818del8 | M110 |
| 1423delGC/insA | M111 |
| 1204insT | M112 |
| 451del3 | M115 |
| G516X | M117 |
| 2389+4A>G | M120 |
| 1815del11 | M121 |
| 1186+5G>A | M129 |
| T740M | M131 |
| I771T | M135 |
| R279G | M138 |
| T446I | M141 |
| H562Q | M142 |
| C74Y | M145 |
| D686Y | M147 |
| G(-2)R | M149 |
| E579D | M150 |
| S205C | M151 |
| D200V | M153 |
| V766E | M154 |
| L(-6)P | M155 |
| 2544insC | M156 |
| C42Y | M157 |
| 2389+3A>C | M160 |
| [1587-5del5;1587del31] | M161 |

**TABLE II**

| **MUTATION** | **ID** | **MUTATION** | **ID** |
|---|---|---|---|
| 2393del9 | M001 | C646Y | M053 |
| (-42)C>G | M003 | C677Y | M054 |
| (-49)C>T | M004 | C68W | M055 |
| 1045delC | M005 | C74G | M056 |
| 1061-8T>C | M007 | C95R | M057 |
| A378T | M0102 | D151N | M058 |
| C358R | M0104 | D200G | M060 |
| 1358+1 G>A | M011 | D200Y | M061 |
| 1706-10G>A | M014 | D280G | M062 |
| 1845+1 G>C | M015 | E10X | M064 |
| 2085del19 | M017 | E246A | M066 |
| 211del G | M018 | E256K | M067 |
| 2140+5G>A | M019 | F634L | M069 |
| 2207insT | M021 | G322S | M070 |
| 2390-1 G>C | M023 | G352D | M071 |
| 313+1G>C | M025 | G571 E | M072 |
| 313+1 G>A | M026 | N543H | M073 |
| 313+2insT | M028 | N804K | M075 |
| 518 del G | M031 | Q12X | M076 |
| 7delC | M035 | Q133X | M077 |
| 872delC | M036 | Q357P | M078 |
| 884delT | M038 | Q427X | M079 |
| 920ins4 | M039 | Q71 E | M080 |
| A519T | M042 | R395Q | M081 |
| C113W | M043 | R574W | M082 |
| C127R | M045 | R612C | M083 |
| C255X | M047 | S156L | M084 |
| C281Y | M048 | S205P | M085 |
| C297F | M049 | T413K | M086 |
| C347Y | M051 | T705I | M087 |
| C371X | M052 | V502M | M089 |
| W (-18)X | M090 | | |
| W541 X | M091 | | |
| D679E | M094 | | |
| 1359-1 G>A | M099 | | |
| 681ins21 | M033 | | |
| C122X | M044 | | |
| V408M | M088 | | |
| G528D | M106 | | |
| D412H | M107 | | |
| N619N | M108 | | |
| E80K | M109 | | |
| L534P | M113 | | |
| L621S | M114 | | |
| C356Y | M116 | | |
| R329X | M119 | | |
| G248D | M122 | | |
| C201Y | M125 | | |
| 313+5G>A | M126 | | |
| C358Y | M127 | | |
| C331 R | M128 | | |
| D157N | M130 | | |
| V776M | M134 | | |
| P664L | M136 | | |
| W462X | M137 | | |
| Q328X | M139 | | |
| L584P | M140 | | |
| R395W | M143 | | |
| G314V | M144 | | |
| W469X | M146 | | |
| P678L | M148 | | |
| R612H | M152 | | |
| R236W | M159 | | |

**TABLE III**

| **POLYMORPHISMS** | **ID** |
|---|---|
| 81T>C BstUI Exon 2 | P1 |
| 1060+10G>C SmaI Exon 7 | P2 |
| 1171G>A StuI Exon 8 | P3 |
| 1413G>A Ddel Exon 10 | P4 |
| 1617C>T BstNI Exon 11 | P5 |
| 1725C>T SSCP Exon 12 | P6 |
| 1771C>T HincII Exon 12 | P7 |
| 1959 T>C AvalI Exon 13 | P8 |
| 2232G>A Mspl Exon 15 | P9 |

**TABLE IV**

| **AMINOACID NOMENCLATURE** | | |
|---|---|---|
| Alanine | Ala | A |
| Aspartic acid | Asp | D |
| Glutamic acid | Glu | E |
| Glycine | Gly | G |
| Phenylalanine | Phe | F |
| Serine | Ser | S |
| Tyrosine | Tyr | Y |
| Cysteine | Cys | C |
| Tryptophan | Trp | W |
| Leucine | Leu | L |
| Proline | Pro | P |
| Histidine | His | H |
| Glutamine | Gln | Q |
| Arginine | Arg | R |
| Isoleucine | Ile | I |
| Methionine | Met | M |
| Threonine | Thr | T |
| Asparagine | Asn | N |
| Lysine | Lys | K |
| Valine | Val | V |
| Stop codon | Ter | X |

The diagnosis assay or DNA-array "biochip" is a slide with a large amount of probes onto its surface. The sequence of each synthetic oligonucleotide probe is shown in the sequences list. The probes are able to hybridize with the mutant sequences included in Tables I to III. The methodology involved in the detection of each mutation is:

### Printing of glass slides

- A microarrayer, spotter or oligonucleotide printer spots the designed probes for each mutation onto an aminosilanized glass slide using DMSO as printing buffer.
- During the printing process, temperature and humidity of spotting are controlled.

### Post-spotting process

- The slides are processed, post-spotting by UV crosslinking.

### Target-DNA preparation

- Genomic DNA of each patient is extracted from a blood sample of 300 µl by using a filtration method.
- A multiplex-PCR reaction is performed allowing amplification of the promoter and 18 exons of the LDL receptor gene
- A biotinylated nucleotide is incorporated during the PCR process. As an indirect labeling method, a final step of staining with fluorophore-streptavidin is required after hybridization.
- PCR products are electrophoresed and visualized in agarose gel.
- Target-DNA is fragmented.
- Hybridization buffer is added to the fragmented PCR products.
- Denaturation step takes place at 95°C 15 min.

### Hybridization

- Hybridization is carried out in an automatic hybridization station.
- The glass slide is prehybridized.
- The hybridization solution is applied onto the glass slide.
- One hour is the hybridization time.
- The glass slide is washed for three times and dried.

### Microarray scanning

- Microarrays images are captured using a confocal fluorescent scanner, equipped with a laser.

### Microarray quantification

- Hybridization signal from each oligonucleotide probe is obtained using software from the scanner.
- The hybridization signal of the oligonucleotides used for the genotyping of each mutation allows us to determine if the DNA sample carries the mutation.

Two pairs of oligonucleotides has been designed for each mutation. Each probe pair consists of one probe specific for the wild-type allele and a second probe specific for the mutant allele. The target base is defined as the nucleotide where the DNA sequence variation is found. This target nucleotide is always located in the central position of the oligonucleotides in order to maximize the specificity of hybridization.

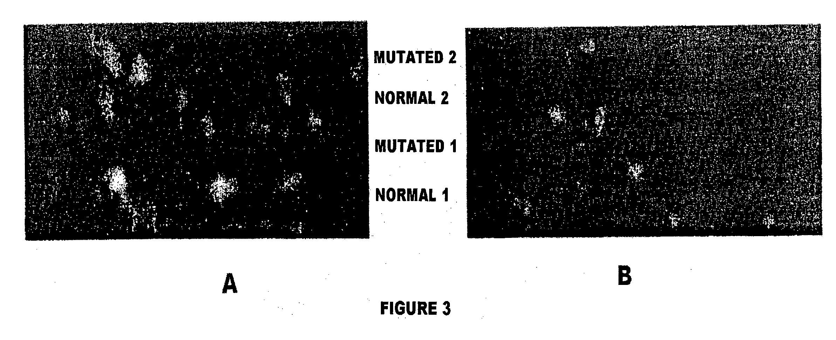

A wild-type subject presents no mutant allele (Figure 3A). Therefore, the hybridization signal of the probes specific for the mutant allele is zero or lowers than the hybridization signal of the probes specific for the normal allele.

A heterozygous mutant patient presents the wild-type and the mutant allele. Then, the hybridization signal of the probes specific for the mutant allele is equivalent to the hybridization signal of the probes specific for the normal allele.

The hybridization of labeled PCR fragments, which has been amplified from DNA of subjects to be analyzed, demonstrate that the DNA-array image in Figure 3A corresponds with a wild-type subject for the LDL receptor E256K gene mutation. However, the DNA-array image in Figure 3B is from a heterozygous mutant patient for this mutation. Consequently, the heterozygous mutant patient is given a definitive genetic diagnosis of Familial Hypercholesterolemia

### EXAMPLE 1: Identification of mutations located in exon 1 of the LDLr gene.

A 215 bp fragment of exon 1 of the LDLr gene was amplified by polymerase chain reaction (PCR) using the primers Ex1F (SEQ ID NO: 2) and Ex1R (SEQ ID NO: 3).

DNA (500ng) was amplified in a 50 µL reaction mixture containing 20mM Tris-HCl, pH 8.4, 50 mM KCI, 1.5 mM MgCL₂, 200 µM each dNTP, 0.2 µM each primer and 1.5 units of Taq DNA polymerase (Gibco BRL, Carlsbad, CA, USA). The amplification cycle was: 10 min of denaturation at 96°C, followed by 35 cycles of denaturation at 94°C for 1 min, annealing at 59°C for 1 min, and elongation at 74°C for 2 min, and a final extension of 72°C for 10 min.

PCR products were analyzed by Single Strand Conformation Polymorphisms (SSCP). Those fragments that showed abnormal SSCP patterns were sequenced using an automated CEQ 2000XL DNA Analysis System (Beckman Coulter, Palo Alto, CA, USA). The presence of the identified mutations was subsequently determined by restriction analysis and by using the device, microarray, described previously, "biochip".

### (-23)A>C mutation analysis

This mutation creates a new Avall digestion site. Fifteen µL of PCR sample were digested with 15 units of Avall in a total volume of 30 µL according to the protocol described by the manufacturer (NEB, Beverly, MA, USA). The fragments obtained had a length of 148 and 67 bp for normal alleles and 93, 67 and 55 bp for mutant alleles. These fragments were electrophoresed in 8% polyacrilamide gel and were visualised by staining with ethidium bromide. Alternatively, this mutation could be analyzed with the microarray ("biochip") by spotting onto the slide the oligonucleotides: ID NO: 36, SEQ ID NO: 37, SEQ ID NO: 38 and SEQ ID NO: 39.

(-23)A>C mutation was detected in a 60 years old woman with arcus corneae and xanthelasmas that has been diagnosed as having familial hypercholesterolemia with a diagnostic score of 8 points (Familial Hypercholesterolemia. Report of a second WHO consultation. The International MedPed FH Organization, Geneva 1998). No evidence of premature cardiovascular event was detected in her family. Analysis of her fasting serum without the use of lipid lowering drugs gave us the following results: TC (352 mg/dL) and LDL-c (271 mg/dL) with normal TG and HDLc levels. Treatment with simvastatin (20mg/day) lowered her TC and LDL-c levels to 251 and 171 mg/dL respectively.

### L(-6)P mutation analysis

This mutation (47T>C, CTC>CCC, Leu(-6)Pro) was identified by DNA sequencing of the 215 bp fragment from exon 1 during screening for mutations in the LDL-r gene in subjects clinically diagnosed as FH. Purified PCR product from DNA sample were directly sequenced in both directions using the amplification primers Ex1 F (SEQ ID NO:2) and Ex1R (SEQ ID NO:3) and the kit CEQ 2000 Dye Terminator Cycle Sequencing with Quick Start (Beckman Coulter, Palo Alto, CA, USA) according to the protocol described by the manufacturer. Sequences were detected using the CEQ 8000 Genetic Analysis System (Beckman Coulter, Inc. Fullerton), and analyzed with CEQ 8000 software. The 47 T>C change was confirmed by sequencing a second PCR product. Alternatively, this mutation could be analyzed with the microarray ("biochip") by spotting onto the slide the oligonucleotides: ID NO: 240, SEQ ID NO: 241, SEQ ID NO: 242 and SEQ ID NO: 243.

L(-6)P mutation was detected in a 47 years old woman with arcurs lipoides corneae that has been previously diagnosed clinically as having familial hypercholesterolemia with a score of 9 points (Familial Hypercholesterolemia. Report of a second WHO consultation. The International MedPed FH Organization, Geneva 1998). Premature cardiovascular disease was detected in two uncles at 24 and 33 years old respectively. Her father had hypercholesterolemia with plasma cholesterol above the 95^{th} percentile value for Spain population. Analysis of her fasting serum lipids without the use of lipid lowering drugs were TC 420 mg/dl, LDL-c 320 mg/dL, TG 155mg/dL and c-HDL-c 49 mg/dL. Treatment with atorvastatin (15mg/day) lowered her TC and LDL-c levels to 289 and 233 mg/dL respectively.

### G(-2)R mutation analysis

This mutation ((58G>A, GGG>AGG, Gly(-2)Arg) was identified by DNA sequencing of the 215 bp fragment from exon 1 during screening for mutations in the LDL-r gene in subjects clinically diagnosed as FH. Purified PCR product from DNA sample were directly sequenced in both directions using the amplification primers Ex1 F (SEQ ID NO:2) and Ex1R (SEQ ID NO:3) and the kit CEQ 2000 Dye Terminator Cycle Sequencing with Quick Start (Beckman Coulter, Palo Alto, CA, USA) according to the protocol described by the manufacturer. Sequences were detected using the CEQ 8000 Genetic Analysis System (Beckman Coulter, Inc. Fullerton), and analyzed with CEQ 8000 software. The 58G>A change was confirmed by sequencing a second PCR product. Alternatively, this mutation could be analyzed with the microarray ("biochip") by spotting onto the slide the oligonucleotides: SEQ ID NO: 220, SEQ ID NO: 221, SEQ ID NO: 222 and SEQ ID NO: 223.

G(-2)R mutation was identified in a 34 years old woman with arcurs corneae and hypercholesterolemia. Their score for FH clinical diagnostic was 10 points (Familial Hypercholesterolemia. Report of a Second WHO Consultation. The International MedPed FH Organization, Geneva 1998). Plasma lipid levels before treatment were: TC 354 mg/dL, LDL-c 264 mg/dL, normal TG and HDL-c of 64 mg/dL. Her mother had also hypercholesterolemia with TC of 400mg/dL.

### EXAMPLE 2: Identification of mutations located in exon 2 of the LDLr gene.

A 183 bp fragment of exon 2 of the LDLr gene was amplified by polymerase chain reaction (PCR) using the following primers: Ex2F (SEQ ID NO: 4) and Ex2R (SEQ ID NO: 5).

DNA (500ng) was amplified in a 50 µL reaction mixture containing 20mM Tris-HCl, pH 8.4, 50 mM KCI, 1.5 mM MgCL₂, 200 µM each dNTP, 0.2 µM each primer and 1.5 units of Taq DNA polymerase (Gibco BRL, Carlsbad, CA, USA). The amplification cycle was: 10 min of denaturation at 96°C, followed by 35 cycles of denaturation at 94°C for 1 min, annealing at 59°C for 1 min, and elongation at 72°C for 2 min, and a final extension of 72°C for 10 min.

PCR products were analyzed by Single Strand Conformation Polymorphisms (SSCP). Those fragments that showed abnormal SSCP patterns were sequenced using an automated CEQ 2000XL DNA Analysis System (Beckman Coulter, Palo Alto, CA, USA). The presence of the identified mutations was subsequently determined by restriction analysis and by the methodological procedure and device described previously "biochip".

### 108delC mutation analysis

This mutation creates a new MnII digestion site. Fifteen µL of PCR sample were digested with 15 units of MnII in a total volume of 30 µL according to the protocol described by the manufacturer (Fermentas Inc., Hanover, MD, USA). The fragments obtained had a length of 150 and 33 bp in normal alleles and 118, 33 and 32 bp in mutant alleles. These fragments were electrophoresed in 8% polyacrilamide gel and were visualised by staining with ethidium bromide. Alternatively, this mutation could be analyzed with the device ("biochip") by spotting onto the slide the oligonucleotides: SEQ ID NO: 40, SEQ ID NO: 41, SEQ ID NO: 42 and SEQ ID NO: 43.

108delC mutation was detected in a 50 years old woman, without any clinical skin manifestation of her hypercholesterolemia. She was diagnosed clinically as having FH MedçPed score of 9 points. Premature cardiovascular disease was detected in one first degree relative. Fasting plasma lipid levels while off hypolipidemic drug therapy were: TC (381 mg/dL), TG (142 mg/dL), LDLc (321) mg/dL) and HDLc (32 mg/dL).

### T41M mutation analysis

This mutation (185C>T, ACG>ATG, Thr41 Met) destroys a cleavage restriction site for the enzyme Tail. Fifteen µL of PCR sample were digested with 15 units of Tail in a total volume of 30 µL according to the protocol described by the manufacturer (NEB, Beverly, MA, USA). The fragments obtained had a length of 154 and 29 bp for normal alleles and 183 bp for mutant alleles. These fragments were electrophoresed in 8% polyacrilamide (PAA) gel and were visualised by staining with ethidium bromide. Alternatively, this mutation could be analyzed with the device ("biochip") by spotting onto the slide the oligonucleotides: SEQ ID NO: 140, SEQ ID NO: 141, SEQ ID NO: 142 and SEQ ID NO: 143.

T41M mutation was detected in a 69 years old man who suffered a myocardial infartion at the age of 55 years and that has been diagnosed as having familial hypercholesterolemia with a diagnostic score of 6 points (Familial hypercholesterolemia. Report of a second WHO consultation. The International MedPed FH Organization, Geneva 1998). Evidence of premature cardiovascular event was detected in relatives. Analysis of his fasting serum without the use of lipid lowering drugs was: TC (274 mg/dL) and LDL-c (217 mg/dL) with normal TG and HDLc levels.

### C42Y mutation analysis

This mutation (C42Y (188G>A, TGC>TAG, Cys42Tyr) was identified by DNA sequencing of the 183 bp fragment from exon 2 during screening for mutations in the LDL-r gene in subjects clinically diagnosed as FH. Purified PCR product from DNA sample were directly sequenced in both directions using the amplification primers Ex2F (SEQ ID NO:4) and Ex2R (SEQ ID NO:5) and the kit CEQ 2000 Dye Terminator Cycle Sequencing with Quick Start (Beckman Coulter, Palo Alto, CA, USA) according to the protocol described by the manufacturer. Sequences were detected using the CEQ 8000 Genetic Analysis System (Beckman Coulter, Inc. Fullerton), and analyzed with CEQ 8000 software. The G>A change was confirmed by sequencing a second independent PCR product. Alternatively, this mutation could be analyzed with the microarray ("biochip") by spotting onto the slide the oligonucleotides: SEQ ID NO: 248, SEQ ID NO: 249, SEQ ID NO: 250 and SEQ ID NO: 251.

C42Y mutation was detected in a 17 years old man with arcus lipoides corneae that has been diagnosed as having familial hypercholesterolemia with a diagnostic score of 10 points (Familial Hypercholesterolemia. Report of a second WHO consultation. The Intemational MedPed FH Organization, Geneva 1998). His mother had severe hypercholesterolemia. Analysis of his fasting serum without the use of lipid lowering drugs was: TC (350 mg/dL) with normal TG and HDLc levels. Treatment with simvastatin (20mg/day) lowered his TC and LDL-c levels to 274 and 214 mg/dL respectively.

### C74Y mutation analysis

This mutation C74Y (284 G>A, TGC>TAC, Cys74Tyr) was identified by DNA sequencing of the 196 bp fragment from exon 3 during screening for mutations in the LDL-r gene in subjects clinically diagnosed as FH. Purified PCR product from DNA sample were directly sequenced in both directions using the amplification primers Ex3F (SEQ ID NO:6) and Ex3R (SEQ ID NO:7) and the kit CEQ 2000 Dye Terminator Cycle Sequencing with Quick Start (Beckman Coulter, Palo Alto, CA, USA) according to the protocol described by the manufacturer. Sequences were detected using the CEQ 8000 Genetic Analysis System (Beckman Coulter, Inc. Fullerton), and analyzed with CEQ 8000 software. The G>A change was confirmed by sequencing a second independent PCR product. Alternatively, this mutation could be analyzed with the microarray ("biochip") by spotting onto the slide the oligonucleotides: SEQ ID NO: 212, SEQ ID NO: 213, SEQ ID NO: 214 and SEQ ID NO: 215.

C74Y mutation was detected in a 52 years old man with arcus cornealis, tendon xanthomas and family history of hypercholesterolemia. He has been diagnosed as having familial hypercholesterolemia with a diagnostic score of 17 points (Familial Hypercholesterolemia. Report of a second WHO consultation. The International MedPed FH Organization, Geneva 1998). Analysis of her fasting serum before the use of lipid lowering drugs was: TC (420 mg/dL) TG (96mg/dL) and HDLc (69mg/dL). Treatment with an HMGCoA reductase inhibitor (10mg/day) lowered his LDL-c levels by 22%.

### EXAMPLE 3: Identification of mutations located in exon 3 of the LDLr gene.

A 196 bp fragment of exon 3 of the LDLr gene was amplified by polymerase chain reaction (PCR) using the following primers: Ex3F (SEQ ID NO: 6) and Ex3R (SEQ ID NO: 7).

DNA (500ng) was amplified in a 50 µL reaction mixture containing 20mM Tris-HCl, pH 8.4, 50 mM KCI, 1.5 mM MgCL₂, 200 µM each dNTP, 0.2 µM each primer and 1.5 units of Taq DNA polymerase (Gibco BRL, Carlsbad, CA, USA). The amplification cycle was: 10 min of denaturation at 96°C, followed by 35 cycles of denaturation at 94°C for 1 min, annealing at 59°C for 1 min, and elongation at 72°C for 1 min, and a final extension of 72°C for 10 min.

PCR products were analyzed by Single Strand Conformation Polymorphisms (SSCP). Those fragments that showed abnormal SSCP patterns were sequenced using an automated CEQ 2000XL DNA Analysis System (Beckman Coulter, Palo Alto, CA, USA). The presence of the identified mutations was subsequently determined by restriction analysis and by the device described previously "biochip.

### 191-2delAinsCT mutation analysis

As this mutation does not change the restriction map, we designed a pair of mutagenic primers to introduce the recognition site of BfaI in presence of the normal allele but not in presence of mutant allele.

A 184 bp fragment of exon 3 of the LDLr gene was amplified by polymerase chain reaction (PCR) using the following primers: Ex3R (SEQ ID NO: 7) and Mut191-2F (SEQ ID NO: 8).

DNA (500ng) was amplified in a 50 µL reaction mixture containing 20mM Tris-HCl, pH 8.4, 50 mM KCI, 1.5 mM MgCL₂, 200 µM each dNTP, 0.2 µM each primer and 1.5 units of Taq DNA polymerase (Gibco BRL, Carlsbad, CA, USA). The amplification cycle was: 10 min of denaturation at 96°C, followed by 35 cycles of denaturation at 94°C for 1 min, annealing at 59°C for 1 min, and elongation at 72°C for 2 min, and a final extension of 72°C for 10 min.

Fifteen µL of PCR sample were digested with 15 units of BfaI in a total volume of 30 µL according to the protocol described by the manufacturer (NEB, Beverly, MA, USA). The fragments obtained had a length of 23 and 161 bp for normal alleles and 185 bp for mutant alleles. These fragments were electrophoresed in 8% polyacrilamide gel and were visualized by staining with ethidium bromide. Alternatively, this mutation could be analyzed with the microarray ("biochip") by spotting onto the slide the oligonucleotides: SEQ ID NO: 44, SEQ ID NO: 45, SEQ ID NO: 46 and SEQ ID NO: 47.

191-2delAinsCT mutation was detected in two unrelated families with autosomal dominant hypercholesterolemia. The index case of one of these families was a 58 years old woman, with tendon xanthomas, xanthelasmas, angina pectoris, family history of coronary heart disease and hypercholesterolemia. She has been diagnosed as having familial hypercholesterolemia with a MedPed diagnostic score of 15 points. Her plasma lipid levels were: TC (559 mg/dL) and LDLc (467 mg/dL), TG (175 mg/dL) and HDLc (57 mg/dL). Treatment with simvastatin (40mg/day) lowered her TC and LDL-c levels to 302 and 228 mg/dL respectively.

### N59K mutation analysis

This mutation (240C>A, AAC>AAA, Asn59Lys) destroys a cleavage endonuclease site for the enzyme HincII. Fifteen µL of PCR sample were digested with 15 units of HincII in a total volume of 30 µL according to the protocol described by the manufacturer (Amersham Pharmacia Biotech Inc., Piscataway, NJ, USA). The fragments obtained had a length of 111 and 85 bp (normal alleles) or 196 bp (mutant alleles). These fragments were electrophoresed in 8% polyacrilamide (PAA) gel and were visualized by staining with ethidium bromide. Alternatively, this mutation could be analyzed with the microarray ("biochip") by spotting onto the slide the oligonucleotides: SEQ ID NO: 48, SEQ ID NO: 49, SEQ ID NO: 50 and SEQ ID NO: 51.

N59K mutation was detected in a 43 years old man diagnosed clinically as having FH with a MedPed diagnostic score of 12 points. His plasma lipid levels without lipid lowering therapy were TC (465 mg/dL), LDLc (397 mg/dL), TG (100 mg/dL) and HDLc (48 mg/dL). Treatment with simvastatin (40mg/dy) lowered his TC and LDL-c levels to 350 and 282 mg/dL respectively. On the other hand, his mother had suffered an angine pectoris at the age of 58 and he has a son of 8 years old with hypercholesterolemia TC (325 mg/dL) and LDLc (241 mg/dL).

### 231delC mutation analysis

The 231delC mutation destroys a endonuclease HaeIII digestion site. Fifteen µL of PCR sample were digested with 15 units of HaeIII in a total volume of 30 µL according to the protocol described by the manufacturer (Gibco BRL, Carlsbad, CA, USA). The fragments obtained had a length of 76, 51, 42 and 25 bp for normal alleles and 117,51, and 27 bp for mutant alleles. These fragments were electrophoresed in 8% polyacrilamide (PAA) gel and were visualized by staining with ethidium bromide. Alternatively, this mutation could be analyzed with the microarray ("biochip") by spotting onto the slide the oligonucleotides: SEQ ID NO: 52, SEQ ID NO: 53, SEQ ID NO: 54 and SEQ ID NO: 55.

231delC mutation was detected in a 37 years old woman, with arcus corneae. Her brother suffered a myocardial infarction at the age of 38 and her son of 12 years old have hypercholesterolemia with TC concentration of 305 mg/dL. She was diagnosed clinically as having FH with a MedPed score of 16 points Plasma lipid levels without lipid lowering therapy were: TC (543 mg/dL), LDLc (456 mg/dL), TG (178 mg/dL) and HDL-c (51 mg/dL). Treatment with atorvastatin (40mg/day) and colestipol (20g/day) lowered her TC and LDL-c levels to 260 and 190 mg/dL respectively.

### 313+1insT mutation analysis

This mutation creates a new cleavage site for the restriction endonuclease Tru1I. Fifteen µL of PCR sample were digested with 15 units of Tru1I in a total volume of 30 µL according to the protocol described by the manufacturer (Fermentas Inc., Hanover, MD, USA). The fragments obtained had a length of 196 bp for normal alleles and 162 and 34 bp for mutant alleles. These fragments were electrophoresed in 3% NuSieve agarose gel and were visualized by staining with ethidium bromide. Alternatively, this mutation could be analyzed with the microarray ("biochip") by spotting onto the slide the oligonucleotides: SEQ ID NO: 56, SEQ ID NO: 57, SEQ ID NO: 58 and SEQ ID NO: 59.

313+1insT mutation was detected in a 53 years old woman, with xanthomas and arcus corneae. No premature cardiovascular events were detected in her available family members. She was diagnosed clinically as having FH with a MedPed score of 19 points. Analysis of her fasting serum lipid levels without the use of lipid lowering drugs were: TC (574 mg/dL) and LDLc (505 mg/dL) with normal TG and HDLc levels. After lipid lowering treatment with simvastatin (80 mg/day) and colestipol (20 g/day) their TC and LDL-c levels decreased at 282 mg/dL and 225 mg/dL respectively.

### EXAMPLE 4: Identification of mutations located in exon 4 of the LDLr gene.

A 242 bp fragment of LDL-r gene from the 5' region of exon 4 (exon 4A) was amplified by polymerase chain reaction (PCR) using the following primers: Ex 4AF (SEQ ID NO: 9) and Ex 4AR (SEQ ID NO: 10).

DNA (500ng) was amplified in a 50 µL reaction mixture containing 20mM Tris-HCl, pH 8.4, 50 mM KCI, 1.5 mM MgCL₂, 200 µM each dNTP, 0.2 µM each primer and 1.5 units of Taq DNA polymerase (Gibco BRL, Carlsbad, CA, USA). The amplification cycle was: 10 min of denaturation at 96°C, followed by 35 cycles of denaturation at 94°C for 1 min, annealing at 63°C for 1 min, and elongation at 72°C for 2 min, and a final extension of 72°C for 10 min.

PCR products were analyzed by Single Strand Conformation Polymorphisms (SSCP). Those fragments that showed abnormal SSCP patterns were sequenced using an automated CEQ 2000XL DNA Analysis System (Beckman Coulter, Palo Alto, CA, USA). The presence of the identified mutations was subsequently determined by restriction analysis and by the device described previously "biochip".

### 338del16 mutation analysis

This mutation creates a new cleavage site for the restriction endonuclease Van91I. Fifteen µL of PCR sample were digested with 15 units of Van91I in a total volume of 30 µL according to the protocol described by the manufacturer (Amersham Pharmacia Biotech Inc., Piscataway, NJ, USA). The fragments obtained had a length of 242 bp for normal alleles and 174 and 52 bp for mutant alleles. These fragments were electrophoresed in 2% agarose gel and were visualized by staining with ethidium bromide. Alternatively, this mutation could be analyzed with the microarray ("biochip") by spotting onto the slide the oligonucleotides SEQ ID NO: 144, SEQ ID NO: 145, SEQ ID NO: 146 and SEQ ID NO: 147.

338del16 mutation was detected in three unrelated families with autosomal dominant hypercholesterolemia. One index case of these families was a 40 years old man, with TC 542 mg/dL and LDLc 441 mg/dL and normal TG and HDLc levels. He presented xanthomas and arcus corneae. He was diagnosed as having FH with a MedPed score of 19 points. No cardiovascular event was detected in his available family members. Treatment with atorvastatin (10mg/day) lowered his plasma TC and LDL-c levels to 293 and 218 mg/dL respectively.

### 510insC mutation analysis

As this mutation does not change the restriction map, we designed a pair of mutagenic primers to introduce one recognition site for the restriction enzyme MnII in presence of the mutant allele but not in presence of normal allele.

A 244 bp fragment of exon 4A of the LDLr gene was amplified by polymerase chain reaction (PCR) using the following primers: Ex4AF (SEQ ID NO: 9) and Mut509insCR (SEQ ID NO: 11).

DNA (500ng) was amplified in a 50 µL reaction mixture containing 20mM Tris-HCl, pH 8.4, 50 mM KCI, 1.5 mM MgCL₂, 200 µM each dNTP, 0.2 µM each primer and 1.5 units of Taq DNA polymerase (Gibco BRL, Carlsbad, CA, USA). The amplification cycle was: 10 min of denaturation at 96°C, followed by 35 cycles of denaturation at 94°C for 1 min, annealing at 65°C for 1 min, and elongation at 72°C for 1 min, and a final extension of 72°C for 10 min.

Fifteen µL of PCR sample were digested with 15 units of MnII in a total volume of 30 µL according to the protocol described by the manufacturer (Fermentas Inc., Hanover, MD, USA). The fragments obtained had a length of 141, 99 and 4 bp for normal alleles for 141, 88, 12 and 4 bp in mutant alleles. These fragments were electrophoresed in 8% polyacrilamide gel and were visualized by staining with ethidium bromide. Alternatively, this mutation could be analyzed by the microarray ("biochip") using into the slide the oligonucleotides SEQ ID NO: 60, SEQ ID NO: 61, SEQ ID NO: 62 and SEQ ID NO: 63.

510insC mutation was detected in a 44 years old woman. Analysis of her fasting serum lipids without the use of lipid lowering drugs were: TC (477 mg/dL) and LDLc (394 mg/dL) with normal TG and HDLc levels. No personal and familial history of premature coronary heart disease was observed, neither xanthomas nor xanthelasmas. Their diagnostic clinical MedPed score was 9. She has two brothers with hypercholesterolemia.

### 451de13 mutation analysis

This mutation (451de13) was identified by DNA sequencing of the 242 bp fragment from exon 4 (4A) during screening for mutations in the LDL-r gene in subjects clinically diagnosed as having FH. Purified PCR product from DNA sample were directly sequenced in both directions using the amplification primers Ex4AF (SEQ ID NO:9) and Ex 4AR (SEQ ID NO:10) and the kit CEQ 2000 Dye Terminator Cycle Sequencing with Quick Start (Beckman Coulter, Palo Alto, CA, USA) according to the protocol described by the manufacturer. Sequences were detected using the CEQ 8000 Genetic Analysis System (Beckman Coulter, Inc. Fullerton), and analyzed with CEQ 8000 software. The three base pair deletion was confirmed by sequencing second PCR products. Alternatively, this mutation could be analyzed with the microarray ("biochip") by spotting onto the slide the oligonucleotides: SEQ ID NO: 172, SEQ ID NO: 173, SEQ ID NO: 174 and SEQ ID NO: 175.

451del3 mutation was detected in a 36 years old man with arcus lipoides corneae that has been previously suffered a myocardial infartion at age of 34. He has two children 2 and 8 years olds with TC of 320 and 275 mg/dl respectively He was diagnosed clinically as having FH with a score of 17 points. Analysis of his fasting serum lipids without the use of lipid lowering drugs were TC 449 mg/dl, LDL-c 367 mg/dL, TG 218 mg/dL and c-HDL-c 38 mg/dL. Treatment with simvastatin (40mg/day) lowered his LDL-c level to 270 mg/dL.

### EXAMPLE 5: Identification of mutations located in exon 4B of the LDLr gene.

A 237 bp fragment of 3' exon 4 (exon 4B) of the LDLr gene was amplified by polymerase chain reaction (PCR) using the following primers: Ex4BF (SEQ ID NO: 12) and Ex4BR (SEQ ID NO: 13).

DNA (200ng) was amplified in a 50 µL reaction mixture containing 20mM Tris-HCl, pH 8.4, 50 mM KCI, 1.5 mM MgCL₂, 200 µM each dNTP, 0.2 µM each primer and 1.5 units of Taq DNA polymerase (Gibco BRL, Carlsbad, CA, USA). The amplification cycle was: 10 min of denaturation at 96°C, followed by 35 cycles of denaturation at 94°C for 1 min, annealing and elongation at 72°C for 2 min, and a final extension of 72°C for 10 min.

PCR products were analyzed by Single Strand Conformation Polymorphisms (SSCP). Those fragments that showed abnormal SSCP patterns were sequenced using an automated CEQ 2000XL DNA Analysis System (Beckman Coulter, Palo Alto, CA, USA). The presence of the identified mutations was subsequently determined by restriction analysis and by the device described previously "biochip".

### D157G mutation analysis

This mutation (533A>G, GAT>GGT, Asp195Gly) creates a new digestion site for the endonuclease Hphl. Fifteen µL of PCR sample were digested with 15 units of Hphl in a total volume of 30 µL according to the protocol described by the manufacturer (NEB, Beverly, MA, USA). The fragments obtained had a length of 237 bp for normal alleles and 175 and 62 bp for mutant alleles. These fragments were electrophoresed in 3% NuSieve agarose gel and were visualized by staining with ethidium bromide. Alternatively, this mutation could be analyzed with the microarray ("biochip") by spotting onto the slide the oligonucleotides SEQ ID NO: 64, SEQ ID NO: 65, SEQ ID NO: 66 and SEQ ID NO: 67.

D157G mutation was detected in a 32 years old woman. No cardiovascular event was detected in her family. She was diagnosed clinically as having possible FH with a MedPed score of 6 points. Analysis of her fasting serum lipids before the use of lipid lowering therapy were: TC (358 mg/dL) and LDLc (296 mg/dL) with normal TG and HDLc levels. Treatment with atorvastatin (10mg/day) lowered her plasma TC and LDL-c levels to 212 and 140mg/dL respectively. Her father also had elevated levels of plasma cholesterol TC 364 mg/dL, as well as her grandmother 341 mg/dL.

### C195R mutation analysis

This mutation (646T>C, TGT>CGT, Cys195Arg) creates a BshNI digestion site. Fifteen µL of PCR sample were digested with 15 units of BshNI in a total volume of 30 µL according to the protocol described by the manufacturer (Fermentas Inc., Hanover, MD, USA). The fragments obtained had a length of 237 bp for normal alleles and 159 and 78 bp for mutant alleles. These fragments were electrophoresed in 8% polyacrilamide (PAA) gel and were visualized by staining with ethidium bromide. Alternatively, this mutation could be analyzed by the microarray ("biochip") using into the slide the oligonucleotides SEQ ID NO: 68, SEQ ID NO: 69, SEQ ID NO: 70 and SEQ ID NO: 71.

C195R mutation was detected in a 64 years old woman who has hypercholesterolemia and arcus corneae. Premature cardiovascular disease was detected in her mother. She was diagnosed clinically as having FH with a MedPed score of 11 points Plasma lipid levels without lipid lowering therapy were: TC (560 mg/dL) and LDLc (468 mg/dL) with normal TG and HDLc levels.

### 675del15 mutation analysis

This mutation was identified by heteroduplexes analysis; the electrophoresis in 8% polyacrilamide (PAA) gel of exon 4B PCR products visualized by staining with ethidium bromide, showed the presence of heteroduplexes bands instead of the corresponding normal and mutated homoduplexes. The fragments obtained had a length of 237 bp in normal alleles and 222 bp in mutant alleles. The heteroduplex band migrated more slowly because of the formation of the bubble between the michtmached sequences. Alternatively, this mutation could be analyzed with the microarray ("biochip") by spotting onto the slide the oligonucleotides SEQ ID NO: 72, SEQ ID NO: 73, SEQ ID NO: 74 and SEQ ID NO: 75.

675del15 mutation was detected in a 63 years old woman, clinically diagnosed as having FH with a MedPed score of 8 points. No cardiovascular event was detected in her family. An untreatment lipid determination gave us the following results: TC (450 mg/dL) and LDLc (379 mg/dL) with normal TG and HDLc levels. No family members were available to complete the genetic study.

### 684dup12 mutation analysis

This mutation was identified by PCR amplification of exon 4B and restriction analysis, the addition of 12 bp creates a new MnII endonuclease restriction site. Fifteen µL of PCR sample were digested with 15 units of MnII in a total volume of 30 µL according to the protocol described by the manufacturer (Fermentas, Inc., Hanover, MD, USA). The fragments obtained had a length of 192 and 45 bp for normal alleles and 204 and 45 bp for mutant alleles. These fragments were electrophoresed in 8% polyacrilamide gel and were visualized by staining with ethidium bromide. Alternatively, this mutation could be analyzed with the microarray ("biochip") by spotting onto the slide the oligonucleotides SEQ ID NO: 76, SEQ ID NO: 77, SEQ ID NO: 78 and SEQ ID NO: 79.

684dup12 mutation was detected in two unrelated families having autosomal dominant hyperclolesterolemia. The index case of one of these families was a 63 years old man, with xanthomas and arcus corneae He has been suffered a myocardial infarction at the age of 55 and was diagnosed clinically as having FH with a MedPed score of 17 points. No cardiovascular event was detected in his family. Plasma lipid levels without lipid lowering therapy were: TC (469 mg/dL), LDLc (408 mg/dL),TG (100 mg/dL) and HDLc 41 mg/dL.

### D200V mutation analysis

This mutation (D200V (662A>T, GAC>GTC, Asp200Val)) was identified by DNA sequencing of the 237 bp fragment from exon 4 (4B) during screening for mutations in the LDL-r gene in subjects clinically diagnosed as having FH. Purified PCR product from DNA sample were directly sequenced in both directions using the amplification primers Ex4BF (SEQ ID NO:12) and Ex 4BR (SEQ ID NO:13) and the kit CEQ 2000 Dye Terminator Cycle Sequencing with Quick Start (Beckman Coulter, Palo Alto, CA, USA) according to the protocol described by the manufacturer. Sequences were detected using the CEQ 8000 Genetic Analysis System (Beckman Coulter, Inc. Fullerton), and analyzed with CEQ 8000 software. The 662A>T change was confirmed by sequencing a second PCR product. Alternatively, this mutation could be analyzed with the microarray ("biochip") by spotting onto the slide the oligonucleotides: SEQ ID NO: 232, SEQ ID NO: 233, SEQ ID NO: 234 and SEQ ID NO: 235.

D200V mutation was detected in a 43 years old woman with family history of autosomal dominant hypercholesterolemia. She was diagnosed clinically as having familial hypercholesterolemia with a score of 8 points (Familial Hypercholesterolemia. Report of a second WHO consultation. The International MedPed FH Organization, Geneva 1998). Analysis of her fasting serum lipids using lipid lowering drug pravastatin (40 mg/day) were TC 329 mg/dl, LDL-c 273 mg/dL, TG 73 mg/dL and HDL-c 41 mg/dL.

### S205Cmutation analysis

This mutation S205C (677C>G, TCT>TGT, Ser205Cys) was identified by DNA sequencing of the 237 bp fragment from exon 4 (4B) during screening for mutations in the LDL-r gene in subjects clinically diagnosed as having FH. Purified PCR product from DNA sample were directly sequenced in both directions using the amplification primers Ex4BF (SEQ ID NO:12) and Ex 4BR (SEQ ID NO:13) and the kit CEQ 2000 Dye Terminator Cycle Sequencing with Quick Start (Beckman Coulter, Palo Alto, CA, USA) according to the protocol described by the manufacturer. Sequences were detected using the CEQ 8000 Genetic Analysis System (Beckman Coulter, Inc. Fullerton), and analyzed with CEQ 8000 software. The 677C>G change was confirmed by sequencing a second PCR products. Alternatively, this mutation could be analyzed with the microarray ("biochip") by spotting onto the slide the oligonucleotides: SEQ ID NO: 228, SEQ ID NO: 229, SEQ ID NO: 230 and SEQ ID NO: 231.

S205C mutation was detected in a 39 years old woman with family history of autosomal dominant hypercholesterolemia (mother and brother TC 450 mg/dL and 500 mg/dL respectively). She was diagnosed clinically at 20 years old as having familial hypercholesterolemia with a MedPed score of 8 points Analysis of his fasting serum lipids without using lipid lowering drugs were TC 390 mg/dl, LDL-c 325 mg/dL and HDL-c 35 mg/dL. Treatment with simvastatin (10mg/day) lowered her plasma LDL-c level to 270 mg/dL.

### EXAMPLE 6: Identification of mutations located in exon 6 of the LDLr gene.

A 179 bp fragment of exon 6 of the LDLr gene was amplified by polymerase chain reaction (PCR) using the following primers: Ex6F (SEQ ID NO: 14) and Ex6R (SEQ ID NO: 15).

DNA (500ng) was amplified in a 50 µL reaction mixture containing 20mM Tris-HCl, pH 8.4, 50 mM KCI, 1.5 mM MgCL₂, 200 µM each dNTP, 0.2 µM each primer and 1.5 units of Taq DNA polymerase (Gibco BRL, Carlsbad, CA, USA). The amplification cycle was: 10 min of denaturation at 96°C, followed by 35 cycles of denaturation at 94°C for 1 min, annealing at 56°C for 1 min, and elongation at 72°C for 1 min, and a final extension of 72°C for 10 min.

PCR products were analyzed by Single Strand Conformation Polymorphisms (SSCP). Those fragments that showed abnormal SSCP patterns were sequenced using an automated CEQ 2000XL DNA Analysis System (Beckman Coulter, Palo Alto, CA, USA). The presence of the identified mutations was subsequently determined by restriction analysis and by the device described previously "biochip".

### C255G mutation analysis

As this mutation C255G (826T>G, TGC>GGC, Cys255Gly) does not change the restriction map, we designed a pair of mutagenic primers to introduce the recognition site of BstUI restriction enzyme in presence of the mutant allele but not in presence of normal allele.

A 163 bp fragment of exon 6 of the LDLr gene was amplified by polymerase chain reaction (PCR) using the following primers: Ex6R (SEQ ID NO: 15) and MutC255GF (SEQ ID NO: 16).

DNA (500ng) was amplified in a 50 µL reaction mixture containing 20mM Tris-HCl, pH 8.4, 50 mM KCI, 1.5 mM MgCL₂, 200 µM each dNTP, 0.2 µM each primer and 1.5 units of Taq DNA polymerase (Gibco BRL, Carlsbad, CA, USA). The amplification cycle was: 10 min of denaturation at 96°C, followed by 35 cycles of denaturation at 94°C for 1 min, annealing at 63°C for 1 min, and elongation at 72°C for 2 min, and a final extension of 72°C for 10 min.

Fifteen µL of PCR sample were digested with 15 units of BstUI in a total volume of 30 µL according to the protocol described by the manufacturer (NEB, Beverly, MA, USA). The fragments obtained had a length of 163 bp for normal alleles and 141 and 22 bp for mutant alleles. These fragments were electrophoresed in 8% polyacrilamide gel and were visualized by staining with ethidium bromide. Alternatively, this mutation could be analyzed wit the microarray ("biochip") by spotting onto the slide the oligonucleotides SEQ ID NO: 80, SEQ ID NO: 81, SEQ ID NO: 82 and SEQ ID NO: 83.

C255G mutation was detected in a 63 years old woman, with family history of hypercholesterolemia. No cardiovascular disease was detected in her family. An untreatment lipid determination gave us the following results: TC (439 mg/dL) and LDLc (355 mg/dL) with normal TG and HDLc levels. She was diagnosed clinically as having familial hypercholesterolemia with a MedPed score of 8 points

### E291X mutation analysis

As this mutation E291X (934G>T, GAG>TAG, Asp291 Stop) does not change the restriction map, we designed a pair of mutagenic primers to introduce the recognition site of SspI enzyme in presence of the mutant allele but not in presence of normal allele.

A 164 bp fragment of exon 6 of the LDLr gene was amplified by polymerase chain reaction (PCR) using the following primers: Ex6F (SEQ ID NO: 13) and Mut E291XR (SEQ ID NO: 17).

DNA (500ng) was amplified in a 50 µL reaction mixture containing 20mM Tris-HCl, pH 8.4, 50 mM KCI, 1.5 mM MgCL₂, 200 µM each dNTP, 0.2 µM each primer and 1.5 units of Taq DNA polymerase (Gibco BRL, Carlsbad, CA, USA). The amplification cycle was: 10 min of denaturation at 96°C, followed by 35 cycles of denaturation at 94°C for 1 min, annealing at 59°C for 1 min, and elongation at 72°C for 1 min, and a final extension of 72°C for 10 min.

Fifteen µL of PCR sample were digested with 15 units of SspI in a total volume of 30 µL according to the protocol described by the manufacturer (Amersham Pharmacia Biotech Inc., Piscataway, NJ, USA). The fragments obtained had a length of 164 bp for normal alleles and 144 and 20 bp for mutant alleles. These fragments were electrophoresed in 3% NuSieve agarose gel and were visualized by staining with ethidium bromide. Alternatively, this mutation could be analyzed with the microarray ("biochip") by spotting onto the slide the oligonucleotides SEQ ID NO: 84, SEQ ID NO: 85, SEQ ID NO: 86 and SEQ ID NO: 87.

E291X mutation was detected a 44 years old man from an autosomal dominant hypercholesterolemia family. He has arcus lipoides corneae and no cardiovascular disease was observed in his family. Analysis of his fasting serum lipids without using lipid lowering drugs were: TC (381 mg/dL), HDLc (45 mg/dL), TG (111 mg/dL) and LDLc (314 mg/dL). Combined lipid lowering treatment with simvastatin (40mg/day) and colestiramin (12g/day) lowered her plasma TC and LDL-c levels to 253 mg/dL and 188 mg/dL respectively.

### 818de18 mutation analysis

This mutation was identified by heteroduplexes analysis; the electrophoresis in 8% polyacrilamide (PAA) gel of amplified product of exon 6 visualized by staining with ethidium bromide, showed the presence of heteroduplexes bands instead of the corresponding normal and mutated homoduplexes of 179 and 171 bp respectively. The heteroduplex band migrated more slowly because of the formation of the bubble between the michtmached sequences. In addition the mutation could be confirmed by PCR amplification of exon 6 and restriction analysis, the 818del8 mutation creates a new Maelll endonuclease restriction site. Fifteen µL of PCR sample were digested with 15 units of MaeIII in a total volume of 30 µL according to the protocol described by the manufacturer (Roche Diagnostics, Manheim, Germany). The fragments obtained had a length of 118, 34 and 27 bp for normal alleles and 118 and 53 bp for mutant alleles. These fragments were electrophoresed in 8% of polyacrilamide and were visualized by staining with ethidium bromide. Alternatively, this mutation could be analyzed with the microarray ("biochip") by spotting onto the slide the oligonucleotides SEQ ID NO: 160, SEQ ID NO: 161, SEQ ID NO: 162 and SEQ ID NO: 163.

818del8 mutation was detected in a 69 years old woman, clinically diagnosed as having FH with a MedPed score of 10 points. Her two sons have hipercholesterolemia with plasma TC levels of 382 and 304 mg/dL respectively. Analysis of her fasting serum lipids before using lipid lowering drugs were: TC (530 mg/dL) and LDLc (439 mg/dL) TG( 170 mg/dL and HDLc 57 mg/dL Lipid lowering treatment with cerivastatin (0.4mg/day) reduced her LDL-c to 363 mg/dL.

### R279G mutation analysis

This mutation R279G (898A>G, AGA>GGA, Arg279Gly) was identified by DNA sequencing of the 179 bp fragment from exon 6 during screening for mutations in the LDL-r gene in subjects clinically diagnosed as FH. Purified PCR product from DNA sample were directly sequenced in both directions using the amplification primers Ex6F (SEQ ID NO:14) and Ex6R (SEQ ID NO:15) and the kit CEQ 2000 Dye Terminator Cycle Sequencing with Quick Start (Beckman Coulter, Palo Alto, CA, USA) according to the protocol described by the manufacturer. Sequences were detected using the CEQ 8000 Genetic Analysis System (Beckman Coulter, Inc. Fullerton), and analyzed with CEQ 8000 software. The 898A>G change was confirmed by sequencing a second PCR product. Alternatively, this mutation could be analyzed with the microarray ("biochip") by spotting onto the slide the oligonucleotides: SEQ ID NO: 200, SEQ ID NO: 201, SEQ ID NO: 202 and SEQ ID NO: 203.

R279G mutation was identified in a 59 years old woman with xantelasmas and family history of hypercholesterolemia. Their score for FH clinical diagnostic was 10 points (Familial Hypercholesterolemia. Report of a Second WHO Consultation. The International MedPed FH Organization, Geneva 1998) Plasma lipid levels before treatment were: TC 384 mg/dL, LDL-c 314 mg/dL and normal TG and HDL-c levels. Lipid lowering treatment with simvastatin (80 mg/day) lowered her LDL-c to 167 mg/dL.

### EXAMPLE 7: Identification of mutations located in exon 7 of the LDLr gene.

A 234 bp fragment of exon 7 of the LDLr gene was amplified by polymerase chain reaction (PCR) using the following primers Ex7F (SEQ ID NO: 18) and Ex7R (SEQ ID NO: 19.

DNA (500ng) was amplified in a 50 µL reaction mixture containing 20mM Tris-HCl, pH 8.4, 50 mM KCI, 1.5 mM MgCL₂, 200 µM each dNTP, 0.2 µM each primer and 1.5 units of Taq DNA polymerase (Gibco BRL, Carlsbad, CA, USA). The amplification cycle was: 10 min of denaturation at 96°C, followed by 35 cycles of denaturation at 94°C for 1 min, annealing at 57°C for 1 min, and elongation at 72°C for 1 min, and a final extension of 72°C for 10 min.

PCR products were analyzed by Single Strand Conformation Polymorphisms (SSCP). Those fragments that showed abnormal SSCP patterns were sequenced using an automated CEQ 2000XL DNA Analysis System (Beckman Coulter, Palo Alto, CA, USA). The presence of the identified mutations was subsequently determined by restriction analysis and by the device described previously "biochip".

### 941-39C>T mutation analysis

This mutation destroys an Apal digestion site. Fifteen µL of exon 7 PCR sample were digested with 15 units of Apal in a total volume of 30 µL according to the protocol described by the manufacturer (Fermentas Inc., Hanover, MD, USA). The fragments obtained had a length of 186, 26 and 22 bp for normal alleles and 208 and 26 bp for mutant alleles. These fragments were electrophoresed in 8% polyacrilamide (PAA) gel and were visualised by staining with ethidium bromide. Alternatively, this mutation could be analyzed with the microarray ("biochip") by spotting onto the slide the oligonucleotides SEQ ID NO: 88, SEQ ID NO: 89, SEQ ID NO: 90 and SEQ ID NO: 91.

941-39C>T mutation was detected in four unrelated families. The index case of one of these families was 61 years old woman. Premature cardiovascular disease was present in the proband and also in a first degree relative. She was clinically diagnosed as having FH with a MedPed score of 7 points. Plasma lipid levels before treatment were: TC (340 mg/dL) and LDLc (248 mg/dL) with normal TG and HDLc levels. After lipid lowering treatment with atorvastatin (20 mg/day) TC and LDL-c levels decreased at 233 mg/dL and 144 mg/dL respectively without no significant changes in TG and HDL-c levels.

### C319Y mutation analysis

This mutation C319Y (1019G>A, TGC>TAC, Cys319Tyr) creates a new Rsal endonuclease digestion site. Fifteen µL of PCR sample were digested with 15 units of Rsal in a total volume of 30 µL according to the protocol described by the manufacturer (Gibco BRL, Carlsbad, CA, USA). The fragments obtained had a length of 234 bp in normal alleles and 136 and 98 bp in mutant alleles. These fragments were electrophoresed in 8% polyacrilamide (PAA) gel and were visualized by staining with ethidium bromide. Alternatively, this mutation could be analyzed with the microarray ("biochip") by spotting onto the slide the oligonucleotides SEQ ID NO: 92, SEQ ID NO: 93, SEQ ID NO: 94 and SEQ ID NO: 95.

C319Y mutation was detected in a 43 years old man, with arcus corneae and xanthomas at Achiles tendon and dorsum of the hands. His father suffered a sudden death at 45 years old. Premature cardiovascular disease was observed in one first degree relative. He was clinically diagnosed as having FH with a MedPed score of 22 points. Plasma lipid levels before treatment were: TC (428 mg/dL) and LDLc (372 mg/dL) with normal TG and HDLc levels.

### 1054del11 mutation analysis

This mutation destroys a endonuclease restriction site for the Hphl enzyme. Fifteen µL of PCR sample were digested with 15 units of Hphl in a total volume of 30 µL according to the protocol described by the manufacturer (NEB, Beverly, MA, USA). The fragments obtained had a length of 189 and 45 bp in normal alleles and 223 bp in mutant alleles. These fragments were electrophoresed in 8% polyacrilamide gel and were visualized by staining with ethidium bromide. Alternatively, this mutation could be analyzed with the microarray ("biochip") by spotting onto the slide the oligonucleotides SEQ ID NO: 96, SEQ ID NO: 97, SEQ ID NO: 98 and SEQ ID NO: 99.

1054del11 mutation was detected in a 43 years old man, with xanthomas at Achiles tendon and premature cardiovascular disease; on the other hand, he has a first degree relative that suffered a premature myocardial infartion. He was clinically diagnosed as having FH with a MedPed score of 16 points. Plasma lipid levels before treatment were: TC (480 mg/dL), LDLc (416 mg/dL), TG (95 mg/dL and HDLc 36 mg/dL.

### EXAMPLE 8: Identification of mutations located in exon 8 of the LDLr gene.

A 220 bp fragment of exon 8 of the LDLr gene was amplified by polymerase chain reaction (PCR) using the following primers: Ex8F (SEQ ID NO: 148) and Ex8R (SEQ ID NO: 149).

DNA (500ng) was amplified in a 50 µL reaction mixture containing 20mM Tris-HCl, pH 8.4, 50 mM KCI, 1.5 mM MgCL₂, 200 µM each dNTP, 0.2 µM each primer and 1.5 units of Taq DNA polymerase (Gibco BRL, Carlsbad, CA, USA). The amplification cycle was: 10 min of denaturation at 96°C, followed by 35 cycles of denaturation at 94°C for 1 min, annealing at 64°C for 1 min, and elongation at 72°C for 2 min, and a final extension of 72°C for 10 min.

PCR products were analyzed by Single Strand Conformation Polymorphisms (SSCP). Those fragments that showed abnormal SSCP patterns were sequenced using an automated CEQ 2000XL DNA Analysis System (Beckman Coulter, Palo Alto, CA, USA). The presence of the identified mutations was subsequently determined by restriction analysis and by the device described previously "biochip".

### 1186+5 G>A mutation analysis

This mutation (1186+5 G>A) was identified by DNA sequencing of the 220 bp fragment from exon 8 during screening for mutations in the LDL-r gene in subjects clinically diagnosed as FH. Purified PCR product from DNA sample were directly sequenced in both directions using the amplification primers Ex8BF (SEQ ID NO:148) and Ex8BR (SEQ ID NO:149) and the kit CEQ 2000 Dye Terminator Cycle Sequencing with Quick Start (Beckman Coulter, Palo Alto, CA, USA) according to the protocol described by the manufacturer. Sequences were detected using the CEQ 8000 Genetic Analysis System (Beckman Coulter, Inc. Fullerton), and analyzed with CEQ 8000 software. The G>A change was confirmed by sequencing a second PCR product. Alternatively, this mutation could be analyzed with the microarray ("biochip") by spotting onto the slide the oligonucleotides: SEQ ID NO: 188, SEQ ID NO: 189, SEQ ID NO: 190 and SEQ ID NO: 191.

1186+5 G>A mutation was identified in two unrelated families with autosomal dominant hipecholesterolemia. The index case of one of these families was a 34 years old woman with xantelasmas, arcus corneae and tendon xanthomata. She was clinically diagnosed as having FH with a MedPed score of 21 points. Plasma lipid levels before treatment were: TC 411 mg/dL, LDL-c 346 mg/dL and normal TG and HDL-c levels. Lipid lowering treatment with cerivastatin (0.2 mg/day) reduced her LDL-c to 222 mg/dL.

### EXAMPLE 9: Identification of mutations located in exon 9 of the LDLr gene.

A 224 bp fragment of exon 9 of the LDLr gene was amplified by polymerase chain reaction (PCR) using the following primers: Ex9F (SEQ ID NO: 20) and Ex9R (SEQ ID NO: 21).

DNA (500ng) was amplified in a 50 µL reaction mixture containing 20mM Tris-HCl, pH 8.4, 50 mM KCI, 1.5 mM MgCL₂, 200 µM each dNTP, 0.2 µM each primer and 1.5 units of Taq DNA polymerase (Gibco BRL, Carlsbad, CA, USA). The amplification cycle was: 10 min of denaturation at 96°C, followed by 35 cycles of denaturation at 94°C for 1 min, annealing at 63°C for 1 min, and elongation at 72°C for 2 min, and a final extension of 72°C for 10 min.

PCR products were analyzed by Single Strand Conformation Polymorphisms (SSCP). Those fragments that showed abnormal SSCP patterns were sequenced using an automated CEQ 2000XL DNA Analysis System (Beckman Coulter, Palo Alto, CA, USA). The presence of the identified mutations was subsequently determined by restriction analysis and by the device described previously "biochip".

### 1197de19 mutation analysis

This mutation was analyzed by heteroduplex analysis; the electrophoresis in 8% polyacrilamide gel of PCR products visualized by staining with ethidium bromide, showed the presence of heteroduplexes bands instead of the corresponding normal and mutated homoduplexes. The fragments obtained had a length of 224 bp for normal alleles and 215 bp for mutant alleles. The heteroduplex band migrated more slowly because of the formation of the bubble between the michtmached sequences. Alternatively, this mutation could be analyzed with the microarray ("biochip") by spotting onto the slide the oligonucleotides SEQ ID NO: 100, SEQ ID NO: 101, SEQ ID NO: 102 and SEQ ID NO: 103.

1197del9 mutation was detected in eight unrelated families. Index case of one of these families was a 45 years old woman, with xanthomata who suffered an angine pectoris at the age of 41. Her father suffered a myocardial infarction at the age of 36. She was clinically diagnosed as having FH with a MedPed score of 18 points. Plasma lipid levels before treatment were: TC (525 mg/dL), LDLc (443 mg/dL), TG (163 mg/dL) and HDLc (49 mg/dL). Lipid lowering treatment with atorvastatin (20 mg/day) reduced her LDL-c to 323 mg/dL.

### Y379X mutation analysis

This mutation Y379X (1200C>A, TAC>TAA, Tyr379Stop) destroy a cleavage site for the restriction endonuclease MnII. Fifteen µL of PCR sample were digested with 15 units of MnII in a total volume of 30 µL according to the protocol described by the manufacturer (Gibco BRL, Carlbad,CA.USA). The fragments obtained had a length of 87, 56, 34, 22, 18, 4, and 3 bp for normal alleles and 87, 56, 38, 22, 18, and 3 bp for mutant alleles. These fragments were electrophoresed in 16% polyacrilamide (PAA) gel and were visualized by staining with ethidium bromide. Alternatively, this mutation could be analyzed with the microarray ("biochip") by spotting onto the slide the oligonucleotides SEQ ID NO: 104, SEQ ID NO: 105, SEQ ID NO: 106 and SEQ ID NO: 107.

Y379X mutation was detected in an 69 years old man with severe hipercholesterolemia. His father suffered a myocardial infartion at age 50. He was clinically diagnosed as having FH with a MedPed score of 7 points. An untreatment lipid determination gave us the following results: TC (381 mg/dL) and LDLc (306 mg/dL) with normal TG and HDLc levels. Lipid lowering treatment with atorvastatin (20 mg/day) reduced her LDL-c to 259 mg/dL

### 1207delT mutation analysis

This mutation destroys a cleavage site for the restriction enzyme Mboll. Fifteen µL of PCR sample were digested with 15 units of Mboll in a total volume of 30 µL according to the protocol described by the manufacturer (Amersham Pharmacia Biotech Inc., Piscataway, NJ, USA). The fragments obtained had a length of 140, 46, 35, and 3 bp for normal alleles and 140, 48, and35 bp for mutant alleles. These fragments were electrophoresed in 16% polyacrilamide gel and were visualized by staining with ethidium bromide. Alternatively, this mutation could be analyzed with the microarray ("biochip") by spotting onto the slide the oligonucleotides SEQ ID NO: 108, SEQ ID NO: 109, SEQ ID NO: 110 and SEQ ID NO: 111.

1207delT mutation was detected in a member of family with autosomal dominant hypercholesterolemia. She was a 35 years old woman, without family history of cardiovascular disease. Their MedPed score for FH clinical diagnostic was 9 points. The Plasma lipid levels before lipid lowering treatment were: TC (429 mg/dL), LDLc (345 mg/dL), TG (188 mg/dL) and HDLc (46 mg/dL). Combined lipid lowering treatment with simvastatin (40 mg/day) and colestipol (5g/day) reduced her TC and LDL-c to 220 mg/dL and 137 mg/dL respectively.

### Y421X mutation analysis

This mutation Y421X (1326C>G, TAC>TAG, Tyr421Stop) creates a new cleavage site for the endonuclease Bfal. Fifteen µL of PCR sample were digested with 15 units of Bfal in a total volume of 30 µL according to the protocol described by the manufacturer (NEB, Beverly, MA, USA). The fragments obtained had a length of 224 bp for normal alleles and 164 and 60 bp for mutant alleles. These fragments were electrophoresed in 8% polyacrilamide gel and were visualized by staining with ethidium bromide. Alternatively, this mutation could be analyzed with the microarray ("biochip") by spotting onto the slide the oligonucleotides SEQ ID NO: 112, SEQ ID NO: 113, SEQ ID NO: 114 and SEQ ID NO: 115.

Y421X mutation was detected in three unrelated familieswith hipercholesterolemia. Index case of one of these families was a 71 years old woman, with arcus corneae and tendon xanthomas. Her father had hypercholesterolemia and suffered a myocardial infarction at the age of 51. She was clinically diagnosed as having FH with a MedPed score of 16 points. Analysis of her fasting serum lipid levels without the use of lipid lowering drugs were: TC (615 mg/dL) and LDLc (550 mg/dL) with normal TG and HDLc levels.

### 1204insT mutation analysis

This mutation (1204insT) destroys a cleavage site for the endonuclease Mboll. Fifteen µL of exon 9 PCR sample were digested with 15 units of Mboll in a total volume of 30 µL according to the protocol described by the manufacturer (Amersham Pharmacia, NJ, USA). The fragments obtained had a length of 141, 45, 35 and 3 pb in normal alleles and 141, 45 and 39 pb in mutant alleles. These fragments were electrophoresed in 8% polyacrilamide gel and were visualized by staining with ethidium bromide. Alternatively, this mutation could be analyzed with the microarray ("biochip") by spotting onto the slide the oligonucleotides SEQ ID NO: 168, SEQ ID NO: 169, SEQ ID NO: 170 and SEQ ID NO: 171.

This mutation was detected in a girl of 12 years old. Her father and brother had hypercholesterolemia with TC levels of 412 and 321 mg/dL respectively. Their MedPed score for FH clinical diagnostic was 9 points. Analysis of her fasting serum lipid levels without the use of lipid lowering drugs were TC 332 mg/dL, LDL-c 267 mg/dL with normal TG and HDL-c levels. Lipid lowering treatment with colestipol (15 g/day) reduced the LDL-c levels to 248 mg/dL.

### EXAMPLE 10: Identification of mutations located in exon 10 of the LDLr gene.

A 278 bp fragment of exon 10 of the LDLr gene was amplified by polymerase chain reaction (PCR) using the following primers: Ex10F (SEQ ID NO: 22) and Ex10R (SEQ ID NO: 23).

DNA (500ng) was amplified in a 50 µL reaction mixture containing 20mM Tris-HCl, pH 8.4, 50 mM KCI, 1.5 mM MgCL₂, 200 µM each dNTP, 0.2 µM each primer and 1.5 units of Taq DNA polymerase (Gibco BRL, Carlsbad, CA, USA). The amplification cycle was: 10 min of denaturation at 96°C, followed by 35 cycles of denaturation at 94°C for 1 min, annealing at 58°C for 1 min, and elongation at 74°C for 2 min, and a final extension of 72°C for 10 min.

PCR products were analyzed by Single Strand Conformation Polymorphisms (SSCP). Those fragments that showed abnormal SSCP patterns were sequenced using an automated CEQ 2000XL DNA Analysis System (Beckman Coulter, Palo Alto, CA, USA). The presence of the identified mutations was subsequently determined by restriction analysis and by the device described previously "biochip".

### 1432delG mutation analysis

As this mutation does not change the restriction map, we designed a pair of mutagenic primers to introduce the recognition site of Nael in presence of the mutant allele but not in presence of normal allele.

A 200 bp fragment of exon 10 of the LDLr gene was amplified by polymerase chain reaction (PCR) using the following primers: Ex10R (SEQ ID NO: 23) and Mut1432delGF (SEQ ID NO: 24).

DNA (500ng) was amplified in a 50 µL reaction mixture containing 20mM Tris-HCl, pH 8.4, 50 mM KCI, 1.5 mM MgCL₂, 200 µM each dNTP, 0.2 µM each primer and 1.5 units of Taq DNA polymerase (Gibco BRL, Carlsbad, CA, USA). The amplification cycle was: 10 min of denaturation at 96°C, followed by 35 cycles of denaturation at 94°C for 1 min, annealing at 58°C for 1 min, and elongation at 72°C for 2 min, and a final extension of 72°C for 10 min.

Fifteen µL of PCR sample were digested with 15 units of Nael in a total volume of 30 µL according to the protocol described by the manufacturer (Amersham Pharmacia Biotech Inc., Piscataway, NJ, USA). The fragments obtained had a length of 200 bp (undigested fragment) for normal alleles and 179 and 20 bp in mutant alleles. These fragments were electrophoresed in 8% polyacrilamide gel and were visualized by staining with ethidium bromide. Alternatively, this mutation could be analyzed with the microarray ("biochip") by spotting onto the slide the oligonucleotides SEQ ID NO: 116, SEQ ID NO: 117, SEQ ID NO: 118and SEQ ID NO: 119.

1432delG mutation was detected in a 53 years old woman, with tendon xanthomas from an autosomal dominant hypercholesterolemia family. She suffered a premature cardiovascular disease as well as a first degree relative. She was clinically diagnosed as having FH with a MedPed score of 15 points. An lipid analysis without use lipid lowering therapy gave us the following results: TC (548 mg/dL) and LDLc (470 mg/dL) with normal TG and HDLc levels.

### T433N mutation analysis

This mutation T433N (1361 C>A, ACC>AAC, Tyr433Asn) was identified by DNA sequencing of the 278 bp fragment from exon 10 during screening for mutations in the LDL-r gene in subjects clinically diagnosed as having FH. Purified PCR product from DNA sample were directly sequenced in both directions using the amplification primers Ex10F (SEQ ID NO: 22) and Ex10R (SEQ ID NO: 23) and the kit CEQ 2000 Dye Terminator Cycle Sequencing with Quick Start (Beckman Coulter, Palo Alto, CA, USA) according to the protocol described by the manufacturer. Sequences were detected using the CEQ 8000 Genetic Analysis System (Beckman Coulter, Inc. Fullerton), and analyzed with CEQ 8000 software. The C>A change was confirmed by sequencing a second PCR product. Alternatively, this mutation could be analyzed with the microarray ("biochip") by spotting onto the slide the oligonucleotides: SEQ ID NO: 156, SEQ ID NO: 157, SEQ ID NO: 158 and SEQ ID NO: 159.

T433N mutation was detected in a 50 years old man with arcus corneae and family history of autosomal dominant hypercholesterolemia. His daughter of 21 years old has hypercholesterolemia with TC of 310 mg/dL. Their MedPed score for FH clinical diagnostic was 6 points. Analysis of his fasting serum lipids were TC 318mg/dl, LDL-c 249 mg/dL with normal TG and HDL-c. Lipid lowering therapy with lovastatin (20mg/day) reduced his LDL-c to 199 mg/dL.

### T446I mutation analysis

This mutation T446I (1400C>T, ACC>ATC, Tyr446Ile was identified by DNA sequencing of the 278 bp fragment from exon 10 during screening for mutations in the LDL-r gene in subjects clinically diagnosed as having FH. Purified PCR product from DNA sample were directly sequenced in both directions using the amplification primers Ex10F (SEQ ID NO: 22) and Ex10R (SEQ ID NO: 23) and the kit CEQ 2000 Dye Terminator Cycle Sequencing with Quick Start (Beckman Coulter, Palo Alto, CA, USA) according to the protocol described by the manufacturer. Sequences were detected using the CEQ 8000 Genetic Analysis System (Beckman Coulter, Inc. Fullerton), and analyzed with CEQ 8000 software. The C>T change was confirmed by sequencing a second PCR products. Alternatively, this mutation could be analyzed with the microarray ("biochip") by spotting onto the slide the oligonucleotides: SEQ ID NO: 204, SEQ ID NO: 205, SEQ ID NO: 206 and SEQ ID NO: 207.

T446I mutation was detected in a 64 years old woman who suffered an angor at 62 years old. She has two brothers that has been suffered a myocardial infartion at 40 and 46 years old respectively She was diagnosed clinically as having FH with a MedPed score of 9 points. Analysis of her fasting serum lipid levels during use of lipid lowering therapy with pravastatin were: TC (352 mg/dL) and LDLc (281 mg/dL) with normal TG and HDLc levels. After lipid lowering treatment with simvastatin 20 mg/day the LDL-c levels decreased to 150 mg/dL.

### 1423delGC/insA mutation analysis

This mutation 1423delGC/insA destroys a cleavage site for the endonuclease Mval. Fifteen µL of exon 10 PCR product were digested with 15 units of Mval in a total volume of 30 µL according to the protocol described by the manufacturer (Fermentas Inc., Henover, MD, USA). The fragments obtained had a length of 150 and 128 pb for normal alleles and 128, 87 and 63 pb for mutant alleles. These fragments were electrophoresed in 8% polyacrilamide gel and were visualized by staining with ethidium bromide. Alternatively, this mutation could be analyzed with the microarray ("biochip") by spotting onto the slide the oligonucleotides SEQ ID NO: 164, SEQ ID NO: 165, SEQ ID NO: 166 and SEQ ID NO: 167.

This mutation was detected in 34 years old man with family history of hypercholesterolemia. Their MedPed score for FH clinical diagnostic was 9 points. Analysis of her fasting serum lipid levels without the use of lipid lowering drugs were: TC 554 mg/dL, LDL-c 422 mg/dL with normal TG and HDL-c levels. Lipid lowering treatment with atorvastatine (10 g/day) lowered his LDL-c levels to 406mg/dL.

### EXAMPLE 11: Identification of mutations located in exon 11 of the LDLr gene.

A 194 bp fragment of exon 11 of the LDLr gene was amplified by polymerase chain reaction (PCR) using the following primers: Ex11F (SEQ ID NO: 25) and Ex11R (SEQ ID NO: 26).

DNA (500ng) was amplified in a 50 µL reaction mixture containing 20mM Tris-HCl, pH 8.4, 50 mM KCI, 1.5 mM MgCL₂, 200 µM each dNTP, 0.2 µM each primer and 1.5 units of Taq DNA polymerase (Gibco BRL, Carlsbad, CA, USA). The amplification cycle was: 10 min of denaturation at 96°C, followed by 35 cycles of denaturation at 94°C for 1 min, annealing at 65°C for 1 min, and elongation at 72°C for 2 min, and a final extension of 72°C for 10 min.

PCR products were analyzed by Single Strand Conformation Polymorphisms (SSCP). Those fragments that showed abnormal SSCP patterns were sequenced using an automated CEQ 2000XL DNA Analysis System (Beckman Coulter, Palo Alto, CA, USA). The presence of the identified mutations was subsequently determined by restriction analysis and by the device described previously "biochip".

### W515X mutation analysis

This mutation W515X (1607G>A, TGG>TAG, Trp515Stop) creates a new Bfal digestion site. Fifteen µL of PCR sample were digested with 15 units of Bfal in a total volume of 30 µL according to the protocol described by the manufacturer (NEB, Beverly, MA, USA). The fragments obtained had a length of 164 and 30 bp for normal alleles and 97, 67 and 30 bp for mutant alleles. These fragments were electrophoresed in 3% NuSieve agarose gel and were visualized by staining with ethidium bromide. Alternatively, this mutation could be analyzed with the microarray ("biochip") by spotting onto the slide the oligonucleotides: SEQ ID NO: 120, SEQ ID NO: 121, SEQ ID NO: 122 and SEQ ID NO: 123.

W515X mutation was detected in a 39 years old man, with arcus corneae and family history of hipercholesterolemia. His father suffered a myocardial infartion at age of 50. He has been diagnosed as having familial hypercholesterolemia with a MedPed diagnostic score of 13 points. An untreatment lipid determination gave us the following results: TC (364 mg/dL) and LDLc (308 mg/dL) with normal TG and HDLc levels.

### [1587-5del5; 1587del31] mutation analysis

This mutation [1587-5del5; 1587del31] was identified by DNA sequencing of the 194 bp fragment from exon 11 during screening for mutations in the LDL-r gene in subjects clinically diagnosed as having FH. Purified PCR product from DNA sample were directly sequenced in both directions using the amplification primers Ex11 F (SEQ ID NO: 25) and Ex11R (SEQ ID NO: 26) and the kit CEQ 2000 Dye Terminator Cycle Sequencing with Quick Start (Beckman Coulter, Palo Alto, CA, USA) according to the protocol described by the manufacturer. Sequences were detected using the CEQ 8000 Genetic Analysis System (Beckman Coulter, Inc. Fullerton), and analyzed with CEQ 8000 software. The deletion was confirmed by 2% agarose gel electrophoresis analyzing the PCR product two fragments of 194 and 158 pb could be observed. Alternatively, this mutation could be analyzed with the microarray ("biochip") by spotting onto the slide the oligonucleotides: SEQ ID NO: 256, SEQ ID NO: 257, SEQ ID NO: 258 and SEQ ID NO: 259.

[1587-5del5; 1587del31] mutation was detected in a 43 years man with arcus corneae and family history of autosomal dominant hypercholesterolemia. His father suffered a myocardial infartion at age of 50. He was diagnosed clinically as having FH with a MedPed score of 9 points. Analysis of his fasting serum lipid levels without use of lipid lowering therapy were: TC (345mg/dL) and TG (160 mg/dL) and HDLc (34 mg/dL). After combined lipid lowering treatment with simvastatin 40 mg/day and colestipol 10g/day the LDL-c levels decreased to 208 mg/dL.

### G516X Mutation analysis

This mutation (1609G>T, GGA>TGA, Gly516Stop) creates a new HphI digestion site. Fifteen µL of PCR sample were digested with 15 units of Hphl in a total volume of 30 µL according to the protocol described by the manufacturer (NEB, Beverly, MA, USA). The fragments obtained had a length 139, 43 and 12 pb for normal alleles and 81, 58, 43 y 12 pb for mutant alleles. These fragments were electrophoresed in 8% polyacrilamide gel and were visualized by staining with ethidium bromide. Alternatively, this mutation could be analyzed with the microarray ("biochip") by spotting onto the slide the oligonucleotides: 176, SEQ ID NO: 177, SEQ ID NO: 178 and SEQ ID NO: 179.

G516X mutation was detected in a 20 years old woman, with tendon xanthomas and family history of hipercholesterolemia. She has been diagnosed as having familial hypercholesterolemia with a MedPed diagnostic score of 17 points. Analysis of his fasting serum lipid levels before lipid lowering therapy were: TC 476 mg/dL, LDL-c 403 mg/dl and normal TG and HDL-c levels. After lipid lowering treatment with an HMGCoA reductase inhibitor the LDL-c levels decreased 202 mg/dL

### H562Q mutation analysis

This mutation H562Q (1749C>A, CAC>CAA, His562Gln) was identified by DNA sequencing of the 194 bp fragment from exon 11 during screening for mutations in the LDL-r gene in subjects clinically diagnosed as having FH. Purified PCR product from DNA sample were directly sequenced in both directions using the amplification primers Ex11F (SEQ ID NO: 25) and Ex11R (SEQ ID NO: 26) and the kit CEQ 2000 Dye Terminator Cycle Sequencing with Quick Start (Beckman Coulter, Palo Alto, CA, USA) according to the protocol described by the manufacturer. Sequences were detected using the CEQ 8000 Genetic Analysis System (Beckman Coulter, Inc. Fullerton), and analyzed with CEQ 8000 software. The C>A change was confirmed by sequencing a second PCR product. Alternatively, this mutation could be analyzed with the microarray ("biochip") by spotting onto the slide the oligonucleotides: SEQ ID NO: 208, SEQ ID NO: 209, SEQ ID NO: 210 and SEQ ID NO: 211.

H562Q mutation was detected in a 37 years woman with family history of autosomal dominant hypercholesterolemia. His father had hypercholesterolemia and suffered a myocardial infartion at age of 48 and her son at age of 13 has TC levels of 500 mg/dL She was diagnosed clinically as having FH with a MedPed score of 9 points. Analysis of her fasting serum lipid levels without use of lipid lowering therapy were: TC (350mg/dL) with normal TG and HDLc levels. After lipid lowering treatment with atorvastatin 20 mg/day the TC level lowered to 333 mg/dL.

### EXAMPLE 12: Identification of mutations located in exon 12 of the LDLr gene.

A 236 bp fragment of exon 12 of the LDLr gene was amplified by polymerase chain reaction (PCR) using the following primers: Ex12F (SEQ ID NO: 150) and Ex12R (SEQ ID NO:151).

DNA (500ng) was amplified in a 50 µL reaction mixture containing 20mM Tris-HCl, pH 8.4, 50 mM KCI, 1.5 mM MgCL₂, 200 µM each dNTP, 0.2 µM each primer and 1.5 units of Taq DNA polymerase (Gibco BRL, Carlsbad, CA, USA). The amplification cycle was: 10 min of denaturation at 96°C, followed by 35 cycles of denaturation at 94°C for 1 min, annealing at 58°C for 1 min, and elongation at 72°C for 2 min, and a final extension of 72°C for 10 min.

PCR products were analyzed by Single Strand Conformation Polymorphisms (SSCP). Those fragments that showed abnormal SSCP patterns were sequenced using an automated CEQ 2000XL DNA Analysis System (Beckman Coulter, Palo Alto, CA, USA). The presence of the identified mutations was subsequently determined by restriction analysis and by the device described previously "biochip".

### E579D mutation analysis

This mutation E579D (1800G>C, GAG>GAC, Glu579Asp)) was identified by DNA sequencing of 236 bp fragment from exon 12 during screening for mutations in the LDL-r gene in subjects clinically diagnosed as having FH. Purified PCR product from DNA sample were directly sequenced in both directions using the amplification primers Ex12F (SEQ ID NO: 150) and Ex12R (SEQ ID NO: 151) and the kit CEQ 2000 Dye Terminator Cycle Sequencing with Quick Start (Beckman Coulter, Palo Alto, CA, USA) according to the protocol described by the manufacturer. Sequences were detected using the CEQ 8000 Genetic Analysis System (Beckman Coulter, Inc. Fullerton), and analyzed with CEQ 8000 software. The G>C change was confirmed by sequencing a second PCR product. Alternatively, this mutation could be analyzed with the microarray ("biochip") by spotting onto the slide the oligonucleotides: SEQ ID NO: 224, SEQ ID NO: 225, SEQ ID NO: 226 and SEQ ID NO: 227.

E579D mutation was detected in a 49 years old man with family history of autosomal dominant hypercholesterolemia (Father TC 450mg/dL and his brother and two children with TC above to 95 percentile). He was diagnosed clinically as having FH with a MedPed score of 8 points. Analysis of her fasting serum lipid levels without use of lipid lowering therapy were: TC (320mg/dL), LDL-c (250 mg/dL) with normal TG and HDLc levels. After lipid lowering treatment with atorvastatin (10 mg/day) the LDL-c level lowered to 187 mg/dL.

### 1815del11 mutation analysis

This mutation was confirmed by heteroduplexes analysis; the electrophoresis in 8% polyacrilamide (PAA) gel of exon 12 PCR products visualized by staining with ethidium bromide, showed the presence of heteroduplexes bands instead of the corresponding normal and mutated homoduplexes. The fragments obtained had a length of 236 bp for normal alleles and 225 bp for mutant alleles. The heteroduplex band migrated more slowly because of the formation of the bubble between the michtmached sequences. Alternatively, this mutation could be analyzed with the microarray ("biochip") by spotting onto the slide the oligonucleotides SEQ ID NO: 184, SEQ ID NO: 185, SEQ ID NO: 186 and SEQ ID NO: 187.

1815del11 mutation was identified in four unrelated families with hypercholesterolemia. The index case of one of these families was a 69 years old woman with arcus cornealis and premature coronary artery disease at 56 years old. Her two brothers have marked elevation of plasma cholesterol levels, TC 700 and 435 mg/dL respectively. She was clinically diagnosed as having FH with a MedPed score of 13 points. Plasma lipid levels with lipid lowering treatment with simvastatin (40 mg/dL) were: TC 444 mg/dL, LDL-c 368 mg/dL and normal TG and HDL-c levels. After lipid lowering treatment with atorvastatin (30 mg/day) reduced her LDL-c to 225 mg/dL.

### EXAMPLE 13: Identification of mutations located in exon 13 of the LDLr gene.

A 215 bp fragment of exon 13 of the LDLr gene was amplified by polymerase chain reaction (PCR) using the following primers: Ex13F (SEQ ID NO: 27) and Ex13R (SEQ ID NO: 28).

DNA (500ng) was amplified in a 50 µL reaction mixture containing 20mM Tris-HCl, pH 8.4, 50 mM KCI, 1 mM MgCl₂, 200 µM each dNTP, 0.2 µM each primer and 1.5 units of Taq DNA polymerase (Gibco BRL, Carlsbad, CA, USA). The amplification cycle was: 10 min of denaturation at 96°C, followed by 35 cycles of denaturation at 94°C for 1 min, annealing at 59°C for 1 min, and elongation at 74°C for 3 min, and a final extension of 72°C for 10 min.

PCR products were analyzed by Single Strand Conformation Polymorphisms (SSCP). Those fragments that showed abnormal SSCP patterns were sequenced using an automated CEQ 2000XL DNA Analysis System (Beckman Coulter, Palo Alto, CA, USA). The presence of the identified mutations was subsequently determined by restriction analysis and by the device described previously "biochip".

### D630N mutation analysis

This mutation D630N (1951G>A, GAT>AAT, Asp630Asn) destroy a MnII digestion site. Fifteen µL of PCR sample were digested with 15 units of MnII in a total volume of 30 µL according to the protocol described by the manufacturer (Fermentas Inc., Hanover, MD, USA). The fragments obtained had a length of 89, 48, 39, 14+14, 12 and 11 bp in normal alleles and 89, 59, 39, 14+14 and 12 bp in mutant alleles. These fragments were electrophoresed in 8% polyacrilamide gel and were visualized by staining with ethidium bromide. Alternatively, this mutation could be analyzed with the microarray ("biochip") by spotting onto the slide the oligonucleotides: SEQ ID NO: 124, SEQ ID NO: 125, SEQ ID NO: 126 and SEQ ID NO: 127.

D630N mutation was detected in two unrelated families. Index case of one of this family was a 36 years old woman her parents died of myocardial infartion at 62 and 64 years old. Their MedPed score for FH clinical diagnostic was 7 points. An untreatment lipid determination gave us the following results: TC (332 mg/dL) and LDLc (268 mg/dL), TG (81 mg/dL) and HDLc (48 mg/dL).

### H635N mutation analysis

As this mutation H635N (1966C>A, CAC>AAC, His635Asn) does not change the restriction map, we designed a pair of mutagenic primers to introduce the recognition site of Cail in presence of the normal allele but not in presence of mutant allele.

A 169 bp fragment of exon 13 of the LDLr gene was amplified by polymerase chain reaction (PCR) using the following primers: Ex13F (SEQ ID. NO: 27) and MutH635NR (SEQ ID NO: 29).

DNA (500ng) was amplified in a 50 µL reaction mixture containing 20mM Tris-HCl, pH 8.4, 50 mM KCI, 1.5 mM MgCL₂, 200 µM each dNTP, 0.2 µM each primer and 1.5 units of Taq DNA polymerase (Gibco BRL, Carlsbad, CA, USA). The amplification cycle was: 10 min of denaturation at 96°C, followed by 35 cycles of denaturation at 94°C for 1 min, annealing at 56°C for 1 min, and elongation at 72°C for 1 min, and a final extension of 72°C for 10 min.

Fifteen µL of PCR sample were digested with 15 units of Cail in a total volume of 30 µL according to the protocol described by the manufacturer (Fermentas Inc., Hanover, MD, USA). The fragments obtained had a length of 151 and 18 bp in normal alleles and 169 bp in mutant alleles. These fragments were electrophoresed in 8% PAA gel and were visualized by staining with ethidium bromide. Alternatively, this mutation could be analyzed with the microarray ("biochip") by spotting onto the slide the oligonucleotides: SEQ ID NO: 128, SEQ ID NO: 129, SEQ ID NO: 130 and SEQ ID NO: 131.

H635N mutation was detected in a member of autosomal dominant hypercholesterolemia family. He was a 43 years old man, with arcus lipoides corneae. His mother and three sisters had elevated level of plasma cholesterol. He was clinically diagnosed as having FH with a MedPed score of 13 points Analysis of her fasting serum lipid levels without use of lipid lowering therapy were: TC (448 mg/dL) and LDLc (364 mg/dL) with normal TG and HDLc levels.

### EXAMPLE 14: Identification of mutations located in exon 14 of the LDLr gene.

A 288 bp fragment of exon 14 of the LDLr gene was amplified by polymerase chain reaction (PCR) using the following primers: Ex14F (SEQ ID NO: 30) and Ex14R (SEQ ID NO: 31).

DNA (250ng) was amplified in a 50 µL reaction mixture containing 20mM Tris-HCl, pH 8.4, 50 mM KCI, 1.5 mM MgCL₂, 20 µM each dNTP, 0.2 µM each primer and 1.5 units of Taq DNA polymerase (Gibco BRL, Carlsbad, CA, USA). The amplification cycle was: 10 min of denaturation at 96°C, followed by 35 cycles of denaturation at 94°C for 1 min, annealing at 59°C for 1 min, and elongation at 72°C for 2 min, and a final extension of 72°C for 10 min.

PCR products were analyzed by Single Strand Conformation Polymorphisms (SSCP). Those fragments that showed abnormal SSCP patterns were sequenced using an automated CEQ 2000XL DNA Analysis System (Beckman Coulter, Palo Alto, CA, USA). The presence of the identified mutations was subsequently determined by restriction analysis and by the device described previously "biochip".

### D686Ymutation analysis

This mutation D686Y (2119G>T, GAC>TAC, Asp686Tyr) was identified by DNA sequencing of 288 pb fragment from exon 14 during screening for mutations in the LDL-r gene in subjects clinically diagnosed as having FH. Purified PCR product from DNA sample were directly sequenced in both directions using the amplification primers Ex14F (SEQ ID NO: 30) and Ex14R (SEQ ID NO: 31).and the kit CEQ 2000 Dye Terminator Cycle Sequencing with Quick Start (Beckman Coulter, Palo Alto, CA, USA) according to the protocol described by the manufacturer. Sequences were detected using the CEQ 8000 Genetic Analysis System (Beckman Coulter, Inc. Fullerton), and analyzed with CEQ 8000 software. The G>T change was confirmed by sequencing a second PCR product. Alternatively, this mutation could be analyzed with the microarray ("biochip") by spotting onto the slide the oligonucleotides: SEQ ID NO: 216, SEQ ID NO: 217, SEQ ID NO: 218 and SEQ ID NO: 219.

D686Y mutation was detected in a 31 years old man with xantomas, arcus comeae, premature coronary artery disease and family history of autosomal dominant hypercholesterolemia. He was diagnosed clinically as having FH with a MedPed score of 21 points. Analysis of her fasting serum lipid levels without use of lipid lowering therapy were: TC (430mg/dL) with normal TG and HDLc levels. After combined lipid lowering treatment with atorvastatin 40mg/day and colestipol 5 (g/day) the TC levels decreased to 205 mg/dL.

### EXAMPLE 15: Identification of mutations located in exon 15 of the LDLr gene.

A 243 bp fragment of exon 15 of the LDLr gene was amplified by polymerase chain reaction (PCR) using the following primers Ex15F (SEQ ID NO: 32) and Ex15R (SEQ ID NO: 33).

DNA (500ng) was amplified in a 50 µL reaction mixture containing 20mM Tris-HCl, pH 8.4, 50 mM KCI, 1.5 mM MgCL₂, 20 µM each dNTP, 0.2 µM each primer and 1.5 units of Taq DNA polymerase (Gibco BRL, Carlsbad, CA, USA). The amplification cycle was: 10 min of denaturation at 96°C, followed by 35 cycles of denaturation at 94°C for 1 min, annealing at 55°C for 30 seconds, and elongation at 72°C for 1.5 min, and a final extension of 72°C for 10 min.

PCR products were analyzed by Single Strand Conformation Polymorphisms (SSCP). Those fragments that showed abnormal SSCP patterns were sequenced using an automated CEQ 2000XL DNA Analysis System (Beckman Coulter, Palo Alto, CA, USA). The presence of the identified mutations was subsequently determined by restriction analysis and by the device described previously "biochip".

### 2184delG mutation analysis

This mutation creates a new cleavage for the restriction enzyme for Alul.. Fifteen µL of PCR sample were digested with 15 units of Alul in a total volume of 30 µL according to the protocol described by the manufacturer (Gibco BRL, Carlsbad, CA, USA). The fragments obtained had a length of 166 and 78 bp for normal alleles and 166, 67 and 11 bp for mutant alleles. These fragments were electrophoresed in 8% polyacrilamide gel and were visualized by staining with ethidium bromide. Alternatively, this mutation could be analyzed with the microarray ("biochip") by spotting onto the slide the oligonucleotides: SEQ ID NO: 132, SEQ ID NO: 133, SEQ ID NO: 134 and SEQ ID NO: 135.

2184delC mutation was detected in a member autosomal dominant hypercholesterolemia family. She is a 32 years old woman, with family history of premature cardiovascular disease. Their MedPed score for FH clinical diagnostic was 6 points. Analysis of her fasting serum lipid levels without use of lipid lowering therapy were: TC (330 mg/dL) and LDLc (270 mg/dL) with normal TG and HDLc levels.

### T740M mutation analysis

This mutation T740M (2282C>T, ACG>ATG, Tyr740Met) creates a new NlaIII digestion site. Fifteen µL of PCR sample were digested with 15 units of NlaIII in a total volume of 30 µL according to the protocol described by the manufacturer (NEB, Beverly, MA, USA). The fragments obtained had a length 247 pb for normal alleles and 194 y 53 pb for mutant alleles. These fragments were electrophoresed in 8% polyacrilamide gel and were visualized by staining with ethidium bromide. Alternatively, this mutation could be analyzed with the microarray ("biochip") by spotting onto the slide the oligonucleotides: SEQ ID NO: 192, SEQ ID NO: 193, SEQ ID NO: 194 and SEQ ID NO: 195.

T740M mutation was detected in a 60 years old woman, with arcus comeae, family history of hypercholesterolemia and family history of cardiovascular disease. She has been diagnosed as having familial hypercholesterolemia with a MedPed diagnostic score of 10 points. Analysis of her fasting serum lipid levels before lipid lowering therapy were: TC 492 mg/dL and normal TG and HDL-c levels. After lipid lowering treatment with atorvastatin the TC levels lowered to 251 mg/dL.

### EXAMPLE 16: Identification of mutations located in exon 16 of the LDLr gene.

A 273 bp fragment of exon 16 of the LDLr gene was amplified by polymerase chain reaction (PCR) using the following primers: Ex16F (SEQ ID NO: 152) and Ex16R (SEQ ID NO: 153).

DNA (500ng) was amplified in a 50 µL reaction mixture containing 20mM Tris-HCl, pH 8.4, 50 mM KCI, 1.5 mM MgCL₂, 20 µM each dNTP, 0.2 µM each primer and 1.5 units of Taq DNA polymerase (Gibco BRL, Carlsbad, CA, USA). The amplification cycle was: 10 min of denaturation at 96°C, followed by 35 cycles of denaturation at 94°C for 1 min, annealing at 63°C for 1 min, and elongation at 72°C for 2 min, and a final extension of 72°C for 10 min.

PCR products were analyzed by Single Strand Conformation Polymorphisms (SSCP). Those fragments that showed abnormal SSCP patterns were sequenced using an automated CEQ 2000XL DNA Analysis System (Beckman Coulter, Palo Alto, CA, USA). The presence of the identified mutations was subsequently determined by restriction analysis and by the device described previously "biochip".

### V766E mutation analysis

This mutation V766E (2360T>A, GTG>GAG, Val766Glu) was identified by DNA sequencing of the 273 pb fragment from exon 16 during screening for mutations in the LDL-r gene in subjects clinically diagnosed as having FH. Purified PCR product from DNA sample were directly sequenced in both directions using the amplification primers Ex 16F (SEQ ID NO: 152) and EX16R (SEQ ID NO: 153) and the kit CEQ 2000 Dye Terminator Cycle Sequencing with Quick Start (Beckman Coulter, Palo Alto, CA, USA) according to the protocol described by the manufacturer. Sequences were detected using the CEQ 8000 Genetic Analysis System (Beckman Coulter, Inc. Fullerton), and analyzed with CEQ 8000 software. The 2360T>A change was confirmed by sequencing a second PCR product. Alternatively, this mutation could be analyzed with the microarray ("biochip") by spotting onto the slide the oligonucleotides: SEQ ID NO: 236, SEQ ID NO: 237, SEQ ID NO: 238 and SEQ ID NO: 239.

D686Y mutation was detected in a 58 years old woman with tendon xantomas, arcus corneae, xantelasmas and family history of premature coronary artery disease. She was diagnosed clinically as having FH with a MedPed score of 12 points. Analysis of her fasting serum lipid levels without use of lipid lowering therapy were: TC (420mg/dL), LDL-c (324 mg/dL) with normal TG and HDLc levels.

### I771T mutation analysis

As this mutation I771T (2375T>C, ATT>CACT, Ile771Thr), does not change the restriction map, we designed a pair of mutagenic primers to introduce the recognition site of HincII in presence of the mutant allele but not in presence of normal allele.

A 142 bp fragment of exon 16 of the LDLr gene was amplified by polymerase chain reaction (PCR) using the following primers: Ex16R (SEQ ID NO: 153) and Mut1771TF (SEQ ID NO: 154).

DNA (500ng) was amplified in a 50 µL reaction mixture containing 20mM Tris-HCl, pH 8.4, 50 mM KCI, 1.5 mM MgCL₂, 200 µM each dNTP, 0.2 µM each primer and 1.5 units of Taq DNA polymerase (Gibco BRL, Carlsbad, CA, USA). The amplification cycle was: 10 min of denaturation at 96°C, followed by 35 cycles of denaturation at 94°C for 1 min, annealing at 61 °C for 1 min, and elongation at 72°C for 2 min, and a final extension of 72°C for 10 min.

Fifteen µL of PCR sample were digested with 15 units of HincII in a total volume of 30 µL according to the protocol described by the manufacturer fabricante (Amersham Pharmacia Biotech Inc., Piscataway, NJ, USA). The fragments obtained had a length of 142 bp in normal alleles and 121 and 21 bp in mutant alleles. These fragments were electrophoresed in 8% polyacrilamide gel and were visualized by staining with ethidium bromide. Alternatively, this mutation could be analyzed with the microarray ("biochip") by spotting onto the slide the oligonucleotides SEQ ID NO: 196, SEQ ID NO: 197, SEQ ID NO: 198 and SEQ ID NO: 199.

I771T mutation was detected in a 60 years old woman from a family with autosomal dominant hypercholesterolemia and history of premature cardiovascular disease. She has been diagnosed as having familial hypercholesterolemia with a MedPed diagnostic score of 21 points. Her plasma lipid levels were: TC (422 mg/dL) and LDLc (368 mg/dL), and normal TG and HDLc levels.

### 2389+3 C>T mutation analysis

This mutation 2389+3 C>T was identified by DNA sequencing of the 273 pb fragment from exon 16 during screening for mutations in the LDL-r gene in subjects clinically diagnosed as having FH. Purified PCR product from DNA sample were directly sequenced in both directions using the amplification primers Ex 16F (SEQ ID NO: 152) and EX16R (SEQ ID NO: 153) and the kit CEQ 2000 Dye Terminator Cycle Sequencing with Quick Start (Beckman Coulter, Palo Alto, CA, USA) according to the protocol described by the manufacturer. Sequences were detected using the CEQ 8000 Genetic Analysis System (Beckman Coulter, Inc. Fullerton), and analyzed with CEQ 8000 software. The C>T change was confirmed by sequencing a second PCR product. Alternatively, this mutation could be analyzed with the microarray ("biochip") by spotting onto the slide the oligonucleotides: SEQ ID NO: 252, SEQ ID NO: 253, SEQ ID NO: 254 and SEQ ID NO: 255.

2389+3 C>T mutation was detected in a 36 years old man with multiple tendon xantomas and family history of hypercholesterolemia. He was diagnosed clinically as having FH with a MedPed score of 18 points. Analysis of her fasting serum lipid levels without use of lipid lowering therapy were: TC (450mg/dL) with normal TG and HDLc levels. Lipid lowering treatment with atorvastatin (20mg/dL) reduced his LDL-c to 259 mg/dL.

### 2389+4 A>G mutation

As this mutation (2389+4 A>G) does not change the restriction map, we designed a pair of mutagenic primers to introduce the recognition site of BshNI in presence of the mutant allele but not in presence of normal allele.

A 194 bp fragment of exon 16 of the LDLr gene was amplified by polymerase chain reaction (PCR) using the following primers: Ex16F (SEQ ID NO: 152) and Mut2389+4 A>GR (SEQ ID NO: 155).

DNA (500ng) was amplified in a 50 µL reaction mixture containing 20mM Tris-HCl, pH 8.4, 50 mM KCI, 1.5 mM MgCL₂, 200 µM each dNTP, 0.2 µM each primer and 1.5 units of Taq DNA polymerase (Gibco BRL, Carlsbad, CA, USA). The amplification cycle was: 10 min of denaturation at 96°C, followed by 35 cycles of denaturation at 94°C for 1 min, annealing at 61 °C for 1 min, and elongation at 72°C for 2 min, and a final extension of 72°C for 10 min.

Fifteen µL of PCR sample were digested with 15 units of BshNI in a total volume of 30 µL according to the protocol described by the manufacturer fabricante (Fermentas Inc., Hanover, MD, USA). The fragments obtained had a length of 194 bp for normal alleles and 175 and 19 bp for mutant alleles. These fragments were electrophoresed in 8% polyacrilamide gel and were visualized by staining with ethidium bromide. Alternatively, this mutation could be analyzed with the microarray ("biochip") by spotting onto the slide the oligonucleotides SEQ ID NO: 180, SEQ ID NO: 181, SEQ ID NO: 182 and SEQ ID NO: 183.

2389+4 A>G mutation was detected 11 unrelated hypercholesterolemic families. Index case of one of these families was a 22 years old woman tendon xanthomas and family history of premature cardiovascular disease. She has been diagnosed as having familial hypercholesterolemia with a MedPed diagnostic score of 17 points. Her plasma lipid levels without lipid lowering treatment were: TC (356 mg/dL) and LDLc (293 mg/dL), and normal TG and HDLc levels. Combined lipid lowering treatment with atorvastatin (40 mg/day) and colestipol (5g/day) lowered her HLD-c level to 227 mg/dL.

### EXAMPLE 17: Identification of mutations located in exon 17 of the LDLr gene.

A 242 bp fragment of exon 17 of the LDLr gene was amplified by polymerase chain reaction (PCR) using the following primers: Ex17F (SEQ ID NO: 34) and Ex17R (SEQ ID NO: 35).

DNA (300ng) was amplified in a 50 µL reaction mixture containing 20mM Tris-HCl, pH 8.4, 50 mM KCI, 1.5 mM MgCL₂, 200 µM each dNTP, 0.2 µM each primer and 1.5 units of Taq DNA polymerase (Gibco BRL, Carlsbad, CA, USA). The amplification cycle was: 10 min of denaturation at 96°C, followed by 35 cycles of denaturation at 94°C for 1 min, annealing at 58°C for 1 min, and elongation at 72°C for 1 min, and a final extension of 72°C for 10 min.

PCR products were analyzed by Single Strand Conformation Polymorphisms (SSCP). Those fragments that showed abnormal SSCP patterns were sequenced using an automated CEQ 2000XL DNA Analysis System (Beckman Coulter, Palo Alto, CA, USA). The presence of the identified mutations was subsequently determined by restriction analysis and by the device described previously "biochip".

### 2399del5ins4 mutation analysis

This mutation eliminates the sequence TCTTC and introduces the sequence

GGGT at the 2399 position, and creates a new Aval digestion site. Fifteen µL of PCR sample were digested with 15 units of Aval in a total volume of 30 µL according to the protocol described by the manufacturer (Amersham Pharmacia Biotech Inc., Piscataway, NJ, USA). The fragments obtained had a length of 230 and 12 bp in normal alleles and 183, 46 and 12 bp in mutant alleles. These fragments were electrophoresed in 8% polyacrilamide gel and were visualized by staining with ethidium bromide. Alternatively, this mutation could be analyzed by the microarray ("biochip") using into the slide the oligonucleotides SEQ ID NO: 136, SEQ ID NO: 137, SEQ ID NO: 138 and SEQ ID NO: 139.

2399del5ins4 mutation was detected in three unrelated hypercholesterolemic families. The index case of one of these families was a 49 years old woman, with tendon xanthomas. She has two children with hypercholesterolemia, TC 330 and 386 mg/dL respectively. Her father had died of cardiovascular disease at age of 51. Their MedPed score for FH clinical diagnostic was 16 points. Her plasma lipid levels before lipid lowering treatment were: TC (510 mg/dL, LDLc (424 mg/dL).), HDLc (58 mg/dL) and TG (140 mg/dL). Combined treatment with simvastatin 20 mg/day and colestipol 20 g/day lowered her TC to 280 mg/dL.

### 2544insC mutation analysis

This insertion 2544insC was identified by DNA sequencing of the 242 pb fragment from exon 17 during screening for mutations in the LDL-r gene in subjects clinically diagnosed as having FH. Purified PCR product from DNA sample were directly sequenced in both directions using the amplification primers Ex17F (SEQ ID NO: 34) and Ex17R (SEQ ID NO: 35) and the kit CEQ 2000 Dye Terminator Cycle Sequencing with Quick Start (Beckman Coulter, Palo Alto, CA, USA) according to the protocol described by the manufacturer. Sequences were detected using the CEQ 8000 Genetic Analysis System (Beckman Coulter, Inc. Fullerton), and analyzed with CEQ 8000 software. The C insertion was confirmed by sequencing a second PCR product. Alternatively, this mutation could be analyzed with the microarray ("biochip") by spotting onto the slide the oligonucleotides: SEQ ID NO: 244, SEQ ID NO: 245, SEQ ID NO: 246 and SEQ ID NO: 247.

2544insC mutation was detected in a 37 years old man, who has been suffered a myocardial infartion. He has tendon xanthomas, arcus corneae, family history of hypercholesterolemia and coronary heart disease. He was diagnosed clinically as having FH with a MedPed score of 21 points. Analysis of her fasting serum lipid levels without use of lipid lowering therapy were: TC (444 mg/dL), LDL-c (379 mg/dL) with normal TG and HDLc levels. Lipid lowering treatment with atorvastatin (40mg/dL) lowered his LDL-c to 282 mg/dL.

### Brief description of the drawings

**Figure 1** is a schematic representation of the itinerary of the LDL-r in human cells. The LDL-r is synthesized in the endoplasmic reticulum as a precursor of apparent molecular weight of 120 Kd and transported at the Golgi complex where the N-linked carbohydrates are processed. Once transferred to the surface of the cell the receptor recognize the apolipoprotein B-100 component of the LDL. Binding leads to cellular uptake and lysosomal degradation of the LDL by receptor-mediated endocytosis. This uptake process satisfies the cholesterol needs of the cells, and hence keeps cholesterol synthesis suppressed.
**Figure 2** is a schematic representation of the five domains in the structure of the human LDL receptor protein and their corresponding exons in the LDL receptorgene.
**Figure 3** shows the microarray picture of the region used for analyzing the E256K mutation. Two pairs of oligonucleotides were spotted for each mutation. Each probe pair consists of one probe specific for the wild-type allele and a second probe specific for the mutant allele. (A) wild type subject. (B) FH patient carrier of E256K mutation.

## Claims

1. An *in vitro* method for diagnosing familial hypercholesterolemia that comprises detecting at least the mutation 313+1insT in the LDL-r gene represented by SEQ ID NO: 1 from a biological sample of an individual.

2. The method, according to claim 1, further comprising the detection of the mutation 313+1G>C.

3. The method, according to claims 1 or 2, further comprising the detection of the mutation Q71 E.

4. The method, according to claim 1, that further comprises detecting a mutation selected from: (-23)A>C, 1054 del11, 108delC, 1197del9, 1207de1T, 1432delG, 191-2delAinsCT, 2184delG, 231delC, 2399de15ins4, 338dell6, 509insC, 675dell5, 684dup12, 941-39C>T, C195R, C255G, C319Y, D157G, D630N, E291X, H635N, N59K, T41M, W515X, Y379X, Y421X, T433N, 818de18, 1423delGC/insA, 1204insT, 451de13, G516X, 2389+4A>G, 1815del11, 1186+5G>A, T740M, I771T, R279G, T446I, H562Q, C74Y, D686Y, G(-2)R, E579D, S205C, D200V, V766E, L(-6)P, 2544insC, C42Y, 2389+3A>C, [1587-5de15;1587de131].

5. The method, according to claim 1, which comprises hybridising the oligonucleotides having the sequences SEQ ID NO: 56, SEQ ID NO: 57, SEQ ID NO: 58 and SEQ ID NO: 59 to the LDL-r gene represented by SEQ ID NO: 1, from a biological sample of an individual.

6. The method, according to claim 1, which comprises detecting a group of mutations consisting of: 313+linsT, 313+1G>C and Q71E in the LDL-r gene represented by SEQ ID NO: 1, from a biological sample of an individual.

7. Kit for diagnosing familial hypercholesterolemia in a biological sample of an individual, that comprises the oligonucleotides having the sequences: SEQ ID NO: 56, SEQ ID NO: 57, SEQ ID NO: 58 and SEQ ID NO: 59 that hybridize to the LDL-r gene represented by SEQ ID NO: 1.

## Patentansprüche

1. *In vitro* Verfahren zur Diagnostizierung von familiärer Hypercholesterinämie, das den Nachweis von wenigstens der Mutation 313+1insT in dem LDL-r-Gen umfasst, repräsentiert durch die SEQ ID NO:1, aus einer biologischen Probe von einem Individuum.

2. Verfahren nach Anspruch 1, ferner umfassend den Nachweis der Mutation 313+1 G>C.

3. Verfahren nach Anspruch 1 oder 2, ferner umfassend den Nachweis der Mutation Q71 E.

4. Verfahren nach Anspruch 1, ferner umfassend den Nachweis einer Mutation ausgewählt aus: (-23)A>C, 1054 del11, 108delC, 1197del9, 1207delT, 1432delG, 191-2delAinsCT, 2184delG, 231delC, 2399del5ins4, 338del16, 509insC, 675del15, 684dup12, 941-39C>T, C195R, C255G, C319Y, D157G, D630N, E291X, H635N, N59K, T41M, W515X, Y379X, Y421X, T433N, 818del8, 1423delGC/insA, 1204insT, 451del3, G516X, 2389+4A>G, 1815del11, 1186+5G>A, T740M, 1771T, R279G, T4461, H562Q, C74Y, D686Y, G(-2)R, E579D, S205C, D200V, V766E, L(-6)P, 2544insC, C42Y, 2389+3A>C, [1587-5de15;1587del31].

5. Verfahren nach Anspruch 1, umfassend die Hybridisierung der Oligonukleotide, die die Sequenzen SEQ ID NO: 56, SEQ ID NO: 57, SEQ ID NO: 58 und SEQ ID NO: 59 haben, an das LDL-r-Gen, repräsentiert durch die SEQ ID NO: 1, aus einer biologischen Probe von einem Individuum.

6. Verfahren nach Anspruch 1, umfassend den Nachweis einer Gruppe von Mutationen bestehend aus: 313+1insT, 313+1 G>C und Q71 E in dem LDL-r-Gen, repräsentiert durch die SEQ ID NO: 1, aus einer biologischen Probe von einem Individuum.

7. Kit zur Diagnostizierung von familiärer Hypercholesterinämie in einer biologischen Probe von einem Individuum, der die Oligonukleotide umfasst, die die Sequenzen SEQ ID NO: 56, SEQ ID NO: 57, SEQ ID NO: 58 und SEQ ID NO: 59 haben, die an das LDL-r-Gen, repräsentiert durch die SEQ ID NO:1, hybridisieren.

## Revendications

1. Procédé de diagnostic in vitro de l'hypercholestérolémie familiale, qui comprend la détection d'au moins la mutation 313+1insT dans le gène LDL-r représenté par SEQ ID N° : 1 à partir d'un échantillon biologique d'un individu.

2. Procédé selon la revendication 1, comprenant en outre la détection de la mutation 313+1G>G.

3. Procédé selon les revendications 1 ou 2, comprenant en outre la détection de la mutation Q71E.

4. Procédé selon la revendication 1, qui comprend en outre la détection d'une mutation choisie parmi : (-23)A>C, 1054del11, 108delC, 1197del9, 1207delT, 1432delG, 191-2delAinsCT, 2184delG, 231delC, 2399del5ins4, 338del16, 509insC, 675del15, 684dup12, 941-39C>T, C195R, C255G, C319Y, D157G, D630N, E291X, H635N, N59K, T41M, W515X, Y379X, Y421X, T433N, 818del8, 1423delGC/insA, 1204insT, 451del3, G516X, 2389+4A>G, 1815del11, 1186+5G>A, T740M, 1771T, R279G, T446I, H562Q, C74Y, D686Y, G(-2)R, E579D, S205C, D200V, V766E, L(-6)P, 2544insC, C42Y, 2389+3A>C, [1587-5del5;1587del31].

5. Procédé selon la revendication 1, qui comprend l'hybridation des oligonucléotides ayant les séquences SEQ ID N° 56, SEQ ID N° 57, SEQ ID N° 58 et SEQ ID N° : 59 au gène LDL-r représenté par SEQ ID N° : 1, à partir d'un échantillon biologique d'un individu.

6. Procédé selon la revendication 1, qui comprend la détection d'un groupe de mutations consistant en : 313+linsT, 313+1G>C et Q71E dans le gène LDL-r représenté par SEQ ID N° : 1, à partir d'un échantillon biologique d'un individu.

7. Kit de diagnostic de l'hypercholestérolémie familiale dans un échantillon biologique d'un individu, qui comprend les oligonucléotides ayant les séquences : SEQ ID N° 56, SEQ ID N° : 57, SEQ ID N° : 58 et SEQ ID n° 59 qui s'hybride au gène LDL-r représenté par SEQ ID N°: 1.
